(19) Europäisches Patentamt
European Patent Office
Office européen des brevets

(11) Veröffentlichungsnummer: **0 236 939**
**A1**

(12) # EUROPÄISCHE PATENTANMELDUNG

(21) Anmeldenummer: **87103098.7**

(22) Anmeldetag: **05.03.87**

(51) Int. Cl.⁴: **C07D 493/22**, A01N 43/20, A61K 31/365, //(C07D493/22,313:00,311:00,311:00,307:00)

(30) Priorität: **07.03.86 CH 935/86**

(43) Veröffentlichungstag der Anmeldung: **16.09.87 Patentblatt 87/38**

(84) Benannte Vertragsstaaten: **AT BE CH DE ES FR GR IT LI LU NL SE**

(71) Anmelder: **CIBA-GEIGY AG**
**Klybeckstrasse 141**
**CH-4002 Basel(CH)**

(72) Erfinder: **Maienfisch, Peter, Dr.**
**Traugott Meyer-Strasse 5**
**CH-4147 Aesch(CH)**
Erfinder: **O'Sullivan, Anthony Cornelius, Dr.**
**Habsburgerstrasse 38**
**CH-4055 Basel(CH)**

(74) Vertreter: **Zumstein, Fritz, Dr. et al**
**Bräuhausstrasse 4**
**D-8000 München 2(DE)**

(54) **5-Acyloxy-13Beta-alkyl-milbemycinderative zur Bekämpfung von tier- oder pflanzenparasitären Schädlingen.**

(57) Es werden parasitizide 5-Acyloxy-13β-alkyl-milbemycine der allgemeinen Formel I

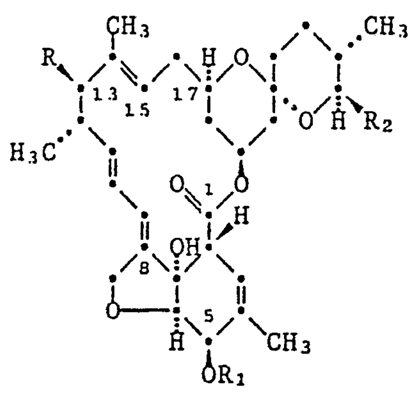

(I)

worin
R für $C_1$-$C_{10}$-Alkyl steht;
$R_1$ eine Acylgruppe darstellt; und $R_2$ für Methyl, Ethyl, Isopropyl oder sek.-Butyl steht; gegebenenfalls unter Einschluss ihrer Säureadditionssalze und Metallkomplexe beschrieben, sowie die Herstellung aus den zugrundeliegenden 13β-Alkyl-5-hydroxy-milbemycinen.

EP 0 236 939 A1

# 5-Acyloxy-13β-alkyl-milbemycin-derivate zur Bekämpfung von tier-oder pflanzenparasitären Schädlingen

Die vorliegende Erfindung betrifft neue 5-Acyloxy-13β-alkyl-milbemycin-Derivate der nachstehenden Formel I, deren Herstellung sowie deren Verwendung zur Bekämpfung von Schädlingen wie Ekto-und Endoparasiten an Tieren und Pflanzenparasiten.

Bei den erfindungsgemässen Verbindungen handelt es sich um 5-Acyloxy-13β-alkyl-milbemycine der allgemeinen Formel I

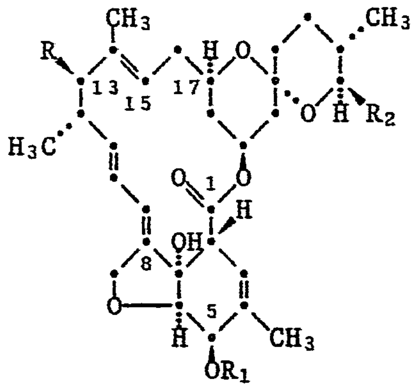

(I)

worin

R für $C_1$-$C_{10}$-Alkyl steht;

$R_1$ eine Acylgruppe darstellt; und $R_2$ für Methyl, Ethyl, Isopropyl oder sek.-Butyl steht; gegebenenfalls unter Einschluss ihrer Säureadditionssalze und Metallkomplexe.

Formel I repräsentiert somit Milbemycin-abkömmlinge, die eine 13β-Alkylgruppe enthalten und in 5-Position acyliert sind und in den Fällen in denen der Acylrest einen stickstoffhaltigen Heterocyclus trägt als Säureadditionssalz oder Metallkomplex vorliegen können.

Unter dem Begriff Acyl selbst oder als Bestandteil einer Acyloxygruppe sollen im Rahmen vorliegender Erfindung beispielsweise unsubstituierte oder substituiertes Alkylcarbonyl (= Alkanoyl), Arylcarbonyl und Aralkylcarbonyl, vorzugsweise unsubstituiertes oder substituiertes Acetyl, Propionyl, Butyryl oder Benzoyl verstanden werden, die im Falle der Alkanoyle als Substituenten z.B. Halogen, Alkoxy, Haloalkoxy, Aryloxy, Hydroxy, Aryl, Aryloxy oder einen ungesättigten oder gesättigten, fünf-oder sechsgliedrigen, heterocyclischen Ring mit ein-bis drei Heteroatomen ausgewählt aus der Reihe Stickstoff, Sauerstoff und Schwefel enthalten können, wobei die als Aryl bezeichneten Teile ihrerseits durch Halogen, Cyano, Alkyl, Alkoxy, Haloalkyl, Haloalkoxy, Alkylthio und/oder Nitro substituiert sein können und z.B. für α-, β-Naphthyl oder vorzugsweise Phenyl stehen.

Aralkyl steht für einen über eine geradkettige oder verzweigte Alkylenbrücke verbundenen aromatischen Rest; der einfachste Vertreter ist die Benzylgruppe.

Unter dem Begriff Alkyl selbst oder als Bestandteil eines anderen Substituenten sind je nach Zahl der angegebenen Kohlenstoffatome beispielsweise folgende geradkettige Gruppen zu verstehen: Methyl, Ethyl, Propyl, Butyl, Pentyl, Hexyl, Heptyl, Octyl, Nonyl, Decyl, Undecyl, Dodecyl, Tridecyl, Tetradecyl, Pentadecyl, Hexadecyl, Heptadecyl, Octadecyl usw. sowie ihre verzweigten Isomeren, wie z.B. Isopropyl, Isobutyl, tert.-Butyl, Isopentyl usw.. Alkenyl steht selbst oder als Bestandteil eines Alkenyloxy z.B. für Propenyl-(1), Allyl, Butenyl-(1), Butenyl-(2), Butenyl-(3) usw. Alkinyl steht beispielsweise für Ethinyl, Propinyl-(1), Propargyl, Butinyl-(1), usw. Unter Halogen selbst oder als Vorsilbe Halo soll hier und im folgenden Fluor, Chlor, Brom oder Jod verstanden werden, vorzugsweise Chlor oder Brom. Beispiele für Cycloalkyl selbst oder als Bestandteil von Cycloalkoxy sind Cyclopropyl, Cyclobutyl, Cyclopentyl, Cyclohexyl, Cycloheptyl, Cyclooctyl etc.. Haloalkyl repräsentiert allein oder als Bestandteil von Haloalkoxy einen einbis perhalogenierten Alkylrest, wie z.B. $CH_2J$, $CH_2Br$, $CH_2Cl$, $CH_2F$, $CHCl_2$, $CHF_2$, $CCl_3$, $CBr_3$, $CF_3$, $C_2F_5$, $C_2Cl_5$, CFClBr, usw., wobei der Rest auch gleichzeitig durch unterschiedliche Halogenatome substituiert sein kann; bevorzugt ist jedoch der $CF_3$-Rest. Typische fünfgliedrige heterocyclische Ringe sind: Furan, Thiophen, Pyrrol, Isoxazol, Isothiazol, Furazan, Imidazol, 1,2,4-Triazol, 1,2,3-Triazol, Pyrazol, Pyrrolin, Oxazol, Thiazol, Thiadiazole, Pyrazolin, Thiazolin, Pyrazolidin, Pyrrolidin, Oxazolidin, Thiazolidin, Oxadiazol, Imidazolin, Imidazolidin, Pyrazolidin, Tetrahydrofuran; und typische sechsgliedrige heterocyclische Ringe sind Pyridin, Pyridazin, Pyrimidin,

Pyrazin, Thiazin, Thiadiazine, Pyrane, Piperidin, Piperazin, Morpholin, Perhydrothiazin, Dioxan sowie ihre teilhydrierten bzw. teilgesättigten Homologen, usw.. Oxo substituierte Heterocyclen sind hier und im folgenden insbesondere fünfgliedrige und sechsgliedrige Lactone und Lactame, wie z.B. Butyrolactone, Valerolactone, Butyrolactam, Valerolactam, aber auch bicyclische Systeme wie Camphan.

Beispiele für den heterocyclischen Substituenten sind Imidazol, Pyrazol, 1,2,3-Triazol, 1,2,4-Triazol, 1,3,4-Triazol oder Tetrazol sowie ein-bis zweifach durch $C_1$-$C_6$-Alkylreste substituierte Azole wie 2-Ethyl-4-methylimidazol, 2-Isopropylimidazol, Methylimidazol, 3,5-Dimethyltriazol, Ethyltriazol, 3,4-Diethylpyrazol u.a.

Nachfolgend werden einige typische Vertreter von $R_1$-Acylresten genannt, wobei diese Aufzählung keinen limitierenden Charakter besitzt: $COCH_3$, $COCH_2Cl$, $COCF_3$, $COCH_2Br$, $COCH_2F$, $COC_2H_5$, $COC_2Cl_5$, $COCH_2OCOCH_3$, $COCH_2OCOC_4H_9$(t), $COCH_2OCH_3$, $COCHFOCOCH_3$, $COCH(CH_3)OCOCH_3$, $COCH_2OCOC_2H_5$, $COCH_2OCOCH_2Cl$, $COCH_2OCOC_6H_4F$(3), $COCH_2OCOC_6H_4OCH_3$(3), $COCH_2OCH_2OCH_3$, $COCH_2OC_2H_5$, $COCH_2SCH_3$, $COCH_2OCOC_6H_4Cl$(3), $COCH_2$-(1H-1,2,4-triazol-1-yl), $COCH_2$-(2,3-dihydropyran-2-yl), $COCH_2$-(1H-imidazol-1-yl) und $COCH_2OCO$-(pyrolidon-(2)-5-yl).

Salze werden aus Verbindungen der Formel I mit anorganischen oder organischen Säuren gebildet. Als Beispiele seien Chlorwasserstoffsäure, Bromwasserstoffsäure, Schwefelsäure, Salpetersäure, Phosphorsäure, Methansulfonsäure, p-Toluolsulfonsäure, Essigsäure, Oxalsäure, Weinsäure, Citronensäure, Ascorbinsäure, Sorbinsäure, Trimethylessigsäure, Benzoesäure, Salicylsäure, Bernsteinsäure, Maleinsäure genannt.

Als Metallkomplex-Bildner sind vor allem Kationen der I. und II. oder der IV. bis VIII. Nebengruppe des Periodensystems der Elemente geeignet. Beispiele sind Kupfer, Zink, Mangan, Chrom, Eisen, Nickel, Kobalt, Molybdän.

Die vorstehenden Aufzählungen stellen keine Limitierungen dar. Der Fachmann kennt weitere physiologisch verträgliche Salze und Komplexbildner.

Im Umfang der Formel I sind unter anderen diejenigen Milbemycin-Derivate bevorzugt, worin R und $R_2$ für Methyl oder Ethyl stehen und $R_1$ die angegebenen Bedeutungen hat.

Im Umfang der Formel I sind folgende interessante Gruppen zu nennen:

<u>Gruppe Ia:</u>

Verbindungen der Formel I, worin
R für $C_1$-$C_6$-Alkyl steht;
$R_2$ für Methyl, Ethyl, Isopropyl oder sek.-Butyl steht;
$R_1$ eine der Acylgruppen

a) 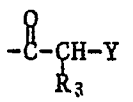 ,

b) 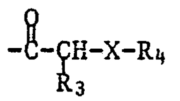 ,

c) 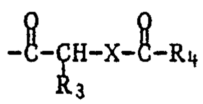 oder

d) 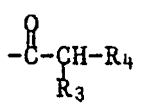 repräsentiert, wobei

X für Sauerstoff oder Schwefel steht;
Y eine nukleophil ersetzbare Abgangsgruppe darstellt;
$R_3$ Wasserstoff, $C_1$-$C_4$-Alkyl oder Halogen bedeutet; und
$R_4$ für Wasserstoff, für unsubstituiertes oder durch Halogen, Hydroxy, $C_1$-$C_6$-Alkoxy oder $C_1$-$C_6$-Haloalkoxy substituiertes $C_1$-$C_{10}$-Alkyl, für einen unsubstituierten oder substituierten Rest ausgewählt aus der Reihe $C_3$-$C_{10}$-Cycloalkyl, $C_2$-$C_6$-Alkenyl und $C_3$-$C_6$-Alkinyl, wobei die Substituenten ausgewählt sind aus der Reihe Halogen, Hydroxy, $C_1$-$C_6$-Alkoxy und $C_1$-$C_6$-Alkanoyloxy, für unsubstituiertes oder durch Halogen, Cyano, $C_1$-$C_3$-Akyl, $C_1$-$C_3$-Haloalkyl, $C_1$-$C_3$-Alkoxy, $C_1$-$C_3$-Haloalkoxy und/oder Nitro substituiertes Phenyl oder für einen unsubstituierten oder substituierten, ungesättigten oder gesättigten, fünf-oder sechsgliedrigen heterocyclischen Ring mit ein bis drei Heteroatomen, ausgewählt aus der Reihe Stickstoff, Sauerstoff und Schwefel, dessen Substituenten ausgewählt sind aus der Reihe Oxo, Halogen, $C_1$-$C_4$-Alkyl, $C_1$-$C_4$-Haloalkyl und $C_1$-$C_4$-Alkoxy steht, unter Einschluss der Säureadditionssalze und Metallkomplexe.

Innerhalb der Gruppe Ia sind folgende, die Gruppe Ib bildende Verbindungen der Formel I hervorzuheben.

Gruppe Ib:

Verbindungen der Formel I, worin
R für $C_1$-$C_6$-Alkyl steht;
$R_2$ für Methyl, Ethyl, Isopropyl oder sek.-Butyl steht;
$R_1$ eine der Acylgruppen

a) 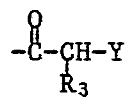 ,

b) 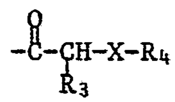 ,

c) 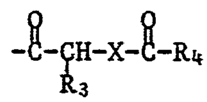 oder

d) 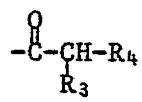 repräsentiert, wobei

X für Sauerstoff oder Schwefel steht;
Y für Halogen, Azido oder einen Sulfonsäurerest steht;
$R_3$ für Wasserstoff, Fluor oder Methyl steht; und
$R_4$ für Wasserstoff, für unsubstituiertes oder durch Fluor, Chlor, Brom, Hydroxy, $C_1$-$C_3$-Alkoxy oder $C_1$-$C_3$-Haloalkoxy substituiertes $C_1$-$C_6$-Alkyl, für einen unsubstituierten oder substituierten Rest ausgewählt aus der Reihe $C_3$-$C_7$-Cycloalkyl, $C_2$-$C_4$-Alkenyl und $C_2$-$C_4$-Alkinyl, wobei die Substituenten ausgewählt sind aus der Reihe Fluor, Chlor, Brom, Hydroxy, $C_1$-$C_3$-Alkoxy und $C_1$-$C_4$-Alkanoyloxy, für unsubstituiertes oder Fluor, Chlor, Brom, Methyl, Ethyl, Methoxy, Halomethoxy, Trifluormethyl und/oder Nitro substituiertes Phenyl oder für einen unsubstituierten oder substituierten, ungesättigten oder gesättigten, fünf-oder sechsgliedrigen heterocyclischen Ring mit ein bis drei Heteroatomen, ausgewählt aus der Reihe Stickstoff, Sauerstoff und Schwefel, dessen Substituenten ausgewählt sind aus der Reihe Oxo, Halogen, $C_1$-$C_4$-Alkyl, $C_1$-$C_4$-Haloalkyl und $C_1$-$C_4$-Alkoxy steht, unter Einschluss der Säureadditionssalze und Metallkomplexe.

Innerhalb der Gruppe Ib sind folgende Gruppen Ic, Id, Ie und If interessant.

Gruppe Ic:

Verbindungen der Formel I, worin
R für $C_1$-$C_6$-Alkyl steht;
$R_2$ für Methyl, Ethyl, Isopropyl oder sek.-Butyl steht;
$R_1$ die Gruppe

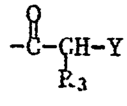

$R_3$ für Wasserstoff, Fluor oder Methyl steht; und
Y Chlor, Brom, Jod, Benzolsulfonyloxy, Paratosyloxy oder Mesyloxy bedeutet.

Gruppe Id:

Verbindungen der Formel I, worin
R für $C_1$-$C_6$-Alkyl steht;
$R_2$ für Methyl, Ethyl, Isopropyl oder sek.-Butyl steht;
$R_1$ die Gruppe

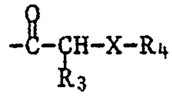

repräsentiert; wobei
X für Sauerstoff oder Schwefel steht;
$R_3$ Wasserstoff, Fluor oder Methyl bedeutet; und
$R_4$ für Wasserstoff, für unsubstituiertes oder durch Fluor, Chlor, Brom, Hydroxy, $C_1$-$C_3$-Alkoxy oder $C_1$-$C_3$-Haloalkoxy substituiertes $C_1$-$C_6$-Alkyl, für einen unsubstituierten oder substituierten Rest ausgewählt aus der Reihe $C_3$-$C_7$-Cycloalkyl, $C_2$-$C_4$-Alkenyl und $C_2$-$C_4$-Alkinyl, wobei die Substituenten ausgewählt sind aus der Reihe Fluor, Chlor, Brom, Hydroxy, $C_1$-$C_3$-Alkoxy und $C_1$-$C_4$-Alkanoyloxy, für unsubstituiertes oder Fluor, Chlor, Brom, Methyl, Ethyl, Methoxy, Halomethoxy, Trifluormethyl und/oder Nitro substituiertes Phenyl oder für einen unsubstituierten oder substituierten, ungesättigten oder gesättigten, fünf-oder sechsgliedrigen heterocyclischen Ring mit ein bis drei Heteroatomen, ausgewählt aus der Reihe Stickstoff, Sauerstoff und Schwefel, dessen Substituenten ausgewählt sind aus der Reihe Oxo, Halogen, $C_1$-$C_4$-Alkyl, $C_1$-$C_4$-Haloalkyl und $C_1$-$C_4$-Alkoxy steht, unter Einschluss der Säureadditionssalze und Metallkomplexe.

Gruppe Ie:

Verbindungen der Formel I, worin
R für $C_1$-$C_6$-Alkyl steht;
$R_2$ für Methyl, Ethyl, Isopropyl oder sek.-Butyl steht;
$R_1$ die Gruppe

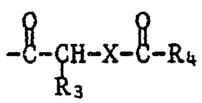

repräsentiert; wobei
X für Sauerstoff oder Schwefel steht;
$R_3$ Wasserstoff, Fluor oder Methyl bedeutet; und
$R_4$ für Wasserstoff, für unsubstituiertes oder durch Fluor, Chlor, Brom, Hydroxy, $C_1$-$C_3$-Alkoxy oder $C_1$-$C_3$-Haloalkoxy substituiertes $C_1$-$C_6$-Alkyl, für einen unsubstituierten oder substituierten Rest ausgewählt aus der Reihe $C_3$-$C_7$-Cycloalkyl, $C_2$-$C_4$-Alkenyl und $C_2$-$C_4$-Alkinyl, wobei die Substituenten ausgewählt sind aus der Reihe Fluor, Chlor, Brom, Hydroxy, $C_1$-$C_3$-Alkoxy und $C_1$-$C_4$-Alkanoyloxy, für unsubstituiertes oder Fluor, Chlor, Brom, Methyl, Ethyl, Methoxy, Halomethoxy, Trifluormethyl und/oder Nitro substituiertes Phenyl oder für einen unsubstituierten oder substituierten, ungesättigten oder gesättigten, fünf-oder sechsgliedrigen heterocyclischen Ring mit ein bis drei Heteroatomen, ausgewählt aus der Reihe Stickstoff, Sauerstoff und Schwefel, dessen Substituenten ausgewählt sind aus der Reihe Oxo, Halogen, $C_1$-$C_4$-Alkyl, $C_1$-$C_4$-Haloalkyl und $C_1$-$C_4$-Alkoxy steht, unter Einschluss der Säureadditionssalze und Metallkomplexe.

Gruppe If:

Verbindungen der Formel I, worin
R für $C_1$-$C_6$-Alkyl steht;
$R_2$ für Methyl, Ethyl, Isopropyl oder sek.-Butyl steht;
$R_1$ die Gruppe

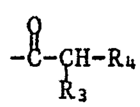

repräsentiert; wobei
$R_3$ für Wasserstoff, Fluor oder Methyl steht; und

$R_4$ für einen in 1-Position verknüpften, 5-gliedrigen Heteroaromaten mit 2 bis 3 Stickstoffatom steht, der unsubstituiert oder ein-bis dreifach durch $C_1$-$C_6$-Alkyl substituiert ist, steht, unter Einschluss der Säureadditionssalze mit organischen und anorganischen Säuren sowie der Metallkomplexe mit Metallkationen der ersten, zweiten, vierten oder achten Nebengruppe des Periodensystems der Elemente.

Bevorzugt werden auch folgende Substanzen:

<u>Gruppe Ig:</u>

Verbindungen der Formel I, worin
R für $C_1$-$C_6$-Alkyl steht;
$R_2$ für Methyl, Ethyl, Isopropyl oder sek.-Butyl steht;
$R_1$ eine der Acylgruppen

a) $-\overset{O}{\overset{\|}{C}}-\underset{R_3}{\underset{|}{C}H}-Y$ ,

b) $-\overset{O}{\overset{\|}{C}}-\underset{R_3}{\underset{|}{C}H}-X-R_4$ ,

c) $-\overset{O}{\overset{\|}{C}}-\underset{R_3}{\underset{|}{C}H}-X-\overset{O}{\overset{\|}{C}}-R_4$ oder

d) $-\overset{O}{\overset{\|}{C}}-\underset{R_3}{\underset{|}{C}H}-R_4$ repräsentiert, wobei

X für Sauerstoff oder Schwefel steht;
Y Halogen bedeutet:
$R_3$ Wasserstoff, Halogen oder Methyl repräsentiert; und
$R_4$ für Wasserstoff, unsubstituiertes oder durch Halogen oder $C_1$-$C_4$-Alkoxy substituiertes $C_1$-$C_4$-Alkyl steht, für Cyclopropyl, für Cyclopentyl, für Cyclohexyl, für unsubstituiertes oder durch Fluor, Chlor, Brom, Methoxy, Trifluormethyl, Methyl und/oder Nitro substituiertes Phenyl für 4H-2,3-Dihydropyran-2-yl oder für einen in 1-Position verknüpften, 5-gliedrigen Heteroaromaten mit 2 bis 3 Stickstoffatom steht, der unsubstituiert oder einbis dreifach durch $C_1$-$C_6$-Alkyl substituiert ist, steht, unter Einschluss der Säureadditionssalze mit organischen und anorganischen Säuren sowie der Metallkomplexe mit Metallkationen der ersten, zweiten, vierten oder achten Nebengruppe des Periodensystems der Elemente.

Besonders bevorzugte Einzelsubstanzen der Formel I sind beispielsweise:
5-O-Chloracetyl-13β-methyl-milbemycin D,
5-O-Chloracetyl-13β-methyl-milbemycin $A_3$,
5-O-Chloracetyl-13β-methyl-milbemycin $A_4$,
5-O-Chloracetyl-13β-ethyl-milbemycin D,
5-O-Chloracetyl-13β-ethyl-milbemycin $A_3$,
5-O-Chloracetyl-13β-ethyl-milbemycin $A_4$,
5-O-Acetoxyacetyl-13β-methyl-milbemycin D,
5-O-Acetoxyacetyl-13β-methyl-milbemycin $A_3$,
5-O-Acetoxyacetyl-13β-methyl-milbemycin $A_4$,
5-O-Acetoxyacetyl-13β-ethyl-milbemycin D,
5-O-Acetoxyacetyl-13β-ethyl-milbemycin $A_3$,
5-O-Acetoxyacetyl-13β-ethyl-milbemycin $A_4$,
5-O-[3,4-Dihydro-2H-pyran-2-yl]carboxyacetyl-13β-methyl-milbemycin D,
5-O-[3,4-Dihydro-2H-pyran-2-yl]carboxyacetyl-13β-methyl-milbemycin $A_3$,
5-O-[3,4-Dihydro-2H-pyran-2-yl]carboxyacetyl-13β-methyl-milbemycin $A_4$,
5-O-[3,4-Dihydro-2H-pyran-2-yl]carboxyacetyl-13β-ethyl-milbemycin D,
5-O-[3,4-Dihydro-2H-pyran-2-yl]carboxyacetyl-13β-ethyl-milbemycin $A_3$,
5-O-[3,4-Dihydro-2H-pyran-2-yl]carboxyacetyl-13β-ethyl-milbemycin $A_4$,
5-O-[1,2,4-Triazol-4'-yl]acetyl-13β-methyl-milbemycin D,
5-O-[1,2,4-Triazol-4'-yl]acetyl-13β-methyl-milbemycin $A_3$,

5-O-[1,2,4-Triazol-4'-yl]acetyl-13β-methyl-milbemycin $A_4$,
5-O-[1,2,4-Triazol-4'-yl]acetyl-13β-ethyl-milbemycin D,
5-O-[1,2,4-Triazol-4'-yl]acetyl-13β-ethyl-milbemycin $A_3$,
5-O-[1,2,4-Triazol-4'-yl]acetyl-13β-ethyl-milbemycin $A_4$,
5-O-Methoxyacetyl-13β-methyl-milbemycin D,
5-O-Methoxyacetyl-13β-methyl-milbemycin $A_3$,
5-O-Methoxyacetyl-13β-methyl-milbemycin $A_4$,
5-O-Methoxyacetyl-13β-ethyl-milbemycin D,
5-O-Methoxyacetyl-13β-ethyl-milbemycin $A_3$,
5-O-Methoxyacetyl-13β-ethyl-milbemycin $A_4$,
5-O-[3-Chlorbenzoyloxy]acetyl-13β-methyl-milbemycin D,
5-O-[3-Chlorbenzoyloxy]acetyl-13β-methyl-milbemycin $A_3$,
5-O-[3-Chlorbenzoyloxy]acetyl-13β-methyl-milbemycin $A_4$,
5-O-[3-Chlorbenzoyloxy]acetyl-13β-ethyl-milbemycin D,
5-O-[3-Chlorbenzoyloxy]acetyl-13β-ethyl-milbemycin $A_3$,
5-O-[3-Chlorbenzoyloxy]acetyl-13β-ethyl-milbemycin $A_4$,

Bemerkenswert sind ferner Verbindungen der Formel I ausgewählt aus der Reihe:

5-O-Acetoxyacetyl-13β-propyl-milbemycin D,
5-O-Acetoxyacetyl-13β-propyl-milbemycin $A_3$,
5-O-Acetoxyacetyl-13β-propyl-milbemycin $A_4$,
5-O-Acetyl-13β-ethyl-milbemycin D,
5-O-Acetyl-13β-ethyl-milbemycin $A_3$,
5-O-Acetyl-13β-ethyl-milbemycin $A_4$,
5-O-Acetyl-13β-methyl-milbemycin D,
5-O-Acetyl-13β-methyl-milbemycin $A_3$,
5-O-Acetyl-13β-methyl-milbemycin $A_4$,
5-O-Methoxyacetyl-13β-butyl-milbemycin D,
5-O-Methoxyacetyl-13β-butyl-milbemycin $A_3$,
5-O-Methoxyacetyl-13β-butyl-milbemycin $A_4$,
5-O-Benzoyloxy-13β-methyl-milbemycin D,
5-O-Benzoyloxy-13β-methyl-milbemycin $A_3$,
5-O-Benzoyloxy-13β-methyl-milbemycin $A_4$,

Die vorliegende Erfindung betrifft auch Verfahren, die es erlauben in der 5-O-Position von 13β-Alkyl-Milbemycin-oder 13-Deoxi-13β-Alkyl-22,23-dihydro-Avermectin-aglykon-Derivaten eine 5-O-Acyl-Gruppe gezielt einzuführen und damit zu den hochwirksamen neuen Parasitiziden und Insektiziden der Formel I zu gelangen.

Zur Herstellung von Verbindungen der Formel I wird erfindungsgemäss folgendes Verfahren durchgeführt, indem man nämlich ein 13β-Alkyl-Milbemycin-Derivat der Formel II

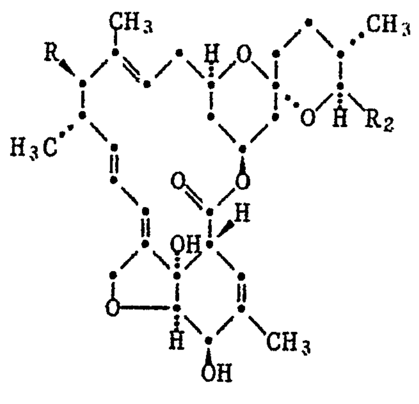

(II)

+ HO-$R_1$ → (I)

(III)

mit einer Säure der Formel III, einem ihrer Säurehalogenide oder ihrem Säureanhydrid an der 5-OH-Gruppe verestert, wobei die Substituenten R, $R_1$ und $R_2$ die unter Formel I angegebenen Bedeutungen haben.

Bevorzugte Säurehalogenide der Säuren III sind deren Chloride und Bromide.

Im Falle der Säurehalogenide und Säureanhydride von III erfolgt die Umsetzung vorzugsweise in einem inerten, hydroxylgruppenfreien Lösungsmittel in Anwesenheit einer organischen Base, wie z.B. Pyridin, 4-Dimethylaminopyridin, Lutidin, Collidin, Trialkylamin, N-Dialkylanilin, oder bicyclische, nicht nucleophile Basen wie z.B. 1,4-Diazabicyclo[2.2.2]octan (DABCO), 1,5-Diazabicyclo[4.3.0]-non-5-en (DBN) oder 1,8-Diazabicyclo[5.4.0]undec-7-en (1,5-5) (DBU). Die Reaktion wird im allgemeinen bei Temperaturen von -30° bis +70°C, vorzugsweise von -10° bis +50°C durchgeführt. Man arbeitet dabei zweckmässigerweise in Gegenwart eines reaktionsinerten Lösungsmittels oder Lösungsmittelgemisches. Es eignen sich hierfür z.B. aliphatische und aromatische Kohlenwasserstoff wie Benzol, Toluol, Xylole, Petrolether, Hexan; halogenierte Kohlenwasserstoffe wie Chlorbenzol, Methylenchlorid, Ethylenchlorid, Chloroform, Tetrachlorkohlenstoff, Tetrachlorethylen; Ether und etherartige Verbindungen wie Dialkylether (Diethylether, Diisopropylether, tert.-Butylmethylether usw.), Anisol, Dioxan, Tetrahydrofuran; Nitrile wie Acetonitril, Propionitril; Ester wie Ethylacetat (Essigsäureethylester), Propylacetat oder Butylacetat; Ketone wie Aceton, Diethylketon, Methylethylketon; Verbindungen wie Dimethylsulfoxid (DMSO), Dimethylformamid (DMF) und Gemische solcher Lösungsmittel untereinander. Man kann die Reaktion aber auch im Ueberschuss einer der oben genannten Basen durchführen.

Geht man von der Säure der Formel III selbst aus, so wird die Veresterungsreaktion II mit III vorteilhafterweise in Gegenwart für Veresterungen üblicher, wasserabspaltenden Reagenzien durchgeführt, wie z.B. in Anwesenheit eines Carbodiimids [Dicyclohexylcarbodiimid (DCC)] oder eines 1-Alkyl-2-halogenpyridiniumsalzes [1-Methyl-2-chlorpyridiniumjodid]. Diese Reaktion wird vorzugsweise in einem der obengenannten, reaktionsinerten Lösungsmittel und bei Temperaturen von -30° bis +70°C, vorzugsweise -10° bis +50°C durchgeführt. Man arbeitet bevorzugt in Gegenwart einer Base wie z.B. in Gegenwart eines organischen Amins z.B. eines Trialkylamins (Trimethylamin, Triethylamin, Tripropylamin, Diisopropylethylamin usw.), eines Pyridins (Pyridin selbst, 4-Dimethylaminopyridin, 4-Pyrrolidylaminopyridin usw.), eines Morpholins (N-Methylmorpholin), eines N,N-Dialkylanilins (N,N-Dimethylanilin, N-Methyl-N-ethylanilin) usw.

Verbindungen der Formel I können aber auch durch geeignete Reaktionen aus anderen Verbindungen der Formel I hergestellt werden.

Beispielsweise lässt sich eine Verbindung mit $R_1$ = $-CO-CH_2Cl$ durch Umsetzung mit Natriumacetat in DMF bei 80°C in eine Verbindung mit $R_1$ = $-CO-CH_2-OCOCH_3$ umwandeln. Diese wiederum lässt sich beispielsweise auch aus einer Verbindung mit $R_1$ = $-CO-CH_2-OH$ durch Veresterung mit Acetylchlorid oder Essigsäureanhydrid herstellen. Derartige Umwandlungen von Verbindungen der Formel I in andere Vertreter der Formel I werden als zum Hauptverfahren gehörig betrachtet.

Das beschriebene Herstellungsverfahren ist einschliesslich aller Teilstufen essentieller Bestandteil der vorliegenden Erfindung.

Die Säuren der Formel III, ihre Halogenide und Anhydride sind grösstenteils bekannt oder lassen sich analog zu den bekannten Vertretern herstellen.

Die 13β-Alkyl-5-hydroxymilbemycin der Formel II lassen sich dadurch herstellen, dass man einen Allylester der Formel IV

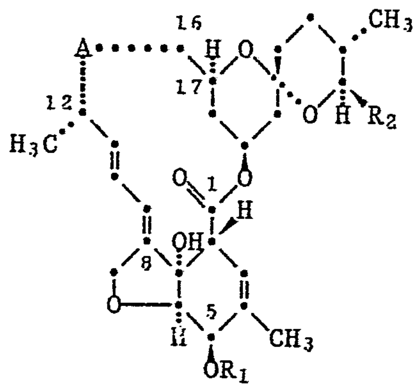

(IV)

worin A für eine der Gruppen a oder b

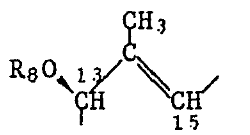 (a) oder 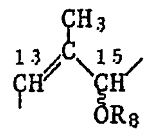 (b)

[= 13β-Ester-$\Delta^{14,15}$] [= $\Delta^{13,14}$-15-Ester]

steht, $R_8$ eine Acylgruppe repräsentiert, $R_1$ Wasserstoff oder vorzugsweise eine Silylgruppe bedeutet und $R_2$ die unter Formel I angegebene Bedeutung hat, mit einer Trialkylaluminium-Verbindung der Formel V

$Al(R)_3$ (V)

behandelt, wobei R die unter Formel I angegebenen Bedeutungen hat, woraufhin man die $R_1$-Silylgruppe, sofern freie 5-Hydroxi-Verbindungen erwünscht sind, hydrolytisch abspaltet.

Das Verfahren wird im allgemeinen in einem reaktions-inerten Lösungsmittel durchgeführt. Geeignete Lösungsmittel sind z.B.: Ether und etherartige Verbindungen wie Dialkylether (Diethylether, Diisopropylether, tert.-Butylmethylether, Dimethoxyethan, Dioxan, Tetrahydrofuran, Anisol, usw.); halogenierte Kohlenwasserstoffe wie Chlorbenzol, Methylenchlorid, Ethylenchlorid, usw.; oder Sulfoxide wie Dimethylsulfoxid, wobei auch aromatische oder aliphatische Kohlenwasserstoffe, wie Benzol, Toluol, Xylole, Petrolether, Ligroin, Cyclohexan, usw. anwesend sein können. Es kann von Vorteil sein, wenn die Reaktion oder Teilschritte davon unter Schutzgasatmosphäre (z.B. Argon, Helium, Stickstoff, etc.) und/oder in absoluten Lösungsmitteln durchgeführt werden. Gewünschtenfalls können Zwischenprodukte aus dem Reaktionsmedium isoliert und falls erwünscht, vor der Weiterreaktion, auf übliche Art und Weise gereinigt werden, z.B. durch Waschen, Digerieren, Extraktion, Umkristallisation, Chromatographie usw. Man kann jedoch auch auf derartige Reinigungsschritte verzichten und diese erst mit entsprechenden Endprodukten durchführen.

Zur Einführung der 13β-Alkylgruppe geeigneten Trialkylaluminium-Verbindungen sind ($C_1$-$C_{10}$-Alkyl)-$_3$Aluminium-Verbindungen, wie z.B. Trimethylaluminium, Triethylaluminium, Triisobutylaluminium, Trihexylaluminium usw.. Die Reaktion wird im allgemeinen im Temperaturbereich -100°C bis 100°C, bevorzugt bei -20°C bis +60°C durchgeführt. Die Trialkyl-Aluminiumverbindung der Formel V wird dabei in Substanz oder in einem reaktionsinerten Lösungsmittel wie z.B. Hexan, Toluol oder Benzol in mindestens equimolarer Menge zur Lösung der Verbindung der Formel II gegeben.

Nach erfolgter Reaktion wird die Silyl-Schutzgruppe zweckmässigerweise durch Behandlung der Verbindungen der Formel I mit einer verdünnten Säure wie z.B. mit 1 proz. p-Toluolsulfonsäure in Methanol oder mit einer wässrigen HF-Lösung in Acetonitril im Temperaturbereich -20°C bis 50°C, bevorzugt bei 0°C bis 30°C oder mit Pyridiniumfluorid in Pyridin wieder abgespalten.

Die Trialkylaluminium-Verbindungen der Formel V sind allgemein bekannt oder lassen sich analog zu den bekannten Vertretern herstellen.

Die als Ausgangssubstanzen eingesetzten Ester der Formel IV können aus den zugrundeliegenden Allylalkoholen der Formel VI

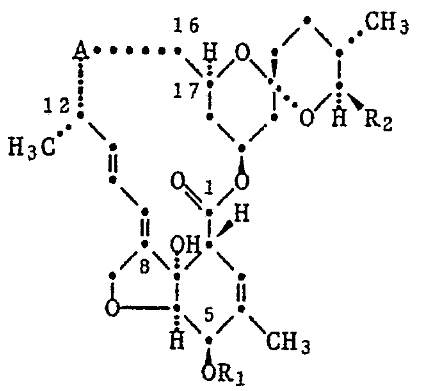

(VI)

worin A für eine der Gruppen a oder b

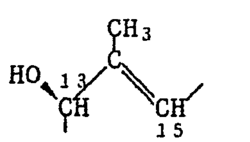 (a) oder 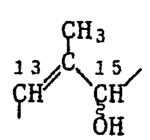 (b)

[= 13β-Hydroxi-$\Delta^{14,15}$] [= $\Delta^{13,14}$-15-Hydroxi]

steht $R_2$ die unter Formel I angegebenen Bedeutungen hat und $R_1$ für Wasserstoff oder eine Silylgruppe

steht; durch übliche, literaturbekannte Acylierungsmethoden wie z.B. durch Reaktionen mit Säurechloriden - ($R_8COCl$) oder Säureanhydriden ($(R_8CO)_2O$, wobei $R_8$ die unter Formel IV angegebenen Bedeutungen hat, in Gegenwart einer Base (Triethylamin, Pyridin, N,N-Dimethylaminopyridin usw.) in einem eingangs genannten inerten Lösungsmittel z.B. Dichlormethan, Chloroform, usw. im Temperaturbereich von -20°C bis 100°C, bevorzugt bei 0°C bis 70°C hergestellt werden.

Die Verbindungen der Formel VIb [= $\Delta^{13,14}$-15-Hyroxi] lassen sich aus 14,15-Epoxi-Milbemycinen der Formel VII gewinnen, worin $R_1$ für Wasserstoff oder eine Silylgruppe steht und $R_2$ die für die Formel I genannten Bedeutungen hat,

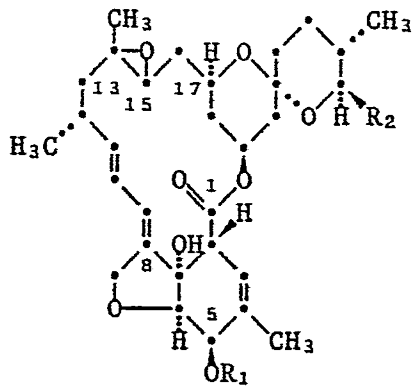

(VII)

mit Hilfe des Komplex-Reagenzes $[HN_3]_x/Al(Ethyl)_3]_y$, worin x und y unabhängig voneinander die Zahl 1 oder 2 oder einen Zahlenwert zwischen 1 und 2 darstellen, in inerten trockenen Lösungsmitteln im Temperaturbereich von -20° bis +150°C, vorzugsweise +20° bis +80°C.

Als inerte Lösungsmittel kommen vorzugsweise aliphatische und aromatische Kohlenwasserstoffe wie Benzol, Toluol, Xylol, Petrolether; Ether wie Diethylether, tert.Butylmethylether, Tetrahydrofuran, Dioxan, Anisol in Frage.

Die Reaktion wird vorteilhaft unter Schutzgas, wie Stickstoff oder Argon, durchgeführt.

Stickstoffwasserstoffsäure $HN_3$ lässt sich in statu nascendi in den $[HN_3]_x/[Al(Et)_3]_y$-Komplex überführen, indem man im vorgesehenen trockenen Lösungsmittel oder Lösungsmittel-Gemisch Na-Azid suspendiert und daraus mit einer stärkeren Säure, z.B. $H_2SO_4$ (bevorzugt Oleum, um absolut trockene Reaktionsbedingungen zu gewährleisten), $HN_3$ in der Lösung in Freiheit setzt. $Al(Et)_3$ sollte in der Lösung bereits vorliegen oder kurz danach zugegeben werden. Die zur Reaktion vorgesehene Epoxi-Verbindung kann gleichfalls bereits vorliegen oder zu einem geeigneten Zeitpunkt zur Lösung dosiert werden.

Die zur Herstellung der Verbindungen VIb verwendeten Ausgangsverbindungen der Formel VII lassen sich leicht herstellen durch Epoxidierung der aus der US-PS 3,950,360 bekanntgewordenen und ursprünglich als "Antibiotika B-41-A", später als "Milbemycin-A"-Verbindungen und der aus der US-PS 4,346,171 bekanngewordenen und als "B-41-D" oder "Milbemycin-D" bezeichneten Verbindungen der Formel sowie der aus der US-PS 4,173,571 bekanntgewordenen 13-Deoxi-22,23-dihydro-Avermectine ($R_2$ = sec.Butyl) der nachstehenden Formel VIII

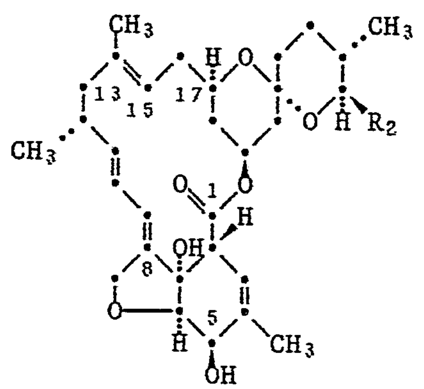

(VIII)

$R_2$ = $CH_3$ Milbemycin $A_3$
$R_2$ = $C_2H_5$ Milbemycin $A_4$
$R_2$ = iso$C_3H_7$ Milbemycin D
$R_2$ = sec.$C_4H_9$ 13-Deoxi-22,23-dihydro-C-076-Bla-aglycon.

Die Epoxidierung wird in einer Lösungsmittelphase im Temperaturbereich von -10° bis +20°C, vorzugsweise -5° bis +5°C, durchgeführt.

Die Epoxidierung wird mit Persäuren wie Peressigsäure, Trifluorperessigsäure, Perbenzoesäure, Chlorperbenzoesäure durchgeführt.

Die 13$\beta$-Hydroxi-$\Delta^{14,15}$-Verbindungen der Formel VIa lassen sich aus Verbindungen der Formel IVb, worin $R_1$ für eine Schutzgruppe steht, durch Reaktion mit Pyridiniumdichromat [= $(Pyr)_2Cr_2O_7$] herstellen. Man arbeitet hierbei in Dimethylformamid und bei Temperaturen zwischen ca. -10° und +60°C. Die $R_1$-Schutzgruppe wird, sofern gewünscht, anschliessend hydrolytisch abgespalten.

Durch Acylierung oder Silylierung der 5-OH-Gruppe werden alle jene Derivate der Formeln II bis VIII hergestellt, bei denen $R_1$ eine andere Bedeutung als Wasserstoff ($R_1$ = OH-Schutzgruppe) hat. Zur Silylierung verwendet man zweckmässigerweise ein Silan der Formel Y-Si$(R_5)(R_6)(R_7)$, wobei $R_5$, $R_6$ und $R_7$ vorzugsweise unabhängig voneinander für $C_1$-$C_6$-Alkyl, Benzyl oder Phenyl stehen und beispielsweise eine der Gruppen Trimethylsilyl, tris(tert.Butyl)silyl, Thexyldimethylsilyl, Diphenyl-tert.butylsilyl, bis(Isopropyl)-methylsilyl, Triphenylsilyl usw. und insbesondere tert.Butyl-dimethylsilyl bildet. Die 5-OH-Gruppe kann auch als Benzylether oder Methoxiethoximethylether vorliegen, und wobei Y eine Silylabgangsgruppe bedeutet. Zu den Silylabgangsgruppen Y zählen beispielsweise Bromid, Chlorid, Cyanid, Azid, Acetamid, Trifluoracetat, Trifluormethansulfonat. Diese Aufzählung stellt keine Limitierung dar, der Fachmann kennt weitere typische Silylabgangsgruppen.

5-O-Silylierungen werden in wasserfreiem Milieu, vorzugsweise in inerten Lösungsmitteln und besonders bevorzugt in aprotischen Lösungsmitteln durchgeführt. Die Reaktion läuft vorteilhaft im Temperaturbereich von 0° bis +80°C, bevorzugt bei +10° bis +40°C, ab. Vorzugsweise wird eine organische Base zugegeben. Es kommen als solche beispielsweise tertiäre Amine wie Triethylamin, Triethylendiamin, Triazol und bevorzugt Pyridin, Imidazol oder 1,8-Diazabicyclo[5.4.0]-undec-7-en (DBU) in Frage.

Die Entfernung dieser Silylreste $R_1$ in der 5-Position geschieht durch selektive milde Hydrolyse ($\rightarrow$ $R_1$=H) mit z.B. Arylsulfonsäure in alkoholischer Lösung oder nach einer anderen dem Fachmann geläufigen Methode.

Das beschriebene Verfahren zur Herstellung der Verbindungen der Formel I ist in allen Teilschritten ein Bestandteil vorliegender Erfindung.

Ein weiterer Gegenstand vorliegender Erfindung betrifft Schädlingsbekämpfungsmittel gegen Ekto-und Endoparasiten sowie Schadinsekten, die neben üblichen Trägerstoffen und/oder Verteilungsmitteln als mindestens einen Wirkstoff eine Verbindung der Formel I enthalten.

Die Verbindungen der Formel I eignen sich ausgezeichnet zur Bekämpfung von Schädlingen an Tieren und Pflanzen, darunter tierparasitären Ekto-Parasiten. Zu letzteren zählen unter der Ordnung Acarina insbesondere Schädlinge der Familien Ixodidae, Dermanyssidae, Sarcoptidae, Psoroptidae; die Ordnungen Mallophaga; Siphonaptera, Anoplura (z.B. Familie der Haemotopinidae); unter der Ordnung Diptera insbesondere Schädlinge der Familien Muscidae, Calliphoridae, Oestridae, Tabanidae, Hippoboscidae, Gastrophilidae.

Die Verbindungen I sind auch einsetzbar gegen Hygiene-Schädlinge, insbesondere der Ordnungen Diptera mit den Familien Sarcophagidae, Anophilidae, Culicidae; der Ordnung Orthoptera, der Ordnung Dictyoptera (z.B. Familie Blattidae) und der Ordnung Hymenoptera (z.B. Familie Formicidae).

Die Verbindungen I besitzen auch nachhaltige Wirksamkeit bei pflanzenparasitären Milben und Insekten. Bei Spinnmilben der Ordnung Acarina sind sie wirksam gegen Eier, Nymphen und Adulte von Tetranychidae (Tetranychus spp. und Panonychus spp.).

Hohe Aktivität besitzen sie bei den saugenden Insekten der Ordnung Homoptera, insbesondere gegen Schädlinge der Familien Aphidiae, Delphacidae, Cicadellidae, Psyllidae, Loccidae, Diaspididae und Eriophydidae (z.B. die Rostmilbe auf Citrusfrüchten): Der Ordnungen Hemiptera; Heteroptera und Thysanoptera; sowie bei den pflanzenfressenden Insekten der Ordnungen Lepidoptera; Coleoptera; Diptera und Orthoptera.

Sie sind ebenfalls als Bodeninsektizid gegen Schädlinge im Erdboden geeignet.

Die Verbindungen der Formel I sind daher gegen alle Entwicklungsstadien saugender und fressender Insekten an Kulturen wie Getreide, Baumwolle, Reis, Mais, Soja, Kartoffeln, Gemüse, Früchten, Tabak, Hopfen, Citrus, Avocados und anderen wirksam.

Die Verbindungen der Formel I sind auch wirksam gegen Pflanzen-Nematoden der Arten Meloidogyne, Heterodera, Pratylenchus, Ditylenchus, Radopholus, Rizoglyphus und andere.

Besonders aber sind die Verbindungen gegen Helminthen wirksam, unter denen die endoparasitären Nematoden die Ursache schwerer Erkrankungen an Säugetieren und Geflügel sein können, z.B. an Schafen, Schweinen, Ziegen, Rindern, Pferden, Eseln, Hunden, Katzen, Meerschweinchen, Ziervögeln. Typische Nematoden dieser Indikation sind: Haemonchus, Trichostrongylus, Ostertagia, Nematodirus,

Cooperia, Ascaris, Bunostonum, Oesophagostonum, Charbertia, Trichuris, Strongylus, Trichonema, Dictyocaulus, Capillaria, Heterakis, Toxocara, Ascaridia, Oxyuris, Ancylostoma, Uncinaria, Toxascaris und Parascaris. Der besondere Vorteil der Verbindungen der Formel I ist ihre Wirksamkeit gegen solche Parasiten, die gegen Wirkstoffe aur Benzimidazol-Basis resistent sind.

Gewisse Spezies der Arten Nematodirus, Cooperia und Oesophagostomum greifen den Intestinaltrakt des Wirtstiers an, während andere der Arten Haemonchus und Ostertagia im Magen und solche der Art Dictyocaulus im Lungengewebe parasitieren. Parasiten der Familien Filariidae und Setariidae finden sich im internen Zellgewebe und den Organen, z.B. dem Herzen, den Blutgefässen, den Lymphgefässen und dem subcutanen Gewebe. Hier ist vor allem der Herzwurm des Hundes, Dirofilaria immitis, zu nennen. Die Verbindungen der Formel I sind gegen diese Parasiten hoch wirksam.

Sie sind ferner zur Bekämpfung von humanpathogenen Parasiten geeignet, unter denen als typische, im Verdauungstrakt vorkommende Vertreter solche der Arten Ancylostoma, Necator, Ascaris, Strongyloides, Trichinella, Capillaria, Trichuris und Enterobius zu nennen sind. Wirksam sind die Verbindungen vorliegender Erfindung auch gegen Parasiten der Arten Wuchereria, Brugia, Onchocerca und Loa aus der Familie der Filariidae, die im Blut, im Gewebe und verschiedenen Organen vorkommen, ferner gegen Dracunculus und Parasiten der Arten Strongyloides und Trichinella, die speziell den Gastro-Intestinalkanal infizieren.

Die Verbindungen der Formel I werden in unveränderter Form oder vurzugsweise zusammen mit den in der Formulierungstechnik üblichen Hilfsmitteln eingesetzt und werden daher z.B. zu Emulsionskonzentraten, direkt versprühbaren oder verdünnbaren Lösungen, verdünnten Emulsionen, Spritzpulvern, löslichen Pulvern, Stäubemitteln, Granulaten, auch Verkapselungen in z.B. polymeren Stoffen in bekannter Weise verarbeitet. Die Anwendungsverfahren wie Versprühen, Vernebeln, Verstäuben, Verstreuen oder Giessen werden gleich wie die Art der Mittel den angestrebten Zielen und den gegebenen Verhältnissen entsprechend gewählt.

Die Verbindungen der Formel I werden bei Warmblütern in Aufwandmengen von 0,01 bis 10 mg/kg Körpergewicht angewendet, über geschlossenen Kultur-Anbauflächen, in Pferchen, Stallungen oder sonstigen Räumen in Mengen von 10 g bis 1000 g pro Hektar.

Die Formulierungen, d.h. die den Wirkstoff der Formel I enthaltenden Mittel, Zubereitungen oder Zusammensetzungen werden in bekannter Weise hergestellt, z.B. durch inniges Vermischen und/oder Vermahlen der Wirkstoffe mit Streckmitteln, wie z.B. mit Lösungsmitteln, festen Trägerstoffen, und gegebenenfalls oberflächenaktiven Verbindungen (Tensiden).

Als Lösungsmittel können in Frage kommen: Aromatische Kohlenwasserstoffe, bevorzugt die Fraktionen $C_8$ bis $C_{12}$, wie z.B. Xylolgemische oder substituierte Naphthaline, Phthalsäureester wie Dibutyl-oder Dioctylphthalat, aliphatische Kohlenwasserstoffe wie Cyclohexan oder Paraffine, Alkohole und Glykole sowie deren Ether und Ester, wie Ethanol, Ethylenglykol, Ethylenglykolmonomethyl-oder -ethylether, Ketone wie Cyclohexanon, stark polare Lösungsmittel wie N-Methyl-2-pyrrolidon, Dimethylsulfoxid oder Dimethylformamid, sowie gegebenenfalls epoxidierte Pflanzenöle, wie epoxidiertes Kokosnussöl oder Sojaöl; oder Wasser.

Als feste Trägerstoffe, z.B. für Stäubemittel und dispergierbare Pulver, werden in der Regel natürliche Gesteinsmehle verwendet, wie Calcit, Talkum, Kaolin, Montmorillonit oder Attapulgit. Zur Verbesserung der physikalischen Eigenschaften können auch hochdisperse Kieselsäure oder hochdisperse saugfähige Polymerisate zugesetzt werden. Als gekörnte, adsorptive Granulatträger kommen poröse Typen wie z.B. Bimsstein, Ziegelbruch, Sepiolit oder Bentonit, als nicht sorptive Trägermaterialien z.B. Calcit oder Sand in Frage. Darüberhinaus kann eine Vielzahl von vorgranulierten Materialien anorganischer oder organischer Natur wie insbesondere Dolomit oder zerkleinerte Pflanzenrückstände verwendet werden.

Als oberflächenaktive Verbindungen kommen je nach der Art des zu formulierenden Wirkstoffes nichtionogene, kation-und/oder anionaktive Tenside mit guten Emulgier-, Dispergier-und Netzeigenschaften in Betracht. Unter Tensiden sind auch Tensidgemische zu verstehen.

Geeignete anionische Tenside können sowohl sog. wasserlösliche Seifen als auch wasserlösliche synthetische oberflächenaktive Verbindungen sein.

Als Seifen seien die Alkali-, Erdalkali-oder gegebenenfalls substituierte Ammoniumsalze von höheren Fettsäuren ($C_{10}$-$C_{22}$), wie z.B. die Na-oder K-Salze der Oel-oder Stearinsäure, oder von natürlichen Fettsäuregemischen, die z.B. aus Kokosnuss-oder Talgöl gewonnen werden können, genannt. Ferner sind auch die Fettsäure-methyl-taurinsalze zu erwähnen.

Häufiger werden jedoch sogenannte synthetische Tenside verwendet, insbesondere Fettsulfonate, Fettsulfate, sulfonierte Benzimidazolderivate oder Alkylarylsulfonate.

Die Fettsulfonate oder -sulfate liegen in der Regel als Alkali-, Erdalkali-oder gegebenenfalls substituierte Ammoniumsalze vor und weisen einen Alkylrest mit 8 bis 22 C-Atomen auf, wobei Alkyl auch den Alkylteil von Acylresten einschliesst, z.B. das Na-oder Ca-Salz der Ligninsulfonsäure, des Dodecylschwefelsäureesters oder eines aus natürlichen Fettsäuren hergestellten Fettalkoholsulfatgemisches. Hierher gehören auch die Salze der Schwefelsäureester und Sulfonsäuren von Fettalkohol-Aethylenoxid-Addukten. Die sulfonierten Benzimidazolderivate enthalten vorzugsweise 2-Sulfonsäuregruppen und einen Fettsäurerest mit 8 bis 22 C-Atomen. Alkylarylsulfonate sind z.B. die Na-, Ca-oder Triäthanolaminsalze der Dodecylbenzolsulfonsäure, der Dibutylnaphthalinsulfonsäure oder eines Naphthalinsulfonsäure-Formaldehydkondensationsproduktes.

Ferner kommen auch entsprechende Phosphate wie z.B. Salze des Phosphorsäureesters eines p-Nonylphenol-(4-14)-Aethylenoxid-Adduktes oder Phospholipide in Frage.

Die in der Formulierungstechnik gebräuchlichen Tenside sind u.a. in folgender Publikation beschrieben:
"Mc Cutcheon's Detergents and Emulsifiers Annual"
MC Publishing Corp., Ridgewood, New Jersey, 1982.

Die pestiziden Zubereitungen enthalten in der Regel 0,01 bis 95 %, insbesondere 0,1 bis 80 %, Wirkstoff der Formel I, 5 bis 99,99 % eines festen oder flüssigen Zusatzstoffes und 0 bis 25 %, insbesondere 0,1 bis 25%, eines Tensides.

Während als Handelsware eher konzentrierte Mittel bevorzugt werden, verwendet der Endverbraucher in der Regel verdünnte Mittel mit 1-10'000 ppm Wirkstoffgehalt.

Ein weiterer Gegenstand vorliegender Erfindung betrifft daher Schädlingsbekämpfungsmittel, die neben üblichen Trägerstoffen und/oder Verteilungsmitteln als mindestens einen Wirkstoff eine Verbindung der Formel I enthalten.

Die Mittel können auch weitere Zusätze wie Stabilisatoren, Entschäumer, Viskositätsregulatoren, Bindemittel, Haftmittel sowie Dünger oder andere Wirkstoffe zur Erzielung spezieller Effekte enthalten.

## Herstellungsbeispiele

### Herstellung von Ausgangs-und Zwischenprodukten

#### Beispiel A1: Herstellung von 14,15-Epoxi-milbemycin D (Formel VII)

Zu einer Lösung von 550 mg Milbemycin D in 5 ml Dichlormethan wird unter Eiskühlung eine Lösung von 170 mg Chlorperbenzoesäure in 5 ml Dichlormethan gegeben. Nach 1-stündigem Rühren bei 0° bis +5°C werden nochmals 170 mg des Oxidationsmittels hinzugefügt und weitere 30 min gerührt. Nach beendeter Reaktion wird die Lösung in eine eisgekühlte Lösung von Natriumsulfit gegossen und mit Essigsäureethylester extrahiert. Die vereinigten Extrakte werden einmal mit Wasser gewaschen, getrocknet und im Vakuum eingedampft. Das Rohprodukt wird durch Chromatographierung über ein Silicagel-Säule - (Elutionsmittel n-Hexan/Essigsäureethylester 20:15) gereinigt. Es werden 450 mg 14,15-Epoxi-milbemycin D als amorphe, weisse Substanz erhalten.

#### Beispiel A2: Herstellung von ~~15-Hydroxi-$\Delta^{13,}$~~ $^{14}$-milbemycin D (Formel VIb)

Es werden bei -20°C zu einer Lösung von 2,1 ml (1,75 g, 15,3 mmol) Triethylaluminium in 8,5 ml abs. Diethylether 9,5 ml (0,41 g, 9,53 mmol) einer 6,69 %igen Lösung von $HN_3$ in Diethylether gegeben, die dann bei -10°C unter stark exothermer Reaktion zu 1,8 g (3,15 mmol) 14,15-Epoxi-milbemycin D (in Substanz) gegeben wird. Nach 1 Stunde bei Raumtemperatur werden 4 ml absoluter Ether zugegeben und das gallertartige Reaktionsgemisch wird kräftig gerührt. Nach 4 Stunden wird wie in Vorschrift A1 aufgearbeitet und die Chromatographie an 70 g Kieselgel ($CH_2Cl_2$/Aceton 10:1) ergibt 200 mg (10 %) 14-Azido-15-hydroxi-milbemycin D und 820 g (45 %) 15-Hydroxy-$\Delta^{13,14}$-milbemycin D, Smp.: 151-153°C (aus Methanol).

Beispiel A3: Herstellung von 5-O-t-Butyldimethylsilyl-14,15-epoxi-milbemycin D (Formel VII)

Eine Lösung von 2,21 g (3,86 mmol) 14,15-Epoxi-milbemycin D, 757 mg (5,02 mmol) t-Butyldimethylchlorsilan und 342 mg (5,02 mmol) Imidazol in 4 ml DMF wird 90 min bei Raumtemperatur gerührt. Anschliessend werden 80 ml Diethylether zugegeben, und das Gemisch wird über 20 g Kieselgel filtriert und eingeengt. Es werden 2,65 g (100 %) 5-O-t-Butyldimethylsilyl-14,15-epoxi-milbemycin D erhalten.
$^1$H-NMR (300 MHz. Lösungsmittel $CDCl_3$. Messwerte $\delta$ bezogen auf $Si(CH_3)_4$ = TMS).
0,12 ppm (s) ($(CH_3)_2Si$-O-)
0,92 ppm (s) ((t.-$C_4H_9$)Si-O-)
1,23 ppm (breites s) $C_{14}CH_3$, d.h. Signal der $CH_3$-Gruppe in 14-Position);
2,56 ppm (d; J = 9) ($C_{15}H$, d.h. Signal des Protons in 15-Position).

Aehnlich lässt sich durch Reaktion mit Trimethylsilyl-trifluor-methansulfonat das entsprechende 5-O-Trimethylsilyl-14,15-epoxi-milbemycin D herstellen, Smp. 92-97°C.

Beispiel A4: Herstellung von 5-O-t-Butyldimethylsilyl-15-hydroxi-$\Delta^{13,14}$-milbemycin D (Formel VIb)

Eine Lösung des $HN_3/Et_3Al$-Komplex-Reagenz (hergestellt aus einer Lösung von 4,97 ml Triethylaluminium in 7 ml abs. Tetrahydrofuran (THF) und 9,15 ml einer 2,39 M Lösung von $HN_3$ (21,9 mmol) in abs. Diethylether) wird unter Argon zu einer Lösung von 5,0 g (7,29 mmol) 5-O-Butyldimethylsilyl-14,15-epoximilbemycin D in ca. 20 ml abs. THF gegeben, und das Gemisch wird 15 Stunden unter Rückfluss erhitzt. Anschliessend werden bei Raumtemperatur 250 ml Ether, 2 ml Methanol und schliesslich ein Gemisch von 10 g $Na_2SO_4$•10 $H_2O$ und 10 g Celite zugegeben. Das Gemisch wird filtriert, eingeengt, und die Chromatographie des Rohproduktes an 160 g Kieselgel (0 bis 30 % Essigsäureethylester in Hexan) ergibt 2,37 g (47 %) 5-O-t-Butyldimethylsilyl-15-hydroxi-$\Delta^{13,14}$-milbemycin D.
$^1$H-NMR (300 MHz, $CDCl_3$):
1,59 ppm(d; J = 1 Hz), ($C_{14}CH_3$);
4,06 ppm (dd; $J_1$ = 11 Hz; $J_2$ = 4) ($C_{15}H$);
5,15 ppm (d; J = 8 Hz) ($C_{13}H$):

Daneben werden 109 mg (2 %) 13$\beta$-Azido-5-O-t-butyldimethylsilyl-Milbemycin D gewonnen.

Beispiel A5: Herstellung von 14,15-Epoxi-milbemycin $A_4$ ($R_2 = C_2H_5$) [Formel VII]

Zu einer Lösung von 5,7 g (10,5 mmol) Milbemycin $A_4$ in 140 ml Dichlormethan und 120 ml 0,5 M $NaHCO_3$-Lösung wird bei Raumtemperatur eine Lösung von 2,43 g (14,08 mmol) m-Chlorobenzopersäure in 70 ml Dichlormethan tropfenweise zugegeben. Das Gemisch wird 1 Stunde bei Raumtemperatur kräftig gerührt und dann mit 300 ml Dichlormethan verdünnt. Die organische Phase wird mit wässriger $NaHCO_3$-Lösung gewaschen, mit $Na_2SO_4$ getrocknet und eingeengt. Es werden 5,7 g Epoxid als Rohprodukt erhalten.

Beispiel A6: Herstellung von 5-O-t-Butyldimethylsilyl-14,15-epoxi-milbemycin $A_4$ (Formel VII)

5,7 g 14,15-Epoxi-milbemycin $A_4$ werden in 10 ml trockenem Dimethylformamid (DMF) gelöst. Bei Raumtemperatur werden 0,63 g (9,16 mmol) Imidazol und 1,4 g (9,34 mmol) t-Butyldimethylchlorsilan zugegeben. Das Gemisch wird 1 Stunde bei Raumtemperatur gerührt und an 150 g Kieselgel chromatographiert (Hexan/Ether 4:1), wobei 2,84 g (40 % d.Th. Ausbeute, bezogen auf Milbemycin $A_4$) des silylierten Epoxi-Derivats erhalten werden.

Beispiel A7: Herstellung von 5-O-t-Butyldimethylsilyl-15-hydroxi-$\Delta^{13,14}$-milbemycin $A_4$ (Formel VIb)

Das Komplex-Reagenz $HN_3$/Al (Ethyl)$_3$ wird wie folgt hergestellt: 2,8 ml (12,2 mmol) $Al(C_2H_5)_3$ in 4 ml abs. THF werden unter Argon bei ca. -20°C langsam mit 5,28 ml (20,4 mmol) einer 10 %igen Lösung von $HN_3$ in abs. Diethylether versetzt. Zu dieser Lösung wird unter Argon eine Lösung von 2,84 g (4,25 mmol) der im Beispiel A6 erhaltenen Verbindung gegeben, und das so erhaltene Gemisch wird 4 Stunden unter Rückfluss erhitzt. Bei Raumtemperatur werden 500 ml Diethylether, 10 g $Na_2SO_4$•10$H_2O$ und 10 g Celite zugegeben, und das Gemisch wird filtriert und eingeengt. Die Chromatographie des Rohproduktes an 100 g

Kieselgel (Hexan/Diethylether 7:2) ergibt 1,72 g (60 % d.Th.) der Titel-Verbindung.
$^1$H-NMR (300 MHz, $CDCl_3$; TMS):
1,59 ppm (br. s) ($C_{14}CH_3$);
4,05 ppm (br. s) ($C_{15}H$);
5,15 ppm (d; J = 6 Hz) ($C_{13}H$).

Daneben werden 0,1 g 13β-Azido-5-O-t-butyldimethylsilyl-milbemycin $A_4$ erhalten.

Beispiel A8: Herstellung von 15-Hydroxi-$\Delta^{13,14}$-milbemycin $A_4$ (Formel VIb)

Die Hydrolyse der im Beispiel A7 genannten Titelverbindung mit einer 1 %igen Lösung von p-Toluolsulfonsäure in Methanol und die Aufarbeitung in Diethylether mit 5 %iger Na-Hydrogencarbonat-Lösung ergibt die Titel-Verbindung.

Beispiel A9: Herstellung von 14,15-Epoxi-milbemycin $A_3$($R_2$ = $CH_3$) (Formel VII)

Nach der Vorschrift des Beispiels A1 werden aus 220 mg Milbemycin $A_3$ in 5 ml Dichlormethan und 75 mg Chlorperbenzoesäure in 5 ml Dichlormethan bei -2° bis +5°C während 1,5 Stunden und Reinigung über eine Silicagel-Säule 190 mg 14,15-Epoxi-Milbemycin $A_3$ gewonnen.

Beispiel A10 : Herstellung von 5-O-tert.-Butyldimethylsilyl-14,15-epoxi-milbemycin $A_3$ (Formel VII)

Nach der Vorschrift des Beispiels A3 werden aus 190 mg 14,15-Epoxi-Milbemycin $A_3$ und 120 mg tert.-Butyldimethylchlorsilan in Gegenwart von Imidazol 217 mg der Titel-Verbindung erhalten.

Beispiel A11: Herstellung von 5-O-tert.-Butyldimethylsilyl-15-hydroxi-$\Delta^{13,14}$-milbemycin $A_3$ (Formel VIb)

Analog zur Epoxid-Spaltung des Beispiels A7 werden aus 210 mg 5-O-tert.-Butyldimethylsilyl-14,15-epoxi-milbemycin $A_3$ in absolutem Diethylether mit Hilfe des Komplex-Reagenz $HN_3/Et_3Al$ unter Argon nach anschliessender Reinigung 203 mg der Titelverbindung erhalten.
$^1$H-NMR (300 HMz, $CDCl_3$; TMS):
1,58 ppm (br. s) ($C_{14}CH_3$);
4,05 ppm (br. s) ($C_{15}H$);
5,15 ppm (d; J=6 Hz)($C_{13}H$).

Beispiel A12: Herstellung von 15-hydroxi-$\Delta^{13,14}$-milbemycin $A_3$ (Formel VIb)

Analog zu Beispiel A2 wird das Reagenz $HN_3/Al(C_2H_5)_3$ frisch hergestellt und bei -10°C zu einer Lösung von 830 mg (3,05 mmol) 14,15-Epoxi-milbemycin $A_3$ in 7 ml trockenem Diethylether getropft. Nach der Aufarbeitung werden 385 mg 15-Hydroxi-$\Delta^{13,14}$-milbemycin $A_3$ und 92 mg 14-Azido-15-hydroxy-milbemycin $A_3$ erhalten.

Beispiel A13: Herstellung von 13-Deoxi-14,15-epoxi-22,23-dihydroavermectin-Bla-aglykon ($R_2$ = sec.$C_4H_9$) -(Formel VII)

Analog zu Beispiel A5 erhält man aus 520 mg 13-Deoxi-22,23-dihydroavermectin-Bla-aglykon - [Tetrahedron Letters, Vol. 24, No. 148, pp. 5333-5336 (1983)] und 210 mg m-Chlorbenzopersäure in 20 ml Dichlormethan 510 mg der Titelverbindung.

Beispiel A14: Herstellung von 5-O-t.Butyldimethylsilyl-13-deoxi14,15-epoxi-22,23-dihydro-avermectin-Bla-aglykon (Formel VII)

Analog zu Beispiel A6 erhält man aus 220 mg der Titelverbindung von Beispiel A13 und 55 mg tert.Butyldimethylchlorsilan in Gegenwart von 25 mg Imidazol in 5 ml trockenem DMF 108 mg der Titelverbindung.

Beispiel A15: Herstellung von 13-Deoxi-15-hydroxi-$\Delta^{13,14}$-22,23-dihydro-avermectin-Bla-aglykon (Formel VIb)

Analog zu Beispiel A2 erhält man aus 220 mg der Titelverbindung von Beispiel A14 mit dem Komplex-Reagenz, bestehend aus 320 mg $Al(C_2H_5)_3$ und 110 mg einer 6,96 %igen Lösung von $HN_3$ in total 16 ml trockenem Diethylether 112 mg der Titelverbindung. Daneben werden 61 mg 13-Deoxi-14-azido-15-hydroxi-22,23-dihydro-avermectin-Bla-aglykon erhalten.

Beispiel A16: a) Herstellung von 5-O-tert.Butyldimethylsilyl-13β-hydroxi-milbemycin D und von 13β-Hydroxi-milbemycin D (Formel VIa)

Eine Lösung bestehend aus 286 mg (0,41 mmol) 5-O-tert.Butyldimethylsilyl-15-hydroxi-$\Delta^{13,14}$-milbemycin D und 209 mg (0,56 mmol) Pyridiniumdichromat (PDC) in 3 ml Dimethylformamid (DMF) wird 30 min bei Raumtemperatur gerührt. Anschliessend wird 1 ml Isopropanol zugegeben, 5 min weitergerührt und dann mit 50 ml Ether verdünnt. Nach weiteren 10 min wird das Gemisch durch Kieselgel filtriert und eingeengt. Bei der Chromatographie des Rohproduktes an 20 g Kieselgel (Ether/Hexan 1:2) werden 165 mg (57 %) 5-O-t-Butyldimethylsilyl-13β-hydroxi-milbemycin D erhalten.
$^1$H-NMR (300 MHz; $CDCl_3$; TMS):
1,59 ppm (br. s) ($C_{14}CH_3$)
3,70 ppm (d; J = 10 Hz)($C_{13}H$).

105 mg (0,153 mmol) der so gewonnenen Verbindung werden mit 1 ml einer 1%igen Lösung von p-Toluolsulfonsäure in Methanol 1 Stunde bei Raumtemperatur gerührt. Das Gemisch wird mit 20 ml Ether verdünnt, durch Kieselgel filtriert, eingeengt, und der Rückstand wird an ca. 10 g Kieselgel chromatographiert (Aceton/Dichlormethan 1:4), wobei 73 mg (83 %) 13β-Hydroxi-Milbemycin D erhalten werden.
$^1$H-NMR (300 MHz; $CDCl_3$; TMS):
1,58 ppm (br. s) ($C_{14}CH_3$)
3,71 ppm (d; J = 10 Hz)($C_{13}H$).

b) Herstellung von 5-O-tert.-Butyldimethylsilyl-13β-hydroxi-milbemycin $A_4$ (Formel VIa)

Setzt man analog zur Vorschrift a) als Ausgangsmaterial 5-O-tert.-Butyldimethylsilyl-15-hydroxi-$\Delta^{13,14}$-milbemycin $A_4$ ein, so erhält man die Titelverbindung, die folgende physikalische Daten aufweist:
$^1$H-NMR (300 MHz; $CDCl_3$; TMS):
3,05 ppm (t; J = 9 Hz) ($C_{25}H$)
3,71 ppm (dd; J = 3 und 10 Hz)($C_{13}H$).
Massenspektrum (FD) m/e: 672 ($M^+$; $C_{38}H_{60}O_8Si$).

Beispiel A17: a) Herstellung von 5-O-tert.Butyldimethylsilyl-13β-acetoxi-milbemycin D (Formel IVa)

Eine Lösung von 200 mg (0,29 mmol) 5-O-tert.Butyldimethylsilyl-13β-hydroxi-milbemycin D und 1 ml Pyridin in 2 ml Essigsäureanhydrid wird 2 Std. bei Raumtemperatur gerührt. Die Aufarbeitung in Diethylether liefert 212 mg 5-O-tert.Butyldimethylsilyl-13β-acetoxi-milbemycin D in Form eines amorphen Pulvers.

b) Herstellung von 5-O-tert.-Butyldimethylsilyl-13$\beta$-acetoxi-milbemycin $A_4$ (Formel IVa)

Setzt man analog zur Vorschrift a) als Ausgangsmaterial 5-O-tert.-Butyldimethylsilyl-13$\beta$-hydroxi-milbemycin $A_4$ ein, so erhält man die Titelverbindung, die folgende physikalische Daten aufweist:
$^1$H-NMR (360 MHz; $CDCl_3$; TMS):
1,53 ppm (s) ($C_{14}CH_3$)
2,03 ppm (s) ($CH_3COO$)
4,94 ppm (d; J = 10 Hz) ($C_{13}H$)
Massenspektrum (FD) m/e: 714 ($M^+$; $C_{40}H_{62}O_9Si$).

Beispiel A18: a) Herstellung von 5-O-tert.-Butyldimethylsilyl-15-acetoxi-$\Delta^{13,14}$-milbemycin D (Formel IVb)

Eine Lösung von 627 mg (0,914 mmol) 5-O-tert.-Butyldimethylsilyl-15-hydroxi-$\Delta^{13,14}$-milbemycin D in 2 ml Essigsäureanhydrid und 2 ml Pyridin wird 0,5 Std. bei Raumtemperatur gerührt. Die Aufarbeitung in Diethylether mit 5 proz. wässriger $NaHCO_3$-Lösung und 1 M HCl und Filtration durch Kieselgel ergibt 624 mg (94 %) 5-O-tert.Butyldimethylsilyl-15-acetoxi-$\Delta^{13,14}$-milbemycin D. $^1$H-NMR (300 MHz; $CDCl_3$; TMS):
1,58 ppm (br. s) ($C_{14}CH_3$)
1,79 ppm (br. s) ($C_4CH_3$)
2,02 ppm (s) ($CH_3COO$)
5,12 -5,26 ppm (m) ($C_{10}H$; $C_{13}H$; $C_{15}H$)

b) Herstellung von 5-O-tert.-Butyldimethylsilyl-15-acetoxi-$\Delta^{13,14}$-milbemycin $A_4$ (Formel IVb)

Setzt man gemäss Vorschrift a) als Ausgangsprodukt 5-O-tert.-Butyldimethylsilyl-15-hydroxi-$\Delta^{13,14}$-milbemycin $A_4$ ein, so erhält man die Titelverbindung, die folgende physikalische Daten aufweist:
$^1$H-NMR (250 MHz; $CDCl_3$; TMS):
1,59 ppm (s) ($C_{14}CH_3$)
2,03 ppm (s) ($CH_3COO$)
3,02 ppm (t; J = 8 Hz) ($C_{25}H$)
3,88 ppm (d; J = 6 Hz) ($C_6H$)
Massenspektrum m/e: 714 ($M^+$; $C_{40}H_{62}O_9Si$), 639, 579, 497, 472, 437, 413, 412, 394, 349.

c) Herstellung von 5-O-tert.-Butyldimethylsilyl-15-acetoxi-$\Delta^{13,14}$-milbemycin $A_4$ (Formel IVb)

Die Herstellung verläuft völlig analog zu a) und b) jedoch ausgehend von 5-O-tert.-Butyldimethylsilyl-15-hydroxi-$\Delta^{13,14}$-milbemycin $A_3$.

Beispiel A19: Herstellung von 13$\beta$-Methyl-milbemycin D (Formel II)

Zu einer Lösung von 203 mg (0,28 mmol) 5-O-tert.Butyldimethylsilyl-15-acetoxi-$\Delta^{13,14}$-milbemycin D in 2 ml Dichlormethan wurden unter Argon bei 0°C 1,2 ml einer 17-proz. Lösung von Trimethylaluminium in Toluol unter Rühren zugetropft. Die Lösung wurde 2 Std. bei Raumtemperatur gerührt, dann wurden 0,3 ml Methanol zugetropft, und das Gemisch wurde mit Diethylether verdünnt und mit Celite gerührt. Die Filtration durch Kieselgel (Laufmittel Diethylether) ergab 177 mg 5-O-tert.Butyldimethylsilyl-13$\beta$-methyl-milbemycin D.

Eine Lösung dieses Materials in 0,5 ml Dichlormethan wurde zusammen mit 1 ml einer 40-proz. wässrigen Lösung von HF in Acetonitril (5:95) 1 Std. bei Raumtemperatur gerührt. Das Gemisch wurde in Diethylether aufgearbeitet und durch Kieselgel filtriert. HPLC ($SiO_2$; 0,5 % Methanol in Dichlormethan; Druck 50 bar) des Rohprodukts (154 mg) ergab 116 mg (57 %) 13$\beta$-Methyl-milbemycin D.

$^1$H-NMR (300 MHz; $CDCl_3$; TMS):
1,01 ppm (d, J = 6,7 Hz) ($C_{13}CH_3$)
5,03 ppm (dd; J = 10,5 und 4,6 Hz) ($C_{15}H$)
Massenspektrum m/e: 570 ($M^+$; $C_{34}H_{50}O_7$), 442, 292, 273, 262, 210, 209, 181, 163, 152, 151.

Beispiel A20: a) Herstellung von 13β-Ethyl-milbemycin D und 15-Ethyl-$\Delta^{13,14}$-milbemycin D (Formel II)

Zu einer Lösung von 340 mg (0,47 mmol) 5-O-tert.Butyldimethylsilyl-15-acetoxi-$\Delta^{13,14}$-milbemycin D in 2 ml Dichlormethan wurden unter Argon bei 0°C 0,75 ml (0,63 g; 5,5 mmol) Triethylaluminium unter Rühren zugetropft. Die Lösung wurde 1 Std. bei Raumtemperatur gerührt, dann wurde mit Diethylether verdünnt, das Gemisch mit Celite/$Na_2SO_4$•10 $H_2O$ (1:1) versetzt und 1 Std. gerührt. Die Filtration durch Kieselgel - (Laufmittel Diethylether) ergab 258 mg eines Gemisches, welches in 0,5 ml Dichlormethan gelöst und zusammen mit 1 ml einer 4 proz. wässrigen Lösung von HF in Acetonitril (5:95) 1 Std. bei Raumtemperatur gerührt wurde. Die Aufarbeitung in Diethylether, Filtration durch Kieselgel (Laufmittel Diethylether) und HPLC (Reversed Phase: Wasser/Methanol 1:9; Druck 50 mbar) des Rohprodukts (183 mg) ergab 88 mg (32 %) 13β-Ethyl-milbemycin D.
$^1$H-NMR (300 MHz; $CDCl_3$; TMS):
0,73 ppm (t, J = 7,2 Hz) ($C_{13}CH_2CH_3$)
5,03 ppm (dd; J = 10,5 und 4,4 Hz) ($C_{15}H$)
Massenspektrum m/e: 584 ($M^+$; $C_{35}H_{52}O_7$), 456, 287, 276, 210, 209, 181, 163, 151.
und 57 mg (21 %) 15-Ethyl-$\Delta^{13,14}$-milbemycin D.
$^1$H-NMR (300 MHz; $CDCl_3$; TMS):
3,03 ppm (m) ($C_{12}H$)
4,93 ppm (dd; J = 8,7 und 1,2 Hz) ($C_{13}H$)
Massenspektrum m/e: 584 ($M^+$; $C_{35}H_{52}O_7$), 456, 438, 277, 276, 206, 181, 171, 163, 151, 150, 149.

Analog zur Vorschrift A20a werden durch Reaktion mit den entsprechenden Trialkylverbindungen die nachfolgend in A20b bis A20h genannten Milbemycine der Formel II erhalten:

A20b) 13β-Methyl-milbemycin $A_4$

$^1$H-NMR (250 MHz; $CDCl_3$; TMS):
3,07 ppm (dt; J = 12 und 10 Hz) ($C_{25}H$)
5,05 ppm (dd; J = 10 und 5 Hz) ($C_{15}H$)
Massenspektrum (FD) m/e: 556 ($M^+$; $C_{33}H_{48}O_7$).

A20c) 13β-Ethyl-milbemycin $A_4$

$^1$H-NMR (250 MHz; $CDCl_3$; TMS):
3,03 ppm (breites t; J = 10 Hz) ($C_{25}H$)
5,02 ppm (dd; J = 10 und 7 Hz) ($C_{15}H$)
Massenspektrum (FD) m/e: 570 ($M^+$; $C_{34}H_{50}O_7$).

A20d) 13β-n-Hexyl-milbemycin D

$^1$H-NMR (250 MHz; $CDCl_3$; TMS):
3,08 ppm (d; J = 8 Hz) ($C_{25}H$)
5,00 ppm (breites t; J = 8 Hz) ($C_{15}H$)
Massenspektrum (FD) m/e: 640 ($M^+$; $C_{39}H_{60}O_7$).

A20e) 13β-n-Butyl-milbemycin $A_4$

$^1$H-NMR (250 MHz; $CDCl_3$; TMS):
3,03 ppm (breites t; J = 10 Hz) ($C_{25}H$)
5,02 ppm (breites t; J = 10 Hz) ($C_{15}H$)
Massenspektrum (FD) m/e: 598 ($M^+$; $C_{36}H_{54}O_7$).

A20f) 13β-Isobutyl-milbemycin $A_4$

$^1$H-NMR (250 MHz; $CDCl_3$; TMS):
3,09 ppm (breites t; J = 10 Hz) ($C_{25}H$)
5,05 ppm (dd; J = 10 und 7 Hz) ($C_{15}H$)
Massenspektrum (FD) m/e: 598 ($M^+$; $C_{36}H_{54}O_7$).

A20g) 13β-Methyl-milbemycin $A_3$

$^1$H-NMR (300 MHz; $CDCl_3$; TMS):
3,27 ppm (m) ($C_{25}H$)
5,06 ppm (dd; J = 10 und 6 Hz) ($C_{15}H$)
Massenspektrum (FD) m/e: 542 ($M^+$; $C_{32}H_{46}O_7$).

A20h) 13β-Ethyl-milbemycin $A_3$

$^1$H-NMR (300 MHz; $CDCl_3$; TMS):
3,25 ppm (m) ($C_{25}H$)
5,06 ppm (dd; J = 10 und 6 Hz) ($C_{15}H$)
Massenspektrum (FD) m/e: 556 ($M^+$; $C_{33}H_{48}O_7$).

Beispiel A21: Herstellung von 13β-Methyl-milbemycin-D (Formel II) Aus 5-O-tert.Butyldimethylsilyl-13β-acetoxi-milbemycin-D.

Zu einer Lösung von 14 mg (0,019 mmol) 5-O-tert.Butyldimethylsilyl13β-acetoxi-milbemycin D in 0,5 ml Dichlormethan werden unter Argon bei 0°C, 0,5 ml einer 17-proz. Lösung von Trimethylaluminium in Toluol unter Rühren zugetropft. Die Lösung wird über Nacht bei 5°C gerührt. Die Aufarbeitung wie unter A20 ergibt 10 mg 5-O-tert.-Butyldimethylsilyl-13β-methyl-milbemycin D.

Eine Lösung dieses Materials in 0,5 ml Dichlormethan wird zusammen mit 1 ml einer 40 proz. wässrigen Lösung von HF in Acetonitril, (5:95) 1 Std. bei Raumtemperatur gerührt. Das Gemisch wird in Diethylether aufgearbeitet und durch Kieselgel filtriert, wobei 8 mg 13β-Methyl-milbemycin Danfallen.

## Herstellung der Endprodukte

E1. Herstellung von 5-O-Chloracetyl-13β-methyl-milbemycin $A_4$

Eine Lösung von 350 mg 13β-Methyl-milbemycin $A_4$ und 0,50 ml Pyridin in 3 ml Methylenchlorid wird auf 0°C abgekühlt und dann innert 2 Std. mit 80 µl Chloracetylchlorid versetzt. Nach einer weiteren Std. Rühren bei 0°C wird aufgearbeitet. Die Chromatographie an Kieselgel mit Hexan/Essigsäureäthylester 4:1 ergibt 324 mg Produkt.
$^1$H-NMR (250 MHz; $CDCl_3$):
3,07 ppm (dt, $J_d$ = 3, $J_t$ = 10) ($C_{25}H$)
4,18 ppm (s) ($CH_2Cl$)

E2. Herstellung von 5-O-Acetoxy-acetyl-13β-methyl-milbemycin $A_4$

a) Zu einer Lösung von 50 mg 13β-Methyl-milbemycin $A_4$, 73 µl Pyridin und 1 mg Dimethylaminopyridin in 1 ml Methylenchlorid wird bei 0°C innerhalb von 30 Min. 2 ml einer 0,09 M Lösung von Acetoxyacetylchlorid in Benzol gegeben. Nach 3 Std. Rühren bei 0°C wird die Reaktion aufgearbeitet. Chromatographie des Rohproduktes an Kieselgel mit Hexan/Essigsäureäthylester 3:1 ergibt 49 mg Produkt.
Massenspektrum (MS) (m/e): 656 ($M^+$)
$^1$H-NMR (300 MHz; $CDCl_3$):
2,16 ppm (s) ($CH_3$-CO)
3,07 ppm (dt; $J_d$ = 3, $J_t$ = 10) ($C_{25}$H)
5,03 ppm (dd, J = 11 und 6) ($C_{15}$H)

b) Zu einer Lösung von 80 mg 5-O-Chloracetyl-13β-methyl-milbemycin $A_4$ in 3 ml DMF werden 16 mg Natriumacetat und 2 mg Natriumjodid gegeben. Die Reaktionsmischung wird 3 1/2 Stunden auf 60°C erhitzt und anschliessend aufgearbeitet. Die Chromatographie des Rohproduktes an Kieselgel mit Hexan/Essigsäureäthylester 3:1 ergibt 53 mg 5-O-Acetoxy-acetyl-13β-methyl-milbemycin $A_4$.

E3. Herstellung von 5-O-(3,4-Dihydro-2H-pyran-2-yl)carboxyacetyl-13β-methyl-milbemycin $A_4$

Eine Lösung von 80 mg 5-O-Chloracetyl-13β-methyl-milbemycin $A_4$, 29 mg (±)-3,4-Dihydro-2H-pyran-2-carbonsäure-natriumsalz und 2 mg Natriumjodid in 3 ml DMF wird 6 1/2 Std. unter Argonatmosphäre bei 60°C gerührt und anschliessend mit 50 ml Diäthylether verdünnt. Die erhaltene Lösung wird mit 20 ml Wasser und 20 ml ges. NaCl-Lösung gewaschen, über $MgSO_4$ getrocknet, filtriert und eingedampft. Das erhaltene Rohprodukt ergibt nach Chromatographie an Kieselgel mit Hexan/Essigsäureäthylester 3:1 59 mg Produkt.
MS (m/e): 724 ($M^+$)
$^1$H-NMR (250 MHz; $CDCl_3$):
3,07 ppm (dt; $J_d$ = 3, $J_t$ = 10) ($C_{25}$H)
5,05 ppm (dd, J = 6 und 10) ($C_{15}$H)
6,44 ppm (d, J = 8) (O-C<u>H</u>=CH)

E4. Herstellung von 5-O-(1,2,4-Triazol-1-yl)-acetyl-13β-methyl-milbemycin $A_4$

Zu einer Lösung von 100 mg 13β-Methyl-milbemycin $A_4$ und 57 mg 1H-1,2,4-Triazol-1-yl-essigsäure in 2 ml Tetrahydrofuran werden 0,30 ml Pyridin, 2 mg Dimethylaminopyridin und 74 mg N,N-Dicyclohexylcarbodiimid gegeben. Das Reaktionsgemisch wird anschliessend 2 1/2 Std. bei RT gerührt und aufgearbeitet. Das erhaltene Rohprodukt wird in 3 ml Diäthyläther aufgeschlämmt, filtriert und eingedampft. Chromatographie an Kieselgel mit Methylenchlorid/Methanol (5 %) liefert 100 mg Produkt.
MS (m/e): 665 ($M^+$)
$^1$H-NMR (300 MHz; $CDCl_3$):
3,07 ppm (dt; $J_d$ = 3, $J_t$ = 10) ($C_{25}$H)
5,07 ppm (s) ($CH_2$N)
7,97 ppm (s) (N=CH-N)
8,24 ppm (s) (N=CH-N)

E5. Herstellung von 5-O-(1,2,4-Trizaol-1-yl)acetyl-13β-methyl-milbemycin $A_4$-Zink(II)-Komplex

Zu einer Lösung von 47 mg 5-O-(1,2,4-Triazol-1-yl)-acetyl-13β-methyl-milbemycin $A_4$ in 2 ml Benzol werden 200 µl einer 0,19 M Lösung von Zink(II)chlorid in Aethanol gegeben. Die erhaltene Lösung wird 1 Std. bei RT stehen gelassen und anschliessend gefriergetrocknet. Man erhält 52 mg Produkt.
$^1$H-NMR (300 MHz; $CDCl_3$):
3,06 ppm (dt; $J_d$ = 3, $J_t$ = 10) ($C_{25}$H)

5,12 ppm (s) ($CH_2N$)
8,21 ppm (s) (N=CH-N)
8,65 ppm (s) (N=CH-N)

E6. Herstellung von 5-O-Acetoxy-acetyl-13β-ethyl-milbemycin $A_4$

Zu einer Lösung von 65 mg 13β-Ethyl-milbemycin $A_4$, 92 μl Pyridin und 1 mg Dimethylaminopyridin in 1 ml Methylenchlorid wird bei 0°C 0,92 ml einer 0,25 M Lösung von Acetoxyacetylchlorid in Benzol gegeben. Nach 4 Std. Rühren bei 0°C wird die Reaktion aufgearbeitet. Chromatographie des Rohprodukts an Kieselgel mit Hexan/Essigsäureethylester 4:1 ergibt 70 mg Produkt.
MS (m/e): 670 ($M^+$; $C_{38}H_{54}O_{10}$)
$^1$H-NMR (300 MHz; $CDCl_3$):
0,71 ppm (t, J = 7), ($C_{13}CH_2C\underline{H_3}$)
2,16 ppm (s) ($CH_3$-CO)
3,07 ppm (dt, $J_d$ = 3, $J_t$ = 9) ($C_{25}H$)
5,60 ppm (d, J = 7) ($C_5H$)
Analog lassen sich 5-O-Acetoxy-acetyl-13β-ethyl-milbemycin $A_3$ und 5-O-Acetoxy-acetyl-13β-ethyl-milbemycin D herstellen.

E7. Herstellung von 5-O-Chloracetyl-13β-ethyl-milbemycin $A_4$

Analog Herstellungsbeispiel E1 können aus 61 mg 13β-Ethyl-milbemycin $A_4$ 59 mg Produkt gewonnen werden.
MS (m/e): 646 ($M^+$; $C_{36}H_{51}ClO_8$)
$^1$H-NMR (300 MHz; $CDCl_3$):
3,06 ppm (dt; $J_d$ = 3, $J_t$ = 10) ($C_{25}H$)
4,15 ppm (s) ($CH_2Cl$)
5,01 ppm (dd, J = 6 und 10) ($C_{15}H$)

E8. Herstellung von 5-O-tert.-Butyldimethylsilyloxy-acetyl-13β-methyl-milbemycin $A_4$

Zu einer Lösung von 956 mg tert.-Butyldimethylsilyloxyessigsäure-tert.-butyldimethylsilylester in 4 ml Methylenchlorid wurden ein Tropfen Dimethylformamid (ca. 30 μl) und anschliessend bei 0°C innerhalb 45 Min. 222 μl Oxalylchlorid zugegeben. Danach liess man 30 Min. bei 0°C und 2 Std. bei RT Rühren und gab anschliessend die erhaltene Lösung des Säurechlorids tropfenweise innerhalb 20 Min. zu einer auf 0°C abgekühlten Lösung von 900 mg 13β-Methyl-milbemycin $A_4$ und 1,31 ml Pyridin in 2 ml Methylenchlorid. Nach 90 Min. Rühren bei 0°C wurde das Reaktionsgemisch zwischen Ether und 1 N HCl-Lösung verteilt und die erhaltene organische Phase mit ges. $NaHCO_3$-Lösung und ges. NaCl-Lösung gewaschen. Nach dem Eindampfen wurde der Rückstand an Kieselgel mit Hexan/Essigsäureethylester 10:1 chromatographiert. Man erhielt 988 mg Produkt.
MS (m/e): 728 ($M^+$; $C_{41}H_{64}O_9Si$)
$^1$H-NMR (300 MHz; $CDCl_3$):
0,10 ppm (s) ($(CH_3)_2Si$)
0,80 ppm (s) (tert.$C_4H_9Si$)
3,05 ppm (dt, $J_d$ = 3, $J_t$ = 9) ($C_{25}H$)
5,57 ppm (d, J = 6) ($C_5H$)

E9. Herstellung von 5-O-Hydroxyacetyl-13β-methyl-milbemycin $A_4$

Zu 5 ml einer 5 % Lösung von 40 % HF in Acetonitril wurden 934 mg 5-O-tert.-Butyldimethylsilyloxy-acetyl-13β-methylmilbemycin $A_4$ gegeben. Nach 2 Std. Rühren bei RT wurde das Reaktionsgemisch zwischen Diethylether und ges. $NaHCO_3$-Lösung verteilt und die organische Phase mit ges. NaCl-Lösung verteilt und die organische Phase mit ges. NaCl-Lösung gewaschen. Nach dem Eindampfen wurde der Rückstand chromatographisch gereinigt (Laufmittel: Hexan/Essigsäureethylester 2:1), und man erhielt 690

mg Produkt.
MS (m/e): 614 ($M^+$; $C_{35}H_{50}O_9$)
$^1$H-NMR (300 MHz; $CDCl_3$):
2,38 ppm (t, J = 6) ($CH_2O\underline{H}$)
3,05 ppm (dt, $J_d$ = 2,5, $J_t$ = 9) ($C_{25}H$)
4,21 ppm (dd, $J_1$ = 6, $J_2$ = 16) (CO-C$\underline{H}$HOH)
4,28 ppm (dd, $J_1$ = 6, $J_2$ = 16) (CO-CH$\underline{H}$OH)

E10. Herstellung von 5-O-Methoxy-carbonyloxy-acetyl-13β-methyl-milbemycin $A_4$

Zu einer Lösung von 50 mg 5-O-Hydroxyacetyl-13β-methyl-milbemycin $A_4$ und 66 µl Pyridin in 2 ml Methylenchlorid wurden bei 0°C 13µl Chlorameisensäure-methylester gegeben. Nach 15 Min. Rühren bei 0°C wurde das Kühlbad entfernt und weitere 2 Std. bei RT weitergerührt. Nach Aufarbeitung des Reaktionsgemischs wurde das erhaltene Rohprodukt an Kieselgel mit Hexan/Essigsäureethylester chromatographiert. Man erhielt 50 mg Produkt.
MS (m/e): 672 ($M^+$; $C_{37}H_{52}O_{11}$)
$^1$H-NMR (300 MHz; $CDCl_3$):
3,05 ppm (dt, $J_d$ = 2,5, $J_t$ = 9) ($C_{25}H$)
3,78 ppm (s) ($CH_3O$)
4,68 ppm (d, J = 16) (CO-C$\underline{H}$HO)
4,74 ppm (d, J = 16) (CO-CH$\underline{H}$O)
5,60 ppm (bd, J = 6) ($C_5H$)

E11. Herstellung von 5-O-Methoxy-methoxy-acetyl-13β-methyl-milbemycin $A_4$

Zu einer Lösung von 50 mg 5-O-Hydroxyacetyl-13β-methyl-milbemycin $A_4$ und 580 µl N-Ethyl-diisopropylamin in 1 ml Methylenchlorid wurden 168 µl Brommethyl-methylether gegeben. Nach 2 Tagen Rühren bei RT wurde das Reaktionsgemisch aufgearbeitet und das erhaltene Rohprodukt an Kieselgel mit Hexan/Essigsäureethylester 4:1 chromatographiert. Man erhielt 23 mg Produkt.
MS (m/e): 658 ($M^+$; $C_{37}H_{54}O_{10}$)
$^1$H-NMR (300 MHz; $CDCl_3$):
3,05 ppm (dt, $J_d$ = 2,5, $J_t$ = 9) ($C_{25}H$)
3,38 ppm (s) ($CH_3O$)
4,21 ppm (d, J = 16) (CO-C$\underline{H}$HO)
4,28 ppm (d, J = 16) (CO-CH$\underline{H}$O)
5,60 ppm (d, J = 6) ($C_5H$)

E12. Herstellung von 5-O-(3-Chlorbenzoyloxy)-acetyl-13β-methyl-milbemycin $A_4$

Eine Lösung von 50 mg 5-O-Hydroxyacetyl-13β-methyl-milemycin $A_4$ und 66 µl Pyridin in 2 ml Methylenchlorid wurde mit 21 µl 3-Chlorbenzoylchlorid versetzt und 3 Std. bei RT gerührt. Nach Aufarbeitung des Reaktionsgemischs wurde das erhaltene Rohprodukt an Kieselgel mit Hexan/Essigsäureethylester 6:1 chromatographiert. Man erhielt 54 mg Produkt.
MS (m/e): 752 ($M^+$; $C_{42}H_{53}ClO_{10}$)
$^1$H-NMR (300 MHz; $CDCl_3$):
3,05 ppm (dt, $J_d$ = 2,5, $J_t$ = 9) ($C_{25}H$)
4,90 ppm (d, J = 16) (CO-C$\underline{H}$HO)
4,98 ppm (d, J = 16) (CO-CH $\underline{H}$O)
5,61 ppm (bd, J = 6) ($C_5H$)
7,39 ppm (m) (aromatisches Ringproton)
7,55 ppm (m) (aromatisches Ringproton)
7,97 ppm (m) (aromatisches Ringproton)
8,06 ppm (m) (aromatisches Ringproton)

E13. Herstellung von 5-O-(2-Tetrahydropyranyl)oxy-acetyl-13β-methyl-milbemycin $A_4$

Zu einer Lösung von 50 mg 5-O-Hydroxyacetyl-13β-methyl-milbemycin $A_4$ und 74 µl 3,4-Dihydro-2H-pyran in 2 ml Methylenchlorid wurden 2 mg Pyridinium-(toluol-4-sulfonat) gegeben und 90 Min. bei RT gerührt. Nach der Aufarbeitung des Reaktionsgemischs und Chromatographie des Rohprodukts an Kieselgel mit Hexan/Essigsäureethylester 4:1 konnten 49 mg Produkt isoliert werden.
MS (m/e): 698 ($M^+$; $C_{40}H_{58}O_{10}$)
$^1$H-NMR (300 MHz; $CDCl_3$):
3,05 ppm (dt, $J_d$ = 2,5, $J_t$ = 9) ($C_{25}H$)
4,27 ppm (d, J = 16) (CO-C<u>H</u>HO)
4,33 ppm (d, J = 16) (CO-CH<u>H</u>O)
4,78 ppm (m) (OC<u>H</u>($CH_2$)O)

E14. Herstellung von 5-O-(4-Chlor-butanoyl)oxy-acetyl-13β-methyl-milbemycin $A_4$

Eine Lösung von 50 mg 5-O-Hydroxyacetyl-13β-methyl-milbemycin $A_4$ und 66 µl Pyridin in 2 ml Methylenchlorid wurde bei 0°C mit 18 µl Chlorbuttersäurechlorid versetzt. Das Reaktionsgemisch wurde 2 Std. bei 0°C und anschliessend 2 Std. bei RT gerührt. Aufarbeitung des Reaktionsgemischs und chromatographische Reinigung des Rohprodukts an Kieselgel (Laufmittel: Hexan/Essigsäureethylester 4:1) ergaben 53 mg Produkt.
MS (m/e): 718 ($M^+$; $C_{39}H_{55}ClO_{10}$)
$^1$H-NMR (300 MHz; $CDCl_3$):
3,05 ppm (dt, $J_d$ = 2,5, $J_t$ = 9) ($C_{25}H$)
3,61 ppm (t, J = 7) ($CH_2Cl$)
4,68 ppm (d, J = 16) (CO-C<u>H</u>HO)
4,75 ppm (d, J = 16) (CO-CH<u>H</u>O)
5,59 ppm (bd, J = 6) ($C_5H$)

E15. Herstellung von 5-O-(Thiophen-2-carbonyloxy)acetyl-13β-methyl-milbemycin $A_4$

Zu einer Lösung von 50 mg 5-O-Hydroxyacetyl-13β-methyl-milbemycin $A_4$ und 66 µl Pyridin in 2 ml Methylenchlorid wurden 52 µl Thiophen-2-carbonsäurechlorid gegeben. Nach 4 Std. Rühren bei RT wurde das Reaktionsgemisch aufgearbeitet. Chromatographie des Rohprodukts an Kieselgel mit Hexan/Essigsäureethylester 4:1 ergab 55 mg Produkt.
MS (m/e): 724 ($M^+$; $C_{40}H_{52}O_{10}S$)
$^1$H-NMR (300 MHz; $CDCl_3$):
3,05 ppm (dt, $J_d$ = 2,5, $J_t$ = 9) ($C_{25}H$)
4,85 ppm (d, J = 16) (CO-C<u>H</u>HO)
4,93 ppm (d, J = 16) (CO-CH<u>H</u>O)
5,60 ppm (bd, J = 6) ($C_5H$)
7,11 ppm (m) (Thiophen-Proton)
7,60 ppm (m) (Thiophen-Proton)
7,88 ppm (m) (Thiophen-Proton)

E16. Herstellung von 5-O-Vinylcarbonyloxy-acetyl-13β-methyl-milbemycin $A_4$

Zu einer Lösung von 50 mg 5-O-Hydroxyacetyl-13β-methyl-milbemycin $A_4$ und 132 µl Pyridin in 2 ml Methylenchlorid wurden 65 µl Acrylsäurechlorid gegeben. Nach 5 Std. Rühren bei RT wurde das Reaktionsgemisch aufgearbeitet. Chromatographie des Rohprodukts an Kieselgel mit Hexan/Essigsäureethylester 4:1 ergab 10 mg Produkt.
MS (m/e): 668 ($M^+$; $C_{38}H_{52}O_{10}$)
$^1$H-NMR (300 MHz; $CDCl_3$):
3,05 ppm (dt, $J_d$ = 2,5, $J_t$ = 9) ($C_{25}H$)
4,75 ppm (d, J = 16) (CO-C<u>H</u>HO)
4,18 ppm (d, J = 16) (CO-CH<u>H</u>O)

5,60 ppm (bd, J = 6) ($C_5H$)
5,91 ppm (dd, $J_1$ = 1,5, $J_2$ = 10) (HHC=CHCO)
6,20 ppm (dd, $J_1$ = 10, $J_2$ = 17) HHC=C<u>H</u>CO)
6,50 ppm (dd, $J_1$ = 1,5, $J_2$ = 17) HHC=CHCO)

E17. <u>Herstellung von 5-O-Phenoxyacetyl-13β-methyl-milbemycin $A_4$</u>

Zu einer Lösung von 50 mg 5-O-Hydroxyacetyl-13β-methyl-milbemycin $A_4$ und 73 µl Pyridin in 2 ml Methylenchlorid wurden bei 0°C 25 µl Phenoxyacetylchlorid gegeben. Nach 2 Std. Rühren bei 0°C wurde aufgearbeitet. Chromatographie des Rohprodukts an Kieselgel mit Hexan/Essigsäureethylester 4:1 ergab 57 mg Produkt.
MS (m/e): 690 ($M^+$; $C_{41}H_{54}O_9$)
$^1$H-NMR (300 MHz; $CDCl_3$):
3,05 ppm (dt, $J_d$ = 2,5, $J_t$ = 9) ($C_{25}H$)
4,71 ppm (s) ($COCH_2O$)
5,60 ppm (bd, J = 6) ($C_5H$)
6,93 ppm (m) ($C_6H_3\underline{H_2}$-(2))
6,98 ppm (m) ($C_6H_4\underline{H}$-(4))
7,27 ppm (m) ($C_6H_3\underline{H_2}$-(3))

E18. <u>Herstellung von 5-O-Acetoxy-acetyl-13β-n-butyl-milbemycin $A_4$</u>

Analog Herstellungsbeispiel E6 erhielt man aus 8 mg 13β-n-Butyl-milbemycin $A_4$ 8 mg Produkt.
MS (m/e): 698 ($M^+$; $C_{40}H_{58}O_{10}$)
$^1$H-NMR (300 MHz; $CDCl_3$):
0,85 ppm (t, J = 7) ($C\underline{H_3}CH_2$)
2,16 ppm (s) ($CH_3CO$)
3,05 ppm (dt, $J_d$ = 2,5, $J_t$ = 9) ($C_{25}H$)
4,66 ppm (d, J = 16) (CO-C<u>H</u>HO)
4,73 ppm (d, J = 16) (CO-CH<u>H</u>O)
5,59 ppm (bd, J = 16) ($C_5H$)

E19. <u>Herstellung von 5-O-p-Tosyloxy-acetyl-13β-methyl-milbemycin $A_4$</u>

Zu einer Lösung von 50 mg 13 5-O-Hydroxyacetyl-13β-methyl-milbemycin $A_4$ und 260 µl Pyridin in 2 ml Diethylether wurden bei 0°C 2 mg 4-Dimethylamino-pyridin und anschliessend 150 mg Toluol-4-sulfochlorid gegeben. Das Reaktionsgemisch wurde 24 Std. bei RT gerührt. Nach Aufarbeitung und Chromatographie des erhaltenen Rohprodukts an Kieselgel (Laufmittel: Hexan/Essigsäureethylester 4:1) konnten, neben 7 mg 5-O-Chloracetyl-13β-methyl-milbemycin $A_4$, 15 mg Produkt isoliert werden.
MS (m/e): 768 ($M^+$; $C_{42}H_{56}O_{11}S$)
$^1$H-NMR (300 MHz; $CDCl_3$):
2,43 ppm (s) ($C_6H_4$-$C\underline{H_3}$)
3,05 ppm (dt, $J_d$ = 2,5, $J_t$ = 9) ($C_{25}H$)
4,67 ppm (s), ($COCH_2O$)

E20. <u>Herstellung von 5-O-Methoxyacetyl-13β-methyl-milbemycin $A_4$</u>

Zu einer Lösung von 81 µl Methoxyessigsäure in 5 ml Benzol wurden ein Tropfen Dimethylformamid - (ca. 30 µl) und anschliessend bei 0°C innerhalb 5 Min. 80 µl Oxalylchlorid zugegeben. Danach liess man das Reaktionsgemisch 2 Std. bei RT rühren.

1,56 ml der so erhaltenen Lösung wurden bei 0°C innerhalb 20 Min. zu einer Lösung von 50 mg 13β-Methyl-milbemycin $A_4$, 73 µl Pyridin und 0,6 mg 4-Dimethylamino-pyridin in 2 ml Methylenchlorid gegeben. Nach 2 Std. Rühren bei 0°C wurde das Reaktionsgemisch aufgearbeitet. Chromatographie des Rohprodukts an Kieselgel mit Hexan/Essigsäureethylester ergaben 46 mg Produkt.

MS (m/e): 628 ($M^+$; $C_{36}H_{52}O_9$)
$^1$H-NMR (300 MHz; $CDCl_3$):
3,48 ppm (s) ($CH_3O$)
4,10 ppm (d, J = 16) (COC<u>H</u>HO)
4,18 ppm (d, J = 16) (COCH<u>H</u>O)

E21. Herstellung von 5-O-Chloracetyl-13β-ethyl-milbemycin $A_3$

Analog Herstellungsbeispiel E1 werden aus 25 mg 13β-Ethyl-milbemycin $A_3$ 22 mg 5-O-Chloracetyl-13β-ethyl-milbemycin $A_3$ erhalten.
MS (m/e): 632 ($M^+$; $C_{35}H_{49}ClO_8$)
$^1$H-NMR (300 MHz; $CDCl_3$):
3,25 ppm (m) ($C_{25}H$)
4,16 ppm (s) ($CH_2Cl$)

E22. Herstellung von 5-O-Acetoxyacetyl-13β-methyl-milbemycin $A_3$

Analog Herstellungsbeispiel E6 werden aus 31 mg 13β-Methyl-milbemycin $A_3$ 30 mg 5-O-Acetoxyacetyl-13β-methyl-milbemycin $A_3$ erhalten.
MS (m/e): 642 ($M^+$; $C_{36}H_{50}O_{10}$)
$^1$H-NMR (300 MHz; $CDCl_3$):
0,70 ppm (t, J = 7) ($C_{13}CH_2C\underline{H}_3$)
2,15 ppm (s) ($CH_3CO$)
3,25 ppm (m) ($C_{25}H$)
5,59 ppm (d, J = 7) ($C_5H$)

E23. Herstellung von 5-O-Methoxyacetyl-13β-methyl-milbemycin D

Aus 25 mg 13β-Methyl-milbemycin D werden analog Herstellungsbeispiel E20 21 mg 5-O-Methoxyacetyl-13β-methyl-milbemycin D gewonnen.
MS (m/e): 642 ($M^+$; $C_{37}H_{54}O_9$)
$^1$H-NMR (300 MHz; $CDCl_3$):
3,07 ppm (bd, J = 10) ($C_{25}H$)
3,49 ppm (s) ($CH_3O$).

E24. Herstellung von 5-O-Acetyl-13β-methyl-milbemycin $A_4$

Zu einer Lösung von 20 mg 13β-Me thyl-milbemycin $A_4$ in 1 ml Methylenchlorid wurden bei 0°C 29 µl Pyridin und anschliessend 6 µl Acetylchlorid gegeben. Nach 3½ Rühren bei 0°C wurde aufgearbeitet. Nach Chromatographie des Rohprodukts an Kieselgel mit Hexan/Essigsäureethylester (4:1) konnten 20,5 mg Produkt isoliert werden.
MS (m/e): 598 ($M^+$; $C_{35}H_{50}O_8$)
$^1$H-NMR (300 MHz; $CDCl_3$):
2,14 ppm (s) ($CH_3CO$)
3,06 ppm (dt, $J_d$ = 2,5, $J_t$ = 9) ($C_{25}H$)
5,03 ppm (dd, $J_1$ = 11, $J_2$ = 5) ($C_{15}H$)

Analog zu den beschriebenen Arbeitsweisen werden auch die nachfolgend genannten Verbindungen der Formel I hergestellt; wobei die nachfolgenden Tabellen keinen limitierenden Charakter haben:

Tabelle 1: Typische Vertreter von Verbindungen der Formel I, worin $R_2$ für $CH_3$ steht (= Milbemycin-$A_3$-derviate):

| Verb.Nr. | R | $R_1$ | Physik. Konst. oder Herstellungsbeispiel |
|---|---|---|---|
| 1.1 | $CH_3$ | $COCH_3$ | |
| 1.2 | $C_2H_5$ | $COCH_3$ | |
| 1.3 | $C_3H_7$-n | $COCH_3$ | |
| 1.4 | $C_3H_7$-i | $COCH_3$ | |
| 1.5 | $C_4H_9$-n | $COCH_3$ | |
| 1.6 | $C_6H_{13}$-n | $COCH_3$ | |
| 1.7 | $C_{10}H_{21}$-n | $COCH_3$ | |
| 1.8 | $CH_3$ | $COCH_2Cl$ | |
| 1.9 | $CH_3$ | $COCH_2Br$ | |
| 1.10 | $C_2H_5$ | $COCH_2Cl$ | E21 |
| 1.11 | $CH_3$ | $COCH_2BrF$ | |
| 1.12 | $C_2H_5$ | $COCH_2F$ | |
| 1.13 | $CH_3$ | $COCH_2CH_3$ | |
| 1.14 | $C_2H_5$ | $COCH_2OCH_3$ | |
| 1.15 | $CH_3$ | $COCH_2SCH_3$ | |
| 1.16 | $CH_3$ | $COCH_2OCH_3$ | |
| 1.17 | $CH_3$ | $COCH_2OCOCH_3$ | E22 |
| 1.18 | $C_2H_5$ | $COCH_2OCOCH_3$ | |
| 1.19 | $C_3H_7$-n | $COCH_2OCOCH_3$ | |
| 1.20 | $C_6H_{13}$-n | $COCH_2OCOCH_3$ | |
| 1.21 | $CH_3$ | $COCHFOCOCH_3$ | |
| 1.22 | $CH_3$ | $COCH(CH_3)OCOCH_3$ | |
| 1.23 | $CH_3$ | $COCH_2OCOCH_2Cl$ | |
| 1.24 | $CH_3$ | $COCH_2OCOC_2H_5$ | |
| 1.25 | $CH_3$ | $COCH_2OCOC_6H_4F(3)$ | |
| 1.26 | $C_2H_5$ | $COCH_2OCOC_6H_4Cl(3)$ | |
| 1.27 | $CH_3$ | $COCH_2OCOC_6H_4OCH_3(3)$ | |
| 1.28 | $CH_3$ | $COCH_2OCOC_6H_3Cl_2(2,4)$ | |
| 1.29 | $CH_3$ | $COCH_2OCOC_3H_7$-n | |

Tabelle 1 (Fortsetzung)

| Verb.Nr. | R | $R_1$ | |
|---|---|---|---|
| 1.30 | $C_2H_5$ | $COCH_2OCH_2OCH_3$ | |
| 1.31 | $CH_3$ | 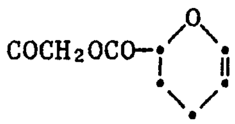 | |
| 1.32 | $C_2H_5$ | 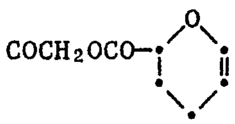 | |
| 1.33 | $CH_3$ | 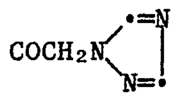 | |
| 1.34 | $C_2H_5$ | 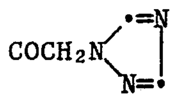 | |
| 1.35 | $CH_3$ | 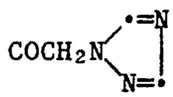 x 1/2 $ZnCl_2$ | |
| 1.36 | $C_2H_5$ | 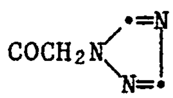 x 1/2 $ZnCl_2$ | |
| 1.37 | $CH_3$ | 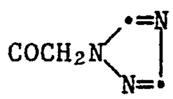 x 1/2 $CuCl_2$ | |
| 1.38 | $C_2H_5$ | 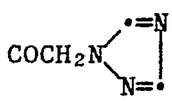 x 1/2 $CuCl_2$ | |
| 1.39 | $CH_3$ | 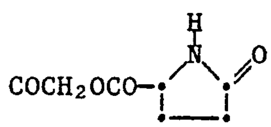 | |
| 1.40 | $C_2H_5$ | 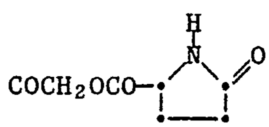 | |
| 1.41 | $CH_3$ | 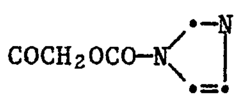 | |
| 1.42 | $C_2H_5$ | 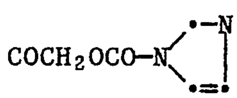 | |
| 1.43 | $CH_3$ | $COCH_2OCH_3$ | |
| 1.44 | $CH_3$ | $COCH_2OCOC_6H_4Cl(3)$ | |

Tabelle 2: Typische Vertreter von Verbindungen der Formel I, worin $R_2$ für $C_2H_5$ steht (=Milbemycin-$A_4$-derviate):

| Verb.Nr. | R | $R_1$ | Physik. Konst. oder Herstellungsbeispiel |
|---|---|---|---|
| 2.1 | $CH_3$ | $COCH_3$ | E24 |
| 2.2 | $C_2H_5$ | $COCH_3$ | |
| 2.3 | $C_3H_7$-n | $COCH_3$ | |
| 2.4 | $C_3H_7$-i | $COCH_3$ | |
| 2.5 | $C_4H_9$-n | $COCH_3$ | |
| 2.6 | $C_6H_{13}$-n | $COCH_3$ | |
| 2.7 | $C_{10}H_{21}$-n | $COCH_3$ | |
| 2.8 | $CH_3$ | $COCH_2Cl$ | E1 |
| 2.9 | $CH_3$ | $COCH_2Br$ | |
| 2.10 | $CH_3$ | $COCH_2F$ | |
| 2.11 | $CH_3$ | $COCH_2BrF$ | |
| 2.12 | $C_2H_5$ | $COCH_2F$ | |
| 2.13 | $CH_3$ | $COCH_2CH_3$ | |
| 2.14 | $C_2H_5$ | $COCH_2OCH_3$ | |
| 2.15 | $CH_3$ | $COCH_2SCH_3$ | |
| 2.16 | $CH_3$ | $COCH_2OC_2H_5$ | |
| 2.17 | $CH_3$ | $COCH_2OCOCH_3$ | E2 |
| 2.18 | $C_2H_5$ | $COCH_2OCOCH_3$ | E6 |
| 2.19 | $C_3H_7$-n | $COCH_2OCOCH_3$ | |
| 2.20 | $C_6H_{13}$-n | $COCH_2OCOCH_3$ | |
| 2.21 | $CH_3$ | $COCHFOCOCH_3$ | |
| 2.22 | $CH_3$ | $COCH(CH_3)OCOCH_3$ | |
| 2.23 | $CH_3$ | $COCH_2OCOCH_2Cl$ | |
| 2.24 | $CH_3$ | $COCH_2OCOC_2H_5$ | |
| 2.25 | $CH_3$ | $COCH_2OCOC_6H_4F(3)$ | |
| 2.26 | $C_2H_5$ | $COCH_2OCOC_6H_4Cl(3)$ | |
| 2.27 | $CH_3$ | $COCH_2OCOC_6H_4OCH_3(3)$ | |
| 2.28 | $CH_3$ | $COCH_2OCOC_6H_3Cl_2(2,4)$ | |
| 2.29 | $CH_3$ | $COCH_2OCOC_3H_7$-n | |
| 2.30 | $C_2H_5$ | $COCH_2OCH_2OCH_3$ | |

Tabelle 2 (Fortsetzung)

| Verb.Nr. | R | $R_1$ | Physik. Konst. oder Herstellungsbeispiel |
|---|---|---|---|
| 2.31 | $CH_3$ | 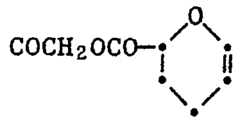 | E3 |
| 2.32 | $C_2H_5$ | 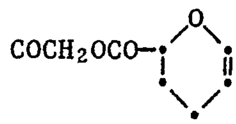 | |
| 2.33 | $CH_3$ | 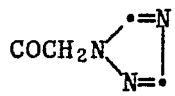 | E4 |
| 2.34 | $C_2H_5$ | 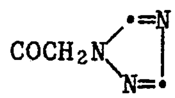 | |
| 2.35 | $CH_3$ | 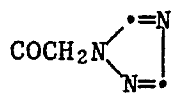 x 1/2 $ZnCl_2$ | E5 |
| 2.36 | $C_2H_5$ | 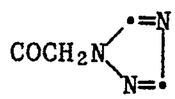 x 1/2 $ZnCl_2$ | |
| 2.37 | $CH_3$ | 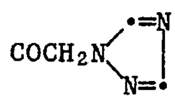 x 1/2 $CuCl_2$ | |
| 2.38 | $C_2H_5$ | 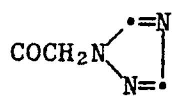 x 1/2 $CuCl_2$ | |
| 2.39 | $CH_3$ | 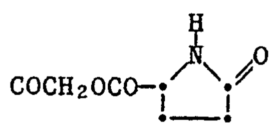 | |
| 2.40 | $C_2H_5$ | 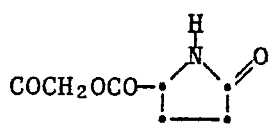 | |
| 2.41 | $CH_3$ | 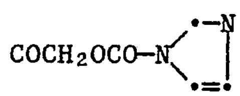 | |
| 2.42 | $C_2H_5$ | 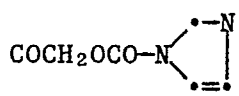 | |
| 2.43 | $CH_3$ | $COCH_2OCH_3$ | E20 |
| 2.44 | $CH_3$ | $COCH_2OCOC_6H_4Cl(3)$ | E12 |

Tabelle 2 (Fortsetzung)

| Verb.Nr. | R | $R_1$ | Physik. Konst. oder Herstellungsbeispiel |
|---|---|---|---|
| 2.45 | $C_2H_5$ | $COCH_2Cl$ | E7 |
| 2.46 | $CH_3$ | $COCH_2OH$ | E9 |
| 2.47 | $CH_3$ | $COCH_2OCOOCH_3$ | E10 |
| 2.48 | $CH_3$ | $COCH_2OCH_2OCH_3$ | E11 |
| 2.49 | $CH_3$ | $COCH_2$—O— 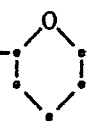 | E13 |
| 2.50 | $CH_3$ | $COCH_2OCOCH_2CH_2CH_2Cl$ | E14 |
| 2.51 | $CH_3$ | $COCH_2OCO$— 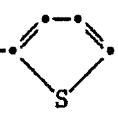 | E15 |
| 2.52 | $CH_3$ | $COCH_2OCO-CH=CH_2$ | E16 |
| 2.53 | $CH_3$ | $COCH_2OC_6H_5$ | E17 |
| 2.54 | $C_4H_9$-n | $COCH_2OCOCH_2$ | E18 |
| 2.55 | $CH_3$ | $COCH_2OSO_2-C_6H_4CH_3(4)$ | E19 |
| 2.56 | $CH_3$ | $COCH_2OSi(CH_3)_2$t-Butyl | E8 |

Tabelle 3: Typische Vertreter von Verbindungen der Formel I, worin $R_2$ für $C_3H_7$-iso steht (= Milbemycin-D-derivate):

| Verb.Nr. | R | $R_1$ | Physik. Konst. oder Herstellungsbeispiel |
|---|---|---|---|
| 3.1 | $CH_3$ | $COCH_3$ | E24 |
| 3.2 | $C_2H_5$ | $COCH_3$ | |
| 3.3 | $C_3H_7$-n | $COCH_3$ | |
| 3.4 | $C_3H_7$-i | $COCH_3$ | |
| 3.5 | $C_4H_9$-n | $COCH_3$ | |
| 3.6 | $C_6H_{13}$-n | $COCH_3$ | |
| 3.7 | $C_{10}H_{21}$-n | $COCH_3$ | |
| 3.8 | $CH_3$ | $COCH_2Cl$ | |
| 3.9 | $CH_3$ | $COCH_2Br$ | |
| 3.10 | $CH_3$ | $COCH_2F$ | |
| 3.11 | $CH_3$ | $COCH_2BrF$ | |
| 3.12 | $C_2H_5$ | $COCH_2F$ | |
| 3.13 | $CH_3$ | $COCH_2OCH_3$ | E23 |
| 3.14 | $C_2H_5$ | $COCH_2OCH_3$ | |
| 3.15 | $CH_3$ | $COCH_2SCH_3$ | |
| 3.16 | $CH_3$ | $COCH_2OC_2H_5$ | |
| 3.17 | $CH_3$ | $COCH_2OCOCH_3$ | |
| 3.18 | $C_2H_5$ | $COCH_2OCOCH_3$ | |
| 3.19 | $C_3H_7$-n | $COCH_2OCOCH_3$ | |
| 3.20 | $C_6H_{13}$-n | $COCH_2OCOCH_3$ | |
| 3.21 | $CH_3$ | $COCHFOCOCH_3$ | |
| 3.22 | $CH_3$ | $COCH(CH_3)OCOCH_3$ | |
| 3.23 | $CH_3$ | $COCH_2OCOCH_2Cl$ | |
| 3.24 | $CH_3$ | $COCH_2OCOC_2H_5$ | |
| 3.25 | $CH_3$ | $COCH_2OCOC_6H_4F(3)$ | |
| 3.26 | $C_2H_5$ | $COCH_2OCOC_6H_4Cl(3)$ | |
| 3.27 | $CH_3$ | $COCH_2OCOC_6H_4OCH_3(3)$ | |
| 3.28 | $CH_3$ | $COCH_2OCOC_6H_3Cl_2(2,4)$ | |
| 3.29 | $CH_3$ | $COCH_2OCOC_3H_7$-n | |
| 3.30 | $C_2H_5$ | $COCH_2OCH_2OCH_3$ | |

Tabelle 3 (Fortsetzung)

| Verb.Nr. | R | $R_1$ |
|---|---|---|
| 3.31 | $CH_3$ | $COCH_2OCO$– 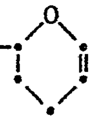 |
| 3.32 | $C_2H_5$ | $COCH_2OCO$– 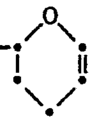 |
| 3.33 | $CH_3$ | $COCH_2$ 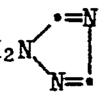 |
| 3.34 | $C_2H_5$ | $COCH_2$ 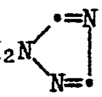 |
| 3.35 | $CH_3$ | $COCH_2$ 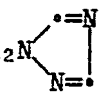 x 1/2 $ZnCl_2$ |
| 3.36 | $C_2H_5$ | $COCH_2$ 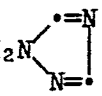 x 1/2 $ZnCl_2$ |
| 3.37 | $CH_3$ | $COCH_2$ 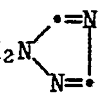 x 1/2 $CuCl_2$ |
| 3.38 | $C_2H_5$ | $COCH_2$ 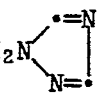 x 1/2 $CuCl_2$ |
| 3.39 | $CH_3$ | $COCH_2OCO$– 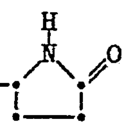 |
| 3.40 | $C_2H_5$ | $COCH_2OCO$– 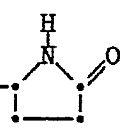 |
| 3.41 | $CH_3$ | $COCH_2OCO$– 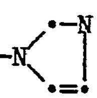 |
| 3.42 | $C_2H_5$ | $COCH_2OCO$– 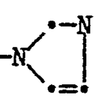 |
| 3.43 | $CH_3$ | $COCH_2OCH_3$ |
| 3.44 | $CH_3$ | $COCH_2OCOC_6H_4Cl(3)$ |

Tabelle 4: Typische Vertreter von Verbindungen der Formel I, worin $R_2$ für sek.-Butyl steht (= 13-Deoxi-22,23-dihydro-C-076-Bla-aglycon-derivate):

| Verb.Nr. | R | $R_1$ | |
|---|---|---|---|
| 4.1 | $CH_3$ | $COCH_3$ | |
| 4.2 | $C_2H_5$ | $COCH_3$ | |
| 4.3 | $C_3H_7$-n | $COCH_3$ | |
| 4.4 | $C_3H_7$-i | $COCH_3$ | |
| 4.5 | $C_4H_9$-n | $COCH_3$ | |
| 4.6 | $C_6H_{13}$-n | $COCH_3$ | |
| 4.7 | $C_{10}H_{21}$-n | $COCH_3$ | |
| 4.8 | $CH_3$ | $COCH_2Cl$ | |
| 4.9 | $CH_3$ | $COCH_2Br$ | |
| 4.10 | $CH_3$ | $COCH_2F$ | |
| 4.11 | $CH_3$ | $COCH_2BrF$ | |
| 4.12 | $C_2H_5$ | $COCH_2F$ | |
| 4.13 | $CH_3$ | $COCH_2CH_3$ | |
| 4.14 | $C_2H_5$ | $COCH_2OCH_3$ | |
| 4.15 | $CH_3$ | $COCH_2SCH_3$ | |
| 4.16 | $CH_3$ | $COCH_2OC_2H_5$ | |
| 4.17 | $CH_3$ | $COCH_2OCOCH_3$ | |
| 4.18 | $C_2H_5$ | $COCH_2OCOCH_3$ | |
| 4.19 | $C_3H_7$-n | $COCH_2OCOCH_3$ | |
| 4.20 | $C_6H_{13}$-n | $COCH_2OCOCH_3$ | |
| 4.21 | $CH_3$ | $COCHFOCOCH_3$ | |
| 4.22 | $CH_3$ | $COCH(CH_3)OCOCH_3$ | |
| 4.23 | $CH_3$ | $COCH_2OCOCH_2Cl$ | |
| 4.24 | $CH_3$ | $COCH_2OCOC_2H_5$ | |
| 4.25 | $CH_3$ | $COCH_2OCOC_6H_4F(3)$ | |
| 4.26 | $C_2H_5$ | $COCH_2OCOC_6H_4Cl(3)$ | |
| 4.27 | $CH_3$ | $COCH_2OCOC_6H_4OCH_3(3)$ | |
| 4.28 | $CH_3$ | $COCH_2OCOC_6H_3Cl_2(2,4)$ | |
| 4.29 | $CH_3$ | $COCH_2OCOC_3H_7$-n | |
| 4.30 | $C_2H_5$ | $COCH_2OCH_2OCH_3$ | |

Tabelle 4 (Fortsetzung)

| Verb.Nr. | R | $R_1$ |
|---|---|---|
| 4.31 | $CH_3$ | 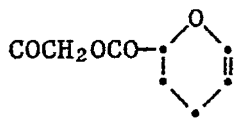 |
| 4.32 | $C_2H_5$ | 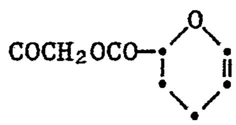 |
| 4.33 | $CH_3$ | 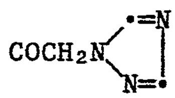 |
| 4.34 | $C_2H_5$ | 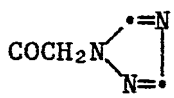 |
| 4.35 | $CH_3$ | 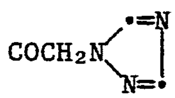 x 1/2 $ZnCl_2$ |
| 4.36 | $C_2H_5$ | 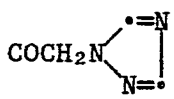 x 1/2 $ZnCl_2$ |
| 4.37 | $CH_3$ | 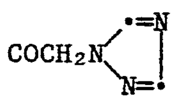 x 1/2 $CuCl_2$ |
| 4.38 | $C_2H_5$ | 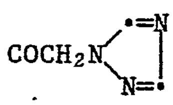 x 1/2 $CuCl_2$ |
| 4.39 | $CH_3$ | 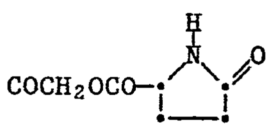 |
| 4.40 | $C_2H_5$ | 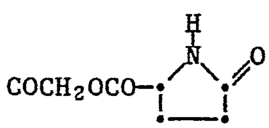 |
| 4.41 | $CH_3$ | 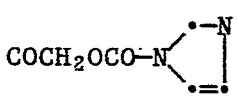 |
| 4.42 | $C_2H_5$ | 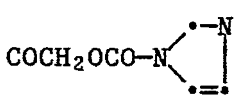 |
| 4.43 | $CH_3$ | $COCH_2OCH_3$ |
| 4.44 | $CH_3$ | $COCH_2OCOC_6H_4Cl(3)$ |

Tabelle 5: Typische Vertreter von Ausgangs-Verbindungen der Formel II, worin $R_1$ für Wasserstoff steht, sind:

| Verb. Nr. | $R_2$ | R |
|---|---|---|
| 5.1 | $CH_3$ | $CH_3$ |
| 5.2 | $CH_3$ | $C_2H_5$ |
| 5.3 | $CH_3$ | $C_3H_7$-n |
| 5.4 | $CH_3$ | $C_3H_7$-iso |
| 5.5 | $CH_3$ | $C_4H_9$-n |
| 5.6 | $CH_3$ | $C_4H_9$-sek |
| 5.7 | $CH_3$ | $C_4H_9$-iso |
| 5.8 | $CH_3$ | $C_5H_{11}$-n |
| 5.9 | $CH_3$ | $C_6H_{13}$-n |
| 5.10 | $CH_3$ | $C_7H_{15}$-n |
| 5.11 | $CH_3$ | $C_8H_{17}$-n |
| 5.12 | $CH_3$ | $C_9H_{18}$-n |
| 5.13 | $CH_3$ | $C_{10}H_{21}$-n |
| 5.14 | $C_2H_5$ | $CH_3$ |
| 5.15 | $C_2H_5$ | $C_2H_5$ |
| 5.16 | $C_2H_5$ | $C_3H_7$-iso |
| 5.17 | $C_2H_5$ | $C_4H_9$-n |
| 5.18 | $C_2H_5$ | $C_4H_9$-sek |
| 5.19 | $C_2H_5$ | $C_4H_9$-iso |
| 5.20 | $C_2H_5$ | $C_5H_{11}$-n |
| 5.21 | $C_2H_5$ | $C_6H_{13}$-n |
| 5.22 | $C_3H_7$-iso | $CH_3$ |
| 5.23 | $C_3H_7$-iso | $C_2H_5$ |
| 5.24 | $C_3H_7$-iso | $C_3H_7$-n |
| 5.25 | $C_3H_7$-iso | $C_3H_7$-iso |
| 5.26 | $C_3H_7$-iso | $C_4H_9$-n |
| 5.27 | $C_3H_7$-iso | $C_4H_9$-sek |
| 5.28 | $C_3H_7$-iso | $C_4H_9$-iso |
| 5.29 | $C_4H_9$-sek | $CH_3$ |
| 5.30 | $C_4H_9$-sek | $C_2H_5$ |

Tabelle 5 (Fortsetzung)

| Verb. Nr. | $R_2$ | R |
|---|---|---|
| 5.31 | $C_4H_9$-sek | $C_3H_7$-n |
| 5.32 | $C_4H_9$-sek | $C_3H_7$-iso |
| 5.33 | $C_4H_9$-sek | $C_4H_9$-n |
| 5.34 | $C_4H_9$-sek | $C_4H_9$-sek |
| 5.35 | $C_4H_9$-sek | $C_4H_9$-tert |
| 5.36 | $C_4H_9$-sek | $C_4H_9$-iso |
| 5.37 | $C_3H_7$-iso | $C_6H_{13}$-n |
| 5.38 | $C_3H_7$-iso | $C_5H_{11}$-n |
| 5.39 | $C_2H_5$ | $C_3H_7$-n |

Formulierungsbeispiele für den Wirkstoff der Formel I

(% = Gewichtsprozent)

| Spritzpulver | a) | b) | c) |
|---|---|---|---|
| Wirkstoff aus den Tabellen | 25 % | 50 % | 75 % |
| Na-Ligninsulfonat | 5 % | 5 % | - |
| Na-Laurylsulfat | 3 % | - | 5 % |
| Na-Diisobutylnaphthalinsulfonat | - | 6 % | 10 % |
| Octylphenolpolyethylenglykol-ether (7-8 Mol AeO) | - | 2 % | - |
| Hochdisperse Kieselsäure | 5 % | 10 % | 10 % |
| Kaolin | 62 % | 27 % | - |

Der Wirkstoff wird mit den Zusatzstoffen gut vermischt und in einer geeigneten Mühle gut vermahlen. Man erhält Spritzpulver, die sich mit Wasser zu Suspensionen jeder gewünschten Konzentration verdünnen lassen.

Emulsions-Konzentrat

Wirkstoff aus den Tabellen 10 %
Octylphenolpolyethylenglykolether (4-5 Mol AeO) 3 %
Ca-Dodecylbenzolsulfonat 3 %
Ricinusölpolyglykolether (36 Mol AeO) 4 %
Cyclohexanon 30 %
Xylolgemisch 50 %

Aus diesem Konzentrat können durch Verdünnen mit Wasser Emulsionen jeder gewünschten Konzentration hergestellt werden.

| Stäubemittel | a) | b) |
|---|---|---|
| Wirkstoff aus den Tabellen | 5 % | 8 % |
| Talkum | 95 % | - |
| Kaolin | - | 92 % |

Man erhält anwendungsfertige Stäubemittel, indem der Wirkstoff mit dem Träger vermischt und auf einer geeigneten Mühle vermahlen wird.

Extruder Granulat

Wirkstoff aus den Tabellen 10 %
Na-Ligninsulfonat 2 %
Carboxymethylcellulose 1 %
Kaolin 87 %

Der Wirkstoff wird mit den Zusatzstoffen vermischt, vermahlen und mit Wasser angefeuchtet. Dieses Gemisch wird extrudiert und anschliessend im Luftstrom getrocknet.

Tabellen bzw. Boli

I Ein Wirkstoff aus den Tabellen 33,0 %
Methylcellulose 0,80 %
Kieselsäure hochdispers 0,80 %
Maisstärke 8,40 %

Methylcellulose in Wasser einrühren und quellen lassen; Kieselsäure in die Quellung einrühren und homogen suspendieren. Wirkstoff und Maisstärke mischen. In diese Mischung die wässrige Suspension einarbeiten und zu einem Teig kneten. Diese Masse durch ein Sieb (Maschenweite 12 M) granulieren und dann trocknen.

II Milchzucker krist. 22,50 %
Maisstärke 17,00 %
mikrokrist. Cellulose 16,50 %
Magnesiumstearat 1,00 %

Alle 4 Hilfsstoffe gut mischen

Phasen I und II mischen und zu Tabletten oder Boli verpressen.

Falls die Verbindungen der Formel I bzw. entsprechende Mittel zur Bekämpfung von endoparasitären Nematoden, Cestoden und Trematoden bei Haus-und Nutztieren, wie Rindern, Schafen, Ziegen, Katzen und Hunden, verwendet werden, können sie den Tieren sowohl als Einzeldosis wie auch wiederholt verabreicht werden, wobei die einzelnen Gaben je nach Tierart vorzugsweise zwischen 0,1 und 10 mg pro kg Körpergewicht betragen. Durch eine protrahierte Verabreichung erzielt man in manchen Fällen eine bessere Wirkung oder man kann mit geringeren Gesamtdosen auskommen. Der Wirkstoff bzw. die ihn enthaltenden Mittel können auch dem Futter oder den Tränken zugesetzt werden. Das Fertigfutter enthält die Wirkstoffkombinationen vorzugsweise in einer Konzentration von 0,005 bis 0,1 Gew. %. Die Mittel können in Form von Lösungen, Emulsionen, Suspensionen, Pulver, Tabletten, Bolussen oder Kapseln peroral den Tieren verabreicht werden. Soweit die physikalischen und toxikologischen Eigenschaften von Lösungen oder Emulsionen dies zulassen, können die Verbindungen der Formel I bzw. die enthaltende Mittel an Tieren auch beispielsweise subcutan injiziert, intraruminal verabreicht oder mittels der Pour-on-Methode auf den Körper der Tiere appliziert werden. Ferner ist eine Verabreichung des Wirkstoffs an die Tiere auch durch Lecksteine (Salz) oder Molasse-Blöcke möglich.

Biologische Beispiele

B-1. Insektizide Frassgift-Wirkung bei Spodoptera littoralis

Eingetopfte Baumwollpflanzen im 5-Blatt-Stadium werden mit einer acetonisch/wässrigen Versuchslösung besprüht, die 3, 12,5 oder 50 ppm der zu prüfenden Verbindung enthält.

Nach dem Antrocknen des Belags werden die Pflanzen mit ca. 30 Larven ($L_1$-Stadium) von Spodoptera littoralis besetzt. Pro Versuchsverbindung und pro Test-Spezies verwendet man zwei Pflanzen. Der Versuch wird bei ca. 24°C und 60 % relativer Luftfeuchtigkeit durchgeführt. Auswertungen und Zwischenauswertungen auf moribunde Tiere, Wachstum und Larven und Frassschäden erfolgen nach 24 Stunden, 48 Stunden und 72 Stunden.

Verbindungen der Formeln I aus den Tabellen erzielten bereits bei einer Wirkstoffkonzentration von 3 ppm eine vollständige Abtötung nach 24 Stunden, so z.B. Nr. 2.8, 2.33, 2.43 und 2.35.

B-2. Wirkung gegen pflanzenschädigende Akariden OP-sensible Tetranychus urticae

Die Primärblätter von Bohnenpflanzen (Phaseolus vulgaris) werden 16 Stunden vor dem Versuch mit einem durch T. urticae infestierten, aus einer Massenzucht stammenden Blattstück belegt. Die so mit allen Milben-Stadien infestierten Pflanzen werden nach Entfernung des Blattstücks mit einer Versuchslösung bis zur Tropfnässe besprüht, die wahlweise 0,4 ppm oder 1,6 ppm der zu prüfenden Verbindung enthält. Die Temperatur in der Gewächshauskabine beträgt ca. 25°C.

Nach sieben Tagen wird unter dem Binokular auf Prozentsatz mobiler Stadien (Adulte und Nymphen) und auf vorhandene Eier ausgewertet.

Verbindungen der Formel I aus den Tabellen, wie z.B. die Verbindungen Nr. 2.8, 2.17 und 2.43 erzielten bereits bei einer Wirkstoffkonzentration von 0,4 ppm vollständige Abtötung.

B-3. Wirkung gegen $L_1$-Larven von Lucilia sericata

1 ml einer wässrigen Suspension der zu prüfenden Aktivsubstanz werden so mit 3 ml eines speziellen Larvenzuchtmediums bei ca. 50°C vermischt, dass ein Homogenisat von wahlweise 250 ppm oder 100 ppm Wirkstoffgehalt entsteht. In jede Reagenzglas-Probe werden ca. 30 Lucilia-Larven ($L_1$) eingesetzt. Nach 4 Tagen wird die Mortalitätsrate bestimmt. Verbindungen der Formel I aus den Tabellen, wie z.B. die Verbindungen Nr. 2.8, 2.17, 2.31, 2.33, 2.35, 2.43, 2.1, 2.18 und 2.45 erzielten mit 100 ppm eine Wirkung von 100 %.

B-4. Akarizide Wirkung gegen Boophilus microplus (Biarra-Stamm)

Auf einer PVC-Platte wird waagrecht ein Klebstreifen so befestigt, das darauf 10 mit Blut vollgesogene Zecken-Weibchen von Boophilus microplus (Biarra-Stamm) nebeneinander in einer Reihe mit dem Rücken aufgeklebt werden können. Jeder Zecke wird mit einer Injektionsnadel 1 µl einer Flüssigkeit injiziert, die eine 1:1-Mischung von Polyethylenglykol und Aceton darstellt und in der eine bestimmte Wirkstoffmenge von wahlweise 1, 0,1 oder 0,01 µg pro Zecke gelöst ist. Kontrolltiere erhalten eine wirkstofffreie Injektion. Nach der Behandlung werden die Tiere unter Normalbedingungen in einem Insektarium bei ca. 28°C und 80 % relativer Luftfeuchtigkeit gehalten, bis die Eiablage erfolgt und die Larven aus den Eiern der Kontrolltiere geschlüpft sind.

Die Aktivität einer geprüften Substanz wird mit der $IR_{90}$ bestimmt, d.h. es wird jene Wirkstoffdosis ermittelt, bei der noch nach 30 Tagen 9 von 10 Zeckenweibchen (= 90 %) Eier ablegen, die nicht schlupffähig sind.

Verbindungen der Formeln I aus den Tabellen, wie z.B. die Verbindungen Nr. 2.8 und 2.33 in diesem Versuch eine sehr gute Wirkung.

B-5. Versuch an mit Nematoden (Haemonchus concortus und Trichostrongylus colubriformis) infizierten Schafen

Der Wirkstoff wird als Suspension formuliert mit einer Magensonde oder durch Injektion in den Pansen eines Schafes gegeben, das mit Haemonchus concortus und Trychostrongylus colubriformis künstlich infiziert worden ist. Pro Dosis werden 1 bis 3 Tiere verwendet. Jedes Schaf wird nur einmal mit einer einzigen Dosis behandelt, und zwar wahlweise mit 0,5 mg oder 0,2 mg/kg Körpergewicht. Die Evaluierung erfolgt durch Vergleich der Anzahl der vor und nach Behandlung im Kot der Schafe ausgeschiedenen Wurmeier.

Gleichzeitig und gleichartig infizierte aber unbehandelte Schafe dienen als Kontrolle. Schafe, die mit einer der Verbindungen der Formeln I wie z.B. mit der Nr. 2.8, 2.17, 2.31, 2.33, 2.35, 2.43, 2.1, 2.18, 2.45, 2.44 und 2.53 bei 0,2 mg/kg behandelt wurden, zeigten im Vergleich zu unbehandelten, aber infizierten Vergleichsgruppen keinen Nematodenbefall (= komplette Reduktion der Wurmeier im Kot).

B-6. Kontaktwirkung auf Aphis craccivora

Erbsenkeimlinge, die mit sämtlichen Entwicklungsstadien der Laus infiziert sind, werden mit einer aus einem Emulsionskonzentrat hergestellten Wirkstofflösung besprüht, die wahlweise 50 ppm, 25 ppm oder 12,5 ppm Wirkstoff enthält. Nach 3 Tagen wird auf mehr als 80 % tote bzw. abgefallene Blattläuse ausgewertet. Nur bei dieser Wirkungshöhe wird ein Präparat als wirksam eingestuft.

Verbindungen der Formel I aus den Tabellen, wie z.B. die Verbindungen Nr. 2.8, 2.17, 2.33, 2.35 und 2.43 erzielten bei einer Konzentration von 12,5 ppm eine vollständige Abtötung (= 100 %).

B-7. Larvizidwirkung gegen Aedes aegypti

Auf die Oberfläche von 150 ml Wasser, das sich in einem Behälter befindet, wird soviel einer 0,1 %igen acetonischen Lösung des Wirkstoffes pipettiert, dass Konzentrationen von wahlweise 10 ppm, 3,3 ppm und 1,6 ppm erhalten werden. Nach Verdunsten des Acetons wird der Behälter mit ca. 30-40 3 Tage alten Aedes-Larven beschickt. Nach 1, 2 und 5 Tagen wird die Mortalität geprüft.

Die Verbindungen der Formeln I aus den Tabellen, wie z.B. die Verbindungen Nr. 2.8, 2.17, 2.31, 2.33, 2.1, 2.18 und 2.43 bewirkten in diesem Test bei einer Konzentration von 1,6 ppm bereits nach einem Tag eine vollständige Abtötung sämtlicher Larven.

B-8. Bodennematizide Wirkung gegen Meloidogyne incognita

Eier von Meloidogyne incognita werden in Sand eingemischt. Dieses Gemisch wird dann in 200 ml fassende Tontöpfe eingebracht (5000 Eier pro Topf). Am gleichen Tag wird pro Topf eine 3 Wochen alte Tomatenpflanze gepflanzt und der formulierte Wirkstoff mittels Druckapplikation in die Töpfe hineingegeben (0,002 % Aktivsubstanz bezogen auf das Bodenvolumen). Die eingetopften Pflanzen werden nun bei einer Temperatur von 26±1 °C und einer relativen Luftfeuchtigkeit von 60 % in einem Gewächshaus aufgestellt. Nach Ablauf von 4 Wochen erfolgt eine Evaluierung durch Untersuchung der Pflanzen auf Wurzelgallbildung nach dem sogenannten Wurzelgall-Index ("Knot Index")

Die Verbindungen der Herstellungsbeispiele zeigen gegen Meloidogyne incognita durch weitgehende Reduktion der Wurzelgallbildung gute Wirksamkeit. Unbehandelte jedoch infizierte Kontrollpflanzen weisen dagegen starke Wurzelgallbildung auf (= 100 %). So hemmen z.B. die Verbindungen Nr. 2.17, 2.33 und 2.18 im obigen Versuch die Wurzelgallbildung fast vollständig (0-10 % Restbefall).

B-9. Milbizide Wirkung gegen Dermanyssus gallinae

2 bis 3 ml einer Testlösung (100, 10, 1 und 0,1 ppm Aktivsubstanz) werden in einen nach oben offenen Glasbehälter gegeben und ca. 200 Milben in unterschiedlichen Entwicklungsstadien in diesen Behälter eingesetzt. Der Glasbehälter wird mit einem Wattebausch verschlossen und 10 Minuten gleichmässig, bis zur vollständigen Benetzung der Milben geschüttelt. Danach wird der Behälter umgekehrt hingestellt, bis die überschüssige Testlösung von der Watte aufgesogen ist. Der Behälter wird erneut gekehrt und die behandelten Zecken zur Evaluierung der Wirksamkeit der Testsubstanzen drei Tage unter Laborbedingungen beobachtet, wobei die Mortalität als Massstab für Wirksamkeit herangezogen wird.

Verbindungen aus den Tabellen, wie z.B. die Verbindungen Nr. 2.8, 2.17, 2.31, 2.33, 2.35, 2.48, 2.51 und 2.55 zeigen bei 100 ppm eine Wirkung von 100 %.

## Ansprüche

1. 5-Acyloxy-13β-alkyl-milbemycine der allgemeinen Formel I

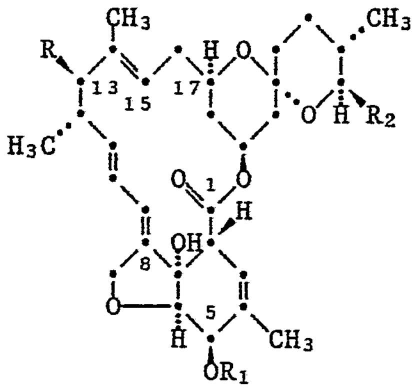

(I)

worin
R für $C_1$-$C_{10}$-Alkyl steht;
$R_1$ eine Acylgruppe darstellt;
und $R_2$ für Methyl, Ethyl, Isopropyl oder sek.-Butyl steht; gegebenenfalls unter Einschluss ihrer Säureadditionssalze und Metallkomplexe.

2. Verbindungen der Formel I nach Anspruch 1, worin
R für $C_1$-$C_6$-Alkyl steht;
$R_2$ für Methyl, Ethyl, Isopropyl oder sek.-Butyl steht;
$R_1$ eine der Acylgruppen

a) 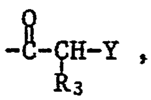 ,

b) 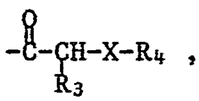 ,

c) 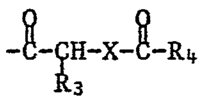 oder

d) 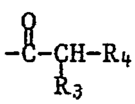

repräsentiert, wobei
X für Sauerstoff oder Schwefel steht;
Y eine nukleophil ersetzbare Abgangsgruppe darstellt;
$R_3$ Wasserstoff, $C_1$-$C_4$-Alkyl oder Halogen bedeutet; und
$R_4$ für Wasserstoff, für unsubstituiertes oder durch Halogen, Hydroxy, $C_1$-$C_6$-Alkoxy oder $C_1$-$C_6$-Haloalkoxy substituiertes $C_1$-$C_{10}$-Alkyl, für einen unsubstituierten oder substituierten Rest ausgewählt aus der Reihe $C_3$-$C_{10}$-Cycloalkyl, $C_2$-$C_6$-Alkenyl und $C_3$-$C_6$-Alkinyl, wobei die Substituenten ausgewählt sind aus der Reihe Halogen, Hydroxy, $C_1$-$C_6$-Alkoxy und $C_1$-$C_6$-Alkanoyloxy, für unsubstituiertes oder durch Halogen, Cyano, $C_1$-$C_3$-Alkyl, $C_1$-$C_3$-Haloalkyl, $C_1$-$C_3$-Alkoxy, $C_1$-$C_3$-Haloalkoxy und/oder Nitro substituiertes Phenyl oder für einen unsubstituierten oder substituierten, ungesättigten oder gesättigten, fünf-oder sechsgliedrigen heterocyclischen Ring mit ein bis drei Heteroatomen, ausgewählt aus der Reihe Stickstoff, Sauerstoff und Schwefel, dessen Substituenten ausgewählt sind aus der Reihe Oxo, Halogen, $C_1$-$C_4$-Alkyl, $C_1$-$C_4$-Haloalkyl und $C_1$-$C_4$-Alkoxy steht, unter Einschluss der Säureadditionssalze und Metallkomplexe.

3. Verbindungen der Formel I nach Anspruch 2, worin
R für $C_1$-$C_6$-Alkyl steht;
$R_2$ für Methyl, Ethyl, Isopropyl oder sek.-Butyl steht;
$R_1$ eine der Acylgruppen

a) 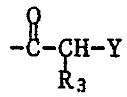 ,

b) 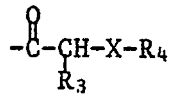 ,

c) 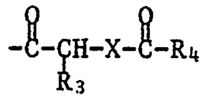 oder

d) 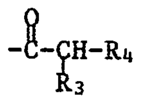 repräsentiert, wobei

X für Sauerstoff oder Schwefel steht;
Y für Halogen, Azido oder einen Sulfonsäurerest steht;
$R_3$ für Wasserstoff, Fluor oder Methyl steht; und
$R_4$ für Wasserstoff, für unsubstituiertes oder durch Fluor, Chlor, Brom, Hydroxy, $C_1$-$C_3$-Alkoxy oder $C_1$-$C_3$-Haloalkoxy substituiertes $C_1$-$C_6$-Alkyl, für einen unsubstituierten oder substituierten Rest ausgewählt aus der Reihe $C_3$-$C_7$-Cycloalkyl, $C_2$-$C_4$-Alkenyl und $C_2$-$C_4$-Alkinyl, wobei die Substituenten ausgewählt sind aus der Reihe Fluor, Chlor, Brom, Hydroxy, $C_1$-$C_3$-Alkoxy und $C_1$-$C_4$-Alkanoyloxy, für unsubstituiertes oder Fluor, Chlor, Brom, Methyl, Ethyl, Methoxy, Halomethoxy, Trifluormethyl und/oder Nitro substituiertes Phenyl oder für einen unsubstituierten oder substituierten, ungesättigten oder gesättigten, fünf-oder sechsgliedrigen heterocyclischen Ring mit ein bis drei Heteroatomen, ausgewählt aus der Reihe Stickstoff, Sauerstoff und Schwefel, dessen Substituenten ausgewählt sind aus der Reihe Oxo, Halogen, $C_1$-$C_4$-Alkyl, $C_1$-$C_4$-Haloalkyl und $C_1$-$C_4$-Alkoxy steht, unter Einschluss der Säureadditionssalze und Metallkomplexe.

4. Verbindungen der Formel I nach Anspruch 3, worin
R für $C_1$-$C_6$-Alkyl steht;
$R_2$ für Methyl, Ethyl, Isopropyl oder sek.-Butyl steht;
$R_1$ die Gruppe

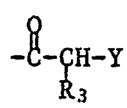

$R_3$ für Wasserstoff, Fluor oder Methyl steht; und
Y Chlor, Brom, Jod, Benzolsulfonyloxy, Paratosyloxy oder Mesyloxy bedeutet.

5. Verbindungen der Formel I nach Anspruch 3, worin
R für $C_1$-$C_6$-Alkyl steht;
$R_1$ für Methyl, Ethyl, Isopropyl oder sek.-Butyl steht;
$R_2$ die Gruppe

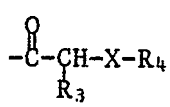

repräsentiert; wobei
X für Sauerstoff oder Schwefel steht;
$R_3$ Wasserstoff, Fluor oder Methyl bedeutet; und
$R_4$ für Wasserstoff, für unsubstituiertes oder durch Fluor, Chlor, Brom, Hydroxy, $C_1$-$C_3$-Alkoxy oder $C_1$-$C_3$-Haloalkoxy substituiertes $C_1$-$C_6$-Alkyl, für einen unsubstituierten oder substituierten Rest ausgewählt aus der Reihe $C_3$-$C_7$-Cycloalkyl, $C_2$-$C_4$-Alkenyl und $C_2$-$C_4$-Alkinyl, wobei die Substituenten ausgewählt sind aus der Reihe Fluor, Chlor, Brom, Hydroxy, $C_1$-$C_3$-Alkoxy und $C_1$-$C_4$-Alkanoyloxy, für unsubstituiertes oder Fluor, Chlor, Brom, Methyl, Ethyl, Methoxy, Halomethoxy, Trifluormethyl und/oder Nitro substituiertes Phenyl oder für einen unsubstituierten oder substituierten, ungesättigten oder gesättigten, fünf-oder sechsgliedrigen heterocyclischen Ring mit ein bis drei Heteroatomen, ausgewählt aus der Reihe Stickstoff, Sauerstoff und Schwefel, dessen Substituenten ausgewählt sind aus der Reihe Oxo, Halogen, $C_1$-$C_4$-Alkyl, $C_1$-$C_4$-Haloalkyl und $C_1$-$C_4$-Alkoxy steht, unter Einschluss der Säureadditionssalze und Metallkomplexe.

6. Verbindungen der Formel I nach Anspruch 3, worin
R für $C_1$-$C_6$-Alkyl steht;
$R_2$ für Methyl, Ethyl, Isopropyl oder sek.-Butyl steht;
$R_1$ die Gruppe

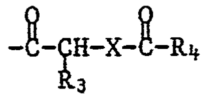

repräsentiert; wobei

X für Sauerstoff oder Schwefel steht;

$R_3$ Wasserstoff, Fluor oder Methyl bedeutet; und

$R_4$ für Wasserstoff, für unsubstituiertes oder durch Fluor, Chlor, Brom, Hydroxy, $C_1$-$C_3$-Alkoxy oder $C_1$-$C_3$-Haloalkoxy substituiertes $C_1$-$C_6$-Alkyl, für einen unsubstituierten oder substituierten Rest ausgewählt aus der Reihe $C_3$-$C_7$-Cycloalkyl, $C_2$-$C_4$-Alkenyl und $C_2$-$C_4$-Alkinyl, wobei die Substituenten ausgewählt sind aus der Reihe Fluor, Chlor, Brom, Hydroxy, $C_1$-$C_3$-Alkoxy und $C_1$-$C_4$-Alkanoyloxy, für unsubstituiertes oder Fluor, Chlor, Brom, Methyl, Ethyl, Methoxy, Halomethoxy, Trifluormethyl und/oder Nitro substituiertes Phenyl oder für einen unsubstituierten oder substituierten, ungesättigten oder gesättigten, fünf-oder sechsgliedrigen heterocyclischen Ring mit ein bis drei Heteroatomen, ausgewählt aus der Reihe Stickstoff, Sauerstoff und Schwefel, dessen Substituenten ausgewählt sind aus der Reihe Oxo, Halogen, $C_1$-$C_4$-Alkyl, $C_1$-$C_4$-Haloalkyl und $C_1$-$C_4$-Alkoxy steht, unter Einschluss der Säureadditionssalze und Metallkomplexe.

7. Verbindungen der Formel I nach Anspruch 3, worin

R für $C_1$-$C_6$-Alkyl steht;

$R_2$ für Methyl, Ethyl, Isopropyl oder sek.-Butyl steht;

$R_1$ die Gruppe

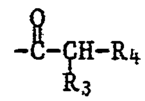

repräsentiert; wobei

$R_3$ für Wasserstoff, Fluor oder Methyl steht; und

$R_4$ für einen in 1-Position verknüpften, 5-gliedrigen Heteroaromaten mit 2 bis 3 Stickstoffatom steht, der unsubstituiert oder ein-bis dreifach durch $C_1$-$C_6$-Alkyl substituiert ist, steht, unter Einschluss der Säureadditionssalze mit organischen und anorganischen Säuren sowie der Metallkomplexe mit Metallkationen der ersten, zweiten, vierten oder achten Nebengruppe des Periodensystems der Elemente.

8. Verbindungen der Formel I nach Anspruch 1, worin

R für $C_1$-$C_6$-Alkyl steht;

$R_2$ für Methyl, Ethyl, Isopropyl oder sek.-Butyl steht;

$R_1$ eine der Acylgruppen

a) 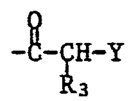 ,

b) 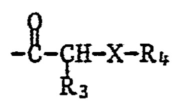 ,

c) 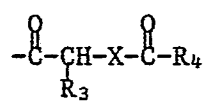 oder

d) 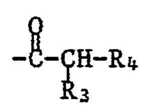 repräsentiert, wobei

X für Sauerstoff oder Schwefel steht;

Y Halogen bedeutet;

$R_3$ Wasserstoff, Halogen oder Methyl repräsentiert; und

$R_4$ für Wasserstoff, unsubstituiertes oder durch Halogen oder $C_1$-$C_4$-Alkoxy substituiertes $C_1$-$C_4$-Alkyl steht, für Cyclopropyl, für Cyclopentyl, für Cyclohexyl, für unsubstituiertes oder durch Fluor, Chlor, Brom, Methoxy, Trifluormethyl, Methyl und/oder Nitro substituiertes Phenyl, für 4H-2,3-Dihydropyran-2-yl oder für einen in 1-Position verknüpften, 5-gliedrigen Heteroaromaten mit 2 bis 3 Stickstoffatom steht, der unsubstituiert oder ein-bis dreifach durch $C_1$-$C_6$-Alkyl substituiert ist, steht, unter Einschluss der Säureadditionssalze mit organischen und anorganischen Säuren sowie der Metallkomplexe mit Metallkationen der ersten, zweiten, vierten oder achten Nebengruppe des Periodensystems der Elemente.

9. Eine Verbindung der Formel I nach Anspruch 1, ausgewählt aus der Reihe:
5-O-Chloracetyl-13β-methyl-milbemycin D,
5-O-Chloracetyl-13β-methyl-milbemycin $A_3$,
5-O-Chloracetyl-13β-methyl-milbemycin $A_4$,
5-O-Chloracetyl-13β-ethyl-milbemycin D,
5-O-Chloracetyl-13β-ethyl-milbemycin $A_3$,
5-O-Chloracetyl-13β-ethyl-milbemycin $A_4$,
5-O-Acetoxyacetyl-13β-methyl-milbemycin D,
5-O-Acetoxyacetyl-13β-methyl-milbemycin $A_3$,
5-O-Acetoxyacetyl-13β-methyl-milbemycin $A_4$,
5-O-Acetoxyacetyl-13β-ethyl-milbemycin D,
5-O-Acetoxyacetyl-13β-ethyl-milbemycin $A_3$,
5-O-Acetoxyacetyl-13β-ethyl-milbemycin $A_4$,
5-O-[3,4-Dihydro-2H-pyran-2-yl]carboxyacetyl-13β-methyl-milbemycin D,
5-O-[3,4-Dihydro-2H-pyran-2-yl]carboxyacetyl-13β-methyl-milbemycin $A_3$,
5-O-[3,4-Dihydro-2H-pyran-2-yl]carboxyacetyl-13β-methyl-milbemycin $A_4$,
5-O-[3,4-Dihydro-2H-pyran-2-yl]carboxyacetyl-13β-ethyl-milbemycin D,
5-O-[3,4-Dihydro-2H-pyran-2-yl]carboxyacetyl-13β-ethyl-milbemycin $A_3$,
5-O-[3,4-Dihydro-2H-pyran-2-yl]carboxyacetyl-13β-ethyl-milbemycin $A_4$,
5-O-[1,2,4-Triazol-4'-yl]acetyl-13β-methyl-milbemycin D,
5-O-[1,2,4-Triazol-4'-yl]acetyl-13β-methyl-milbemycin $A_3$,
5-O-[1,2,4-Triazol-4'-yl]acetyl-13β-methyl-milbemycin $A_4$,
5-O-[1,2,4-Triazol-4'-yl]acetyl-13β-ethyl-milbemycin D,
5-O-[1,2,4-Triazol-4'-yl]acetyl-13β-ethyl-milbemycin $A_3$,
5-O-[1,2,4-Triazol-4'-yl]acetyl-13β-ethyl-milbemycin $A_4$,
5-O-Methoxyacetyl-13β-methyl-milbemycin D,
5-O-Methoxyacetyl-13β-methyl-milbemycin $A_3$,
5-O-Methoxyacetyl-13β-methyl-milbemycin $A_4$,
5-O-Methoxyacetyl-13β-ethyl-milbemycin D,
5-O-Methoxyacetyl-13β-ethyl-milbemycin $A_3$,
5-O-Methoxyacetyl-13β-ethyl-milbemycin $A_4$,
5-O-[3-Chlorbenzoyloxy]acetyl-13β-methyl-milbemycin D,
5-O-[3-Chlorbenzoyloxy]acetyl-13β-methyl-milbemycin $A_3$,
5-O-[3-Chlorbenzoyloxy]acetyl-13β-methyl-milbemycin $A_4$,
5-O-[3-Chlorbenzoyloxy]acetyl-13β-ethyl-milbemycin D,
5-O-[3-Chlorbenzoyloxy]acetyl-13β-ethyl-milbemycin $A_3$, und
5-O-[3-Chlorbenzoyloxy]acetyl-13β-ethyl-milbemycin $A_4$.

10. Eine Verbindung der Formel I nach Anspruch 1, ausgewählt aus der Reihe:
5-O-Acetoxyacetyl-13β-propyl-milbemycin D,
5-O-Acetoxyacetyl-13β-propyl-milbemycin $A_3$,
5-O-Acetoxyacetyl-13β-propyl-milbemycin $A_4$,
5-O-Acetyl-13β-ethyl-milbemycin D,
5-O-Acetyl-13β-ethyl-milbemycin $A_3$,
5-O-Acetyl-13β-ethyl-milbemycin $A_4$,
5-O-Acetyl-13β-methyl-milbemycin D,
5-O-Acetyl-13β-methyl-milbemycin $A_3$,
5-O-Acetyl-13β-methyl-milbemycin $A_4$,
5-O-Methoxyacetyl-13β-butyl-milbemycin D,
5-O-Methoxyacetyl-13β-butyl-milbemycin $A_3$,
5-O-Methoxyacetyl-13β-butyl-milbemycin $A_4$,
5-O-Benzoyloxy-13β-methyl-milbemycin D,
5-O-Benzoyloxy-13β-methyl-milbemycin $A_3$,
5-O-Benzoyloxy-13β-methyl-milbemycin $A_4$.

11. Verfahren zur Herstellung von 5-Acyloxy-13β-alkyl-milbemycinen der allgemeinen Formel I

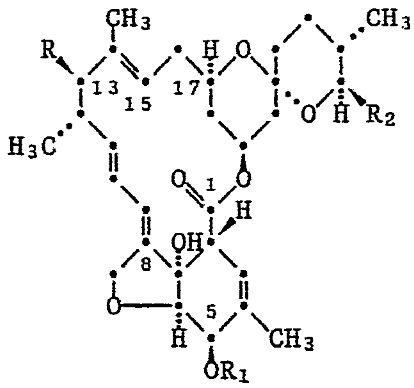

(I)

worin
R für $C_1$-$C_{10}$-Alkyl steht;
$R_1$ eine Acylgruppe darstellt; und $R_2$ für Methyl, Ethyl, Isopropyl oder sek.-Butyl steht; gegebenenfalls unter Einschluss ihrer Säureadditionssalze und Metallkomplexe, dadurch gekennzeichnet, dass man ein 13β-Alkyl-Milbemycin-Derivat der Formel II

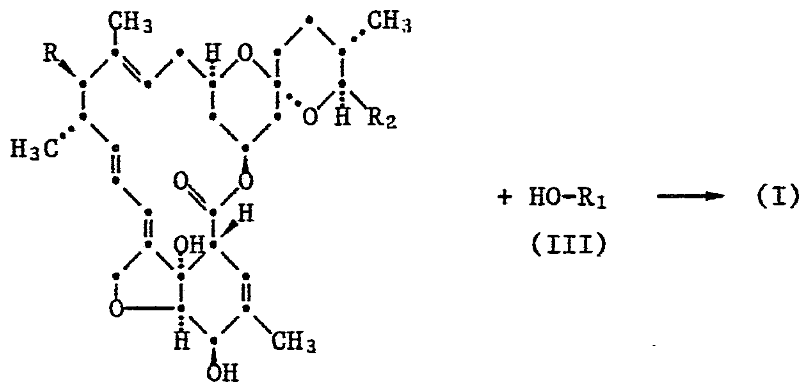

(III)

(II)

mit einer Säure der Formel III, einem ihrer Säurehalogenide oder ihrem Säureanhydrid an der 5-OH-Gruppe verestert, wobei die Substituenten R, $R_1$ und $R_2$ die unter Formel I angegebenen Bedeutungen haben.

12. Verfahren gemäss Anspruch 11, dadurch gekennzeichnet, dass man als Säurehalogenid der Säure der Formel III ihr Säurechlorid oder Säurebromid einsetzt.

13. Mittel zur Bekämpfung von Parasiten an Tieren oder an Pflanzen, dadurch gekennzeichnet, dass es neben üblichen Formulierungshilfsstoffen als Wirksubstanz mindestens eines der 5-Acyloxy-13β-alkyl-milbemycine der allgemeinen Formel I

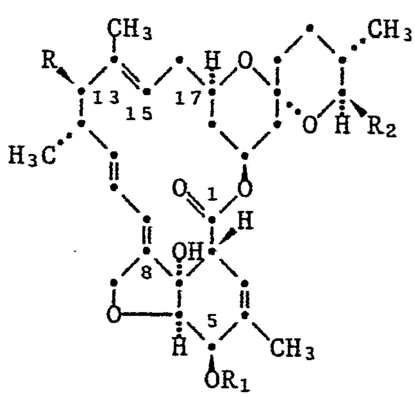

(I)

worin
R für $C_1$-$C_{10}$-Alkyl steht;
$R_1$ eine Acylgruppe darstellt;
und $R_2$ für Methyl, Ethyl, Isopropyl
oder sek.-Butyl steht; gegebenenfalls unter Einschluss ihrer Säureadditionssalze und Metallkomplexe, enthält.

14. Mittel nach Anspruch 13, dadurch gekennzeichnet, dass es als Wirkstoff mindestens eine der Verbindungen der Formel I gemäss einem der Ansprüche 2 bis 10 enthält.

15. Verwendung eines der 5-Acyloxy-13β-alkyl-milbemycine der allgemeinen Formel I

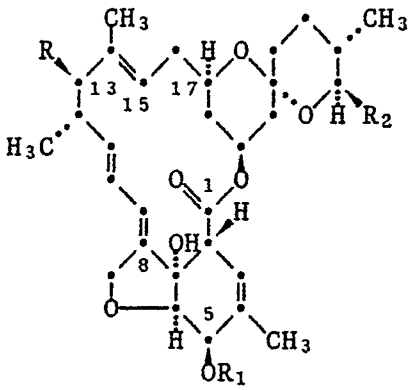

(I)

worin

R für $C_1$-$C_{10}$-Alkyl steht;

$R_1$ eine Acylgruppe darstellt; und $R_2$ für Methyl, Ethyl, Isopropyl oder sek.-Butyl steht; gegebenenfalls unter Einschluss ihrer Säureadditionssalze und Metallkomplexe zur Bekämpfung von Ekto-oder Endoparasiten bei Tieren oder gegen Pflanzenschädlinge.

16. Verwendung nach Anspruch 15, dadurch gekennzeichnet, dass es sich bei dem Wirkstoff um eine Verbindung der Formel I nach einem der Ansprüche 2 bis 10 handelt.

17. Verwendung nach einem der Ansprüche 15 und 16 zur Bekämpfung von Endoparasiten beim Nutztier.

18. Verwendung nach Anspruch 17 zur Bekämpfung von Nematoden.

19. Verfahren zur Bekämpfung von Schädlingen bei Tieren oder Pflanzen, dadurch gekennzeichnet, dass man eines der 5-Acyloxy-13β-alkyl-milbemycine der allgemeinen Formel I

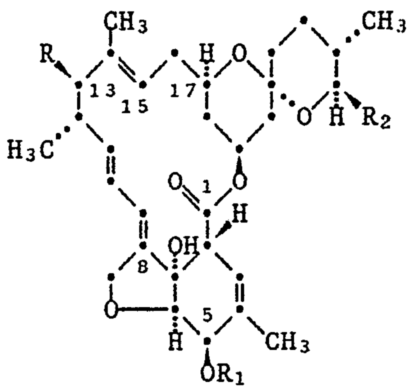

(I)

worin

R für $C_1$-$C_{10}$-Alkyl steht;

$R_1$ eine Acylgruppe darstellt; und $R_2$ für Methyl, Ethyl, Isopropyl oder sek.-Butyl steht; gegebenenfalls unter Einschluss ihrer Säureadditionssalze und Metallkomplexe auf oder in das Tier, auf die Pflanze oder den Lebensraum des Schädlings appliziert.

20. Verfahren nach Anspruch 19, dadurch gekennzeichnet, dass man die Applikation mit einer Verbindung der Formel I gemäss einem der Ansprüche 2 bis 10 durchführt.

Patentansprüche für den folgenden Vertragsstaat: AT

1. Verfahren zur Herstellung von 5-Acyloxy-13β-alkyl-milbemycinen der allgemeinen Formel I

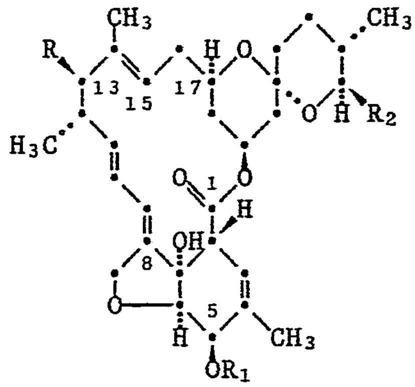

(I)

worin
R für $C_1$-$C_{10}$-Alkyl steht;
$R_1$ eine Acylgruppe darstellt; und $R_2$ für Methyl, Ethyl, Isopropyl oder sek.-Butyl steht; gegebenenfalls unter Einschluss ihrer Säureadditionssalze und Metallkomplexe, dadurch gekennzeichnet, dass man ein 13β-Alkyl-Milbemycin-Derivat der Formel II

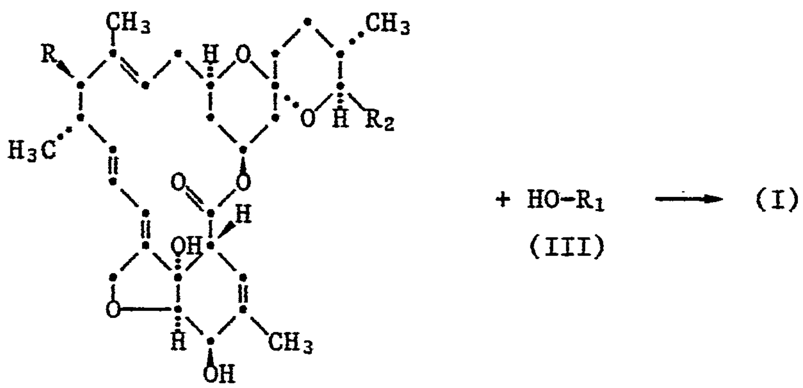


(II)

mit einer Säure der Formel III, einem ihrer Säurehalogenide oder ihrem Säureanhydrid an der 5-OH-Gruppe verestert, wobei die Substituenten R, $R_1$ und $R_2$ die unter Formel I angegebenen Bedeutungen haben.

2. Verfahren gemäss Anspruch 1, dadurch gekennzeichnet, dass man als Säurehalogenid der Säure der Formel III ihr Säurechlorid oder Säurebromid einsetzt.

3. Mittel zur Bekämpfung von Parasiten an Tieren oder an Pflanzen, dadurch gekennzeichnet, dass es neben üblichen Formulierungshilfsstoffen als Wirksubstanz mindestens eines der 5-Acyloxy-13β-alkyl-milbemycine der allgemeinen Formel I

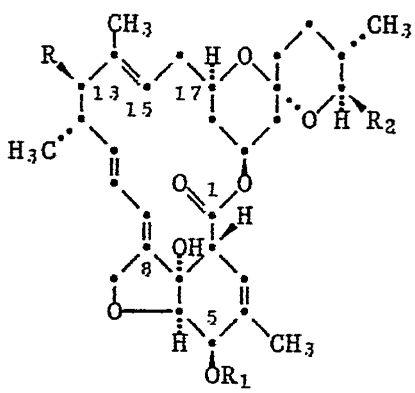

(I)

worin
R für $C_1$-$C_{10}$-Alkyl steht;
$R_1$ eine Acylgruppe darstellt;

und $R_2$ für Methyl, Ethyl, Isopropyl oder sek.-Butyl steht; gegebenenfalls unter Einschluss ihrer Säureadditionssalze und Metallkomplexe, enthält.

4. Mittel nach Anspruch 3, enthaltend als Wirkstoff eine der Verbindungen der Formel I, worin
R für $C_1$-$C_6$-Alkyl steht;
$R_2$ für Methyl, Ethyl, Isopropyl oder sek.-Butyl steht;
$R_1$ eine der Acylgruppen

a) 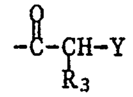 ,

b) 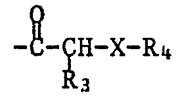 ,

c) 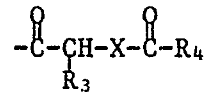 oder

d) 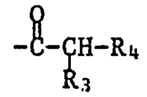 repräsentiert, wobei

X für Sauerstoff oder Schwefel steht;
Y eine nukleophil ersetzbare Abgangsgruppe darstellt;
$R_3$ Wasserstoff, $C_1$-$C_4$-Alkyl oder Halogen bedeutet; und
$R_4$ für Wasserstoff, für unsubstituiertes oder durch Halogen, Hydroxy, $C_1$-$C_6$-Alkoxy oder $C_1$-$C_6$-Haloalkoxy substituiertes $C_1$-$C_{10}$-Alkyl, für einen unsubstituierten oder substituierten Rest ausgewählt aus der Reihe $C_3$-$C_{10}$-Cycloalkyl, $C_2$-$C_6$-Alkenyl und $C_3$-$C_6$-Alkinyl, wobei die Substituenten ausgewählt sind aus der Reihe Halogen, Hydroxy, $C_1$-$C_6$-Alkoxy und $C_1$-$C_6$-Alkanoyloxy, für unsubstituiertes oder durch Halogen, Cyano, $C_1$-$C_3$-Alkyl, $C_1$-$C_3$-Haloalkyl, $C_1$-$C_3$-Alkoxy, $C_1$-$C_3$-Haloalkoxy und/oder Nitro substituiertes Phenyl oder für einen unsubstituierten oder substituierten, ungesättigten oder gesättigten, fünf-oder sechsgliedrigen heterocyclischen Ring mit ein bis drei Heteroatomen, ausgewählt aus der Reihe Stickstoff, Sauerstoff und Schwefel, dessen Substituenten ausgewählt sind aus der Reihe Oxo, Halogen, $C_1$-$C_4$-Alkyl, $C_1$-$C_4$-Haloalkyl und $C_1$-$C_4$-Alkoxy steht, unter Einschluss der Säureadditionssalze und Metallkomplexe.

5. Mittel nach Anspruch 4, enthaltend als Wirkstoff eine der Verbindungen der Formel I, worin R für $C_1$-$C_6$-Alkyl steht;
$R_2$ für Methyl, Ethyl, Isopropyl oder sek.-Butyl steht;
$R_1$ eine der Acylgruppen

a) 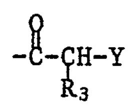 ,

b) 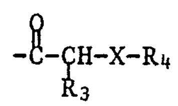 ,

c) 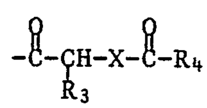 oder

d) 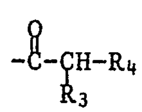 repräsentiert, wobei

X für Sauerstoff oder Schwefel steht;
Y für Halogen, Azido oder einen Sulfonsäurerest steht;
$R_3$ für Wasserstoff, Fluor oder Methyl steht; und
$R_4$ für Wasserstoff, für unsubstituiertes oder durch Fluor, Chlor, Brom, Hydroxy, $C_1$-$C_3$-Alkoxy oder $C_1$-$C_3$-Haloalkoxy substituiertes $C_1$-$C_6$-Alkyl, für einen unsubstituierten oder substituierten Rest ausgewählt aus der Reihe $C_3$-$C_7$-Cycloalkyl, $C_2$-$C_4$-Alkenyl und $C_2$-$C_4$-Alkinyl, wobei die Substituenten ausgewählt sind aus der

Reihe Fluor, Chlor, Brom, Hydroxy, $C_1$-$C_3$-Alkoxy und $C_1$-$C_4$-Alkanoyloxy, für unsubstituiertes oder Fluor, Chlor, Brom, Methyl, Ethyl, Methoxy, Halomethoxy, Trifluormethyl und/oder Nitro substituiertes Phenyl oder für einen unsubstituierten oder substituierten, ungesättigten oder gesättigten, fünf-oder sechsgliedrigen heterocyclischen Ring mit ein bis drei Heteroatomen, ausgewählt aus der Reihe Stickstoff, Sauerstoff und Schwefel, dessen Substituenten ausgewählt sind aus der Reihe Oxo, Halogen, $C_1$-$C_4$-Alkyl, $C_1$-$C_4$-Haloalkyl und $C_1$-$C_4$-Alkoxy steht, unter Einschluss der Säureadditionssalze und Metallkomplexe.

6. Mittel nach Anspruch 5, enthaltend als Wirkstoff eine der Verbindungen der Formel I, worin
R für $C_1$-$C_6$-Alkyl steht;
$R_2$ für Methyl, Ethyl, Isopropyl oder sek.-Butyl steht;
$R_1$ die Gruppe

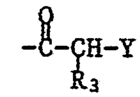

$R_3$ für Wasserstoff, Fluor oder Methyl steht; und
Y Chlor, Brom, Jod, Benzolsulfonyloxy, Paratosyloxy oder Mesyloxy bedeutet.

7. Mittel nach Anspruch 5, enthaltend als Wirkstoff eine der Verbindungen der Formel I, worin
R für $C_1$-$C_6$-Alkyl steht;
$R_1$ für Methyl, Ethyl, Isopropyl oder sek.-Butyl steht;
$R_2$ die Gruppe

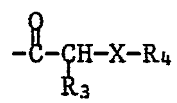

repräsentiert; wobei
X für Sauerstoff oder Schwefel steht;
$R_3$ Wasserstoff, Fluor oder Methyl bedeutet; und
$R_4$ für Wasserstoff, für unsubstituiertes oder durch Fluor, Chlor, Brom, Hydroxy, $C_1$-$C_3$-Alkoxy oder $C_1$-$C_3$-Haloalkoxy substituiertes $C_1$-$C_6$-Alkyl, für einen unsubstituierten oder substituierten Rest ausgewählt aus der Reihe $C_3$-$C_7$-Cycloalkyl, $C_2$-$C_4$-Alkenyl und $C_2$-$C_4$-Alkinyl, wobei die Substituenten ausgewählt sind aus der Reihe Fluor, Chlor, Brom, Hydroxy, $C_1$-$C_3$-Alkoxy und $C_1$-$C_4$-Alkanoyloxy, für unsubstituiertes oder Fluor, Chlor, Brom, Methyl, Ethyl, Methoxy, Halomethoxy, Trifluormethyl und/oder Nitro substituiertes Phenyl oder für einen unsubstituierten oder substituierten, ungesättigten oder gesättigten, fünf-oder sechsgliedrigen heterocyclischen Ring mit ein bis drei Heteroatomen, ausgewählt aus der Reihe Stickstoff, Sauerstoff und Schwefel, dessen Substituenten ausgewählt sind aus der Reihe Oxo, Halogen, $C_1$-$C_4$-Alkyl, $C_1$-$C_4$-Haloalkyl und $C_1$-$C_4$-Alkoxy steht, unter Einschluss der Säureadditionssalze und Metallkomplexe.

8. Mittel nach Anspruch 5, enthaltend als Wirkstoff eine der Verbindungen der Formel I, worin
R für $C_1$-$C_6$-Alkyl steht;
$R_2$ für Methyl, Ethyl, Isopropyl oder sek.-Butyl steht;
$R_1$ die Gruppe

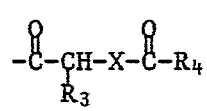

repräsentiert; wobei
X für Sauerstoff oder Schwefel steht;
$R_3$ Wasserstoff, Fluor oder Methyl bedeutet; und
$R_4$ für Wasserstoff, für unsubstituiertes oder durch Fluor, Chlor, Brom, Hydroxy, $C_1$-$C_3$-Alkoxy oder $C_1$-$C_3$-Haloalkoxy substituiertes $C_1$-$C_6$-Alkyl, für einen unsubstituierten oder substituierten Rest ausgewählt aus der Reihe $C_3$-$C_7$-Cycloalkyl, $C_2$-$C_4$-Alkenyl und $C_2$-$C_4$-Alkinyl, wobei die Substituenten ausgewählt sind aus der Reihe Fluor, Chlor, Brom, Hydroxy, $C_1$-$C_3$-Alkoxy und $C_1$-$C_4$-Alkanoyloxy, für unsubstituiertes oder Fluor, Chlor, Brom, Methyl, Ethyl, Methoxy, Halomethoxy, Trifluormethyl und/oder Nitro substituiertes Phenyl oder für einen unsubstituierten oder substituierten, ungesättigten oder gesättigten, fünf-oder sechsgliedrigen heterocyclischen Ring mit ein bis drei Heteroatomen, ausgewählt aus der Reihe Stickstoff, Sauerstoff und Schwefel, dessen Substituenten ausgewählt sind aus der Reihe Oxo, Halogen, $C_1$-$C_4$-Alkyl, $C_1$-$C_4$-Haloalkyl und $C_1$-$C_4$-Alkoxy steht, unter Einschluss der Säureadditionssalze und Metallkomplexe.

7. Mittel nach Anspruch 5, enthaltend als Wirkstoff eine der Verbindungen der Formel I, worin
R für $C_1$-$C_6$-Alkyl steht;
$R_2$ für Methyl, Ethyl, Isopropyl oder sek.-Butyl steht;
$R_1$ die Gruppe

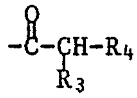

repräsentiert; wobei

$R_3$ für Wasserstoff, Fluor oder Methyl steht; und

$R_4$ für einen in 1-Position verknüpften, 5-gliedrigen Heteroaromaten mit 2 bis 3 Stickstoffatom steht, der unsubstituiert oder ein-bis dreifach durch $C_1$-$C_6$-Alkyl substituiert ist, steht, unter Einschluss der Säureadditionssalze mit organischen und anorganischen Säuren sowie der Metallkomplexe mit Metallkationen der ersten, zweiten, vierten oder achten Nebengruppe des Periodensystems der Elemente.

10. Mittel nach Anspruch 3, enthaltend als Wirkstoff ein der Verbindungen der Formel I, worin

R für $C_1$-$C_6$-Alkyl steht;

$R_2$ für Methyl, Ethyl, Isopropyl oder sek.-Butyl steht;

$R_1$ eine der Acylgruppen

a) 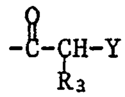 ,

b) 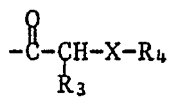 ,

c) 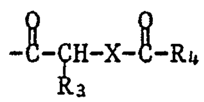 oder

d) 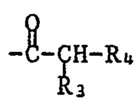 repräsentiert, wobei

X für Sauerstoff oder Schwefel steht;

Y Halogen bedeutet;

$R_3$ Wasserstoff, Halogen oder Methyl repräsentiert; und

$R_4$ für Wasserstoff, unsubstituiertes oder durch Halogen oder $C_1$-$C_4$-Alkoxy substituiertes $C_1$-$C_4$-Alkyl steht, für Cyclopropyl, für Cyclopentyl, für Cyclohexyl, für unsubstituiertes oder durch Fluor, Chlor, Brom, Methoxy, Trifluormethyl, Methyl und/oder Nitro substituiertes Phenyl, für 4H-2,3-Dihydropyran-2-yl oder für einen in 1-Position verknüpften, 5-gliedrigen Heteroaromaten mit 2 bis 3 Stickstoffatom steht, der unsubstituiert oder ein-bis dreifach durch $C_1$-$C_6$-Alkyl substituiert ist, steht, unter Einschluss der Säureadditionssalze mit organischen und anorganischen Säuren sowie der Metallkomplexe mit Metallkationen der ersten, zweiten, vierten oder achten Nebengruppe des Periodensystems der Elemente.

11. Mittel nach Anspruch 3, enthaltend als Wirkstoff eine der Verbindung der Formel I, ausgewählt aus der Reihe:

5-O-Chloracetyl-13β-methyl-milbemycin D,
5-O-Chloracetyl-13β-methyl-milbemycin $A_3$,
5-O-Chloracetyl-13β-methyl-milbemycin $A_4$,
5-O-Chloracetyl-13β-ethyl-milbemycin D,
5-O-Chloracetyl-13β-ethyl-milbemycin $A_3$,
5-O-Chloracetyl-13β-ethyl-milbemycin $A_4$,
5-O-Acetoxyacetyl-13β-methyl-milbemycin D,
5-O-Acetoxyacetyl-13β-methyl-milbemycin $A_3$,
5-O-Acetoxyacetyl-13β-methyl-milbemycin $A_4$,
5-O-Acetoxyacetyl-13β-ethyl-milbemycin D,
5-O-Acetoxyacetyl-13β-ethyl-milbemycin $A_3$,
5-O-Acetoxyacetyl-13β-ethyl-milbemycin $A_4$,
5-O-[3,4-Dihydro-2H-pyran-2-yl]carboxyacetyl-13β-methyl-milbemycin D,
5-O-[3,4-Dihydro-2H-pyran-2-yl]carboxyacetyl-13β-methyl-milbemycin $A_3$,
5-O-[3,4-Dihydro-2H-pyran-2-yl]carboxyacetyl-13β-methyl-milbemycin $A_4$,
5-O-[3,4-Dihydro-2H-pyran-2-yl]carboxyacetyl-13β-ethyl-milbemycin D,
5-O-[3,4-Dihydro-2H-pyran-2-yl]carboxyacetyl-13β-ethyl-milbemycin $A_3$,
5-O-[3,4-Dihydro-2H-pyran-2-yl]carboxyacetyl-13β-ethyl-milbemycin $A_4$,
5-O-[1,2,4-Triazol-4'-yl]acetyl-13β-methyl-milbemycin D,

5-O-[1,2,4-Triazol-4'-yl]acetyl-13β-methyl-milbemycin $A_3$,
5-O-[1,2,4-Triazol-4'-yl]acetyl-13β-methyl-milbemycin $A_4$,
5-O-[1,2,4-Triazol-4'-yl]acetyl-13β-ethyl-milbemycin D,
5-O-[1,2,4-Triazol-4'-yl]acetyl-13β-ethyl-milbemycin $A_3$,
5-O-[1,2,4-Triazol-4'-yl]acetyl-13β-ethyl-milbemycin $A_4$,
5-O-Methoxyacetyl-13β-methyl-milbemycin D,
5-O-Methoxyacetyl-13β-methyl-milbemycin $A_3$,
5-O-Methoxyacetyl-13β-methyl-milbemycin $A_4$,
5-O-Methoxyacetyl-13β-ethyl-milbemycin D,
5-O-Methoxyacetyl-13β-ethyl-milbemycin $A_3$,
5-O-Methoxyacetyl-13β-ethyl-milbemycin $A_4$,
5-O-[3-Chlorbenzoyloxy]acetyl-13β-methyl-milbemycin D,
5-O-[3-Chlorbenzoyloxy]acetyl-13β-methyl-milbemycin $A_3$,
5-O-[3-Chlorbenzoyloxy]acetyl-13β-methyl-milbemycin $A_4$,
5-O-[3-Chlorbenzoyloxy]acetyl-13β-ethyl-milbemycin D,
5-O-[3-Chlorbenzoyloxy]acetyl-13β-ethyl-milbemycin $A_3$, und
5-O-[3-Chlorbenzoyloxy]acetyl-13β-ethyl-milbemycin $A_4$.

12. Mittel nach Anspruch 3, enthaltend als Wirkstoff eine der Verbindung der Formel I, ausgewählt aus der Reihe:

5-O-Acetoxyacetyl-13β-propyl-milbemycin D,
5-O-Acetoxyacetyl-13β-propyl-milbemycin $A_3$,
5-O-Acetoxyacetyl-13β-propyl-milbemycin $A_4$,
5-O-Acetyl-13β-ethyl-milbemycin D,
5-O-Acetyl-13β-ethyl-milbemycin $A_3$,
5-O-Acetyl-13β-ethyl-milbemycin $A_4$,
5-O-Acetyl-13β-methyl-milbemycin D,
5-O-Acetyl-13β-methyl-milbemycin $A_3$,
5-O-Acetyl-13β-methyl-milbemycin $A_4$,
5-O-Methoxyacetyl-13β-butyl-milbemycin D,
5-O-Methoxyacetyl-13β-butyl-milbemycin $A_3$,
5-O-Methoxyacetyl-13β-butyl-milbemycin $A_4$,
5-O-Benzoyloxy-13β-methyl-milbemycin D,
5-O-Benzoyloxy-13β-methyl-milbemycin $A_3$,
5-O-Benzoyloxy-13β-methyl-milbemycin $A_4$.

13. Verwendung eines der 5-Acyloxy-<u>13β</u>-alkyl-milbemycine der allgemeinen Formel I

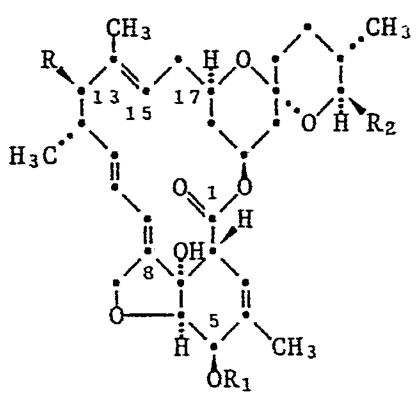

(I)

worin
R für $C_1$-$C_{10}$-Alkyl steht;
$R_1$ eine Acylgruppe darstellt; und $R_2$ für Methyl, Ethyl, Isopropyl oder sek.-Butyl steht; gegebenenfalls unter Einschluss ihrer Säureadditionssalze und Metallkomplexe zur Bekämpfung von Ekto-oder Endoparasiten bei Tieren oder gegen Pflanzenschädlinge.

14. Verwendung nach Anspruch 13, dadurch gekennzeichnet, dass es sich bei dem Wirkstoff um eine Verbindung der Formel I nach einem der Ansprüche 4 bis 11 handelt.

15. Verwendung nach einem der Ansprüche 13 und 14 zur Bekämpfung von Endoparasiten beim Nutztier.

16. Verwendung nach Anspruch 15 zur Bekämpfung von Nematoden.

17. Verfahren zur Bekämpfung von Schädlingen bei Tieren oder Pflanzen, dadurch gekennzeichnet, dass man eines der 5-Acyloxy-13β-alkyl-milbemycine der allgemeinen Formel I

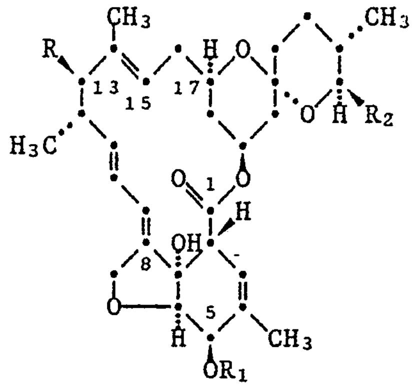

(I)

worin

R für $C_1$-$C_{10}$-Alkyl steht;

$R_1$ eine Acylgruppe darstellt; und $R_2$ für Methyl, Ethyl, Isopropyl oder sek.-Butyl steht; gegebenenfalls unter Einschluss ihrer Säureadditionssalze und Metallkomplexe, auf oder in das Tier, auf die Pflanze oder den Lebensraum des Schädlings appliziert.

18. Verfahren nach Anspruch 17, dadurch gekennzeichnet, dass man die Applikation mit einer Verbindung der Formel I gemäss einem der Ansprüche 4 bis 11 durchführt.

Patentansprüche für den folgenden Vertragsstaat: ES

1. Verfahren zur Herstellung von 5-Acyloxy-13β-alkyl-milbemycinen der allgemeinen Formel I

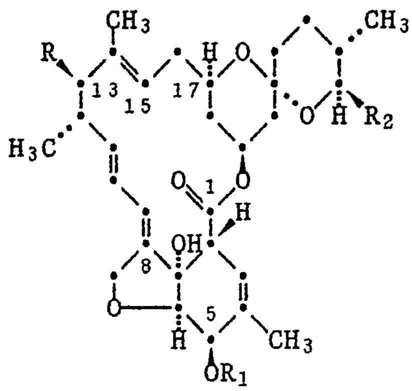

(I)

worin

R für $C_1$-$C_{10}$-Alkyl steht;

$R_1$ eine Acylgruppe darstellt; und $R_2$ für Methyl, Ethyl, Isopropyl oder sek.-Butyl steht; gegebenenfalls unter Einschluss ihrer Säureadditionssalze und Metallkomplexe, dadurch gekennzeichnet, dass man ein 13β-Alkyl-Milbemycin-Derivat der Formel II

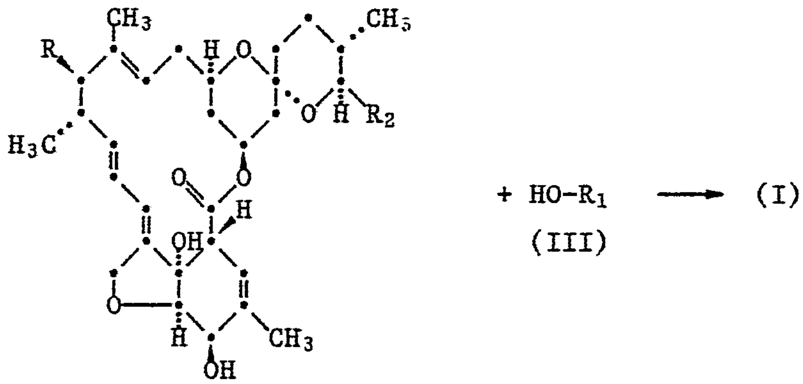


(II)

mit einer Säure der Formel III, einem ihrer Säurehalogenide oder ihrem Säureanhydrid an der 5-OH-Gruppe verestert, wobei die Substituenten R, $R_1$ und $R_2$ die unter Formel I angegebenen Bedeutungen haben.

2. Verfahren gemäss Anspruch 1, dadurch gekennzeichnet, dass man als Säurehalogenid der Säure der Formel III ihr Säurechlorid oder Säurebromid einsetzt.

3. Mittel zur Bekämpfung von Parasiten an Tieren oder an Pflanzen, dadurch gekennzeichnet, dass es neben üblichen Formulierungshilfsstoffen als Wirksubstanz mindestens eines der 5-Acyloxy-13β-alkyl-milbemycine der allgemeinen Formel I

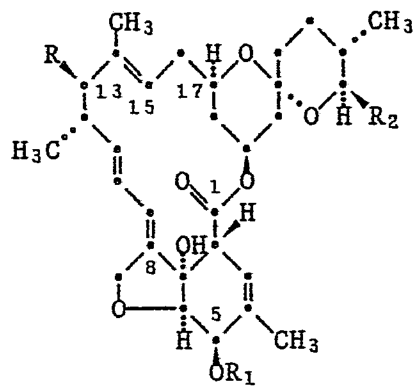

(I)

worin
R für $C_1$-$C_{10}$-Alkyl steht;
$R_1$ eine Acylgruppe darstellt;
und $R_2$ für Methyl, Ethyl, Isopropyl
oder sek.-Butyl steht; gegebenenfalls unter Einschluss ihrer Säureadditionssalze und Metallkomplexe, enthält.

4. Mittel nach Anspruch 3, enthaltend als Wirkstoff eine der Verbindungen der Formel I, worin
R für $C_1$-$C_6$-Alkyl steht;
$R_2$ für Methyl, Ethyl, Isopropyl oder sek.-Butyl steht;
$R_1$ eine der Acylgruppen

a) 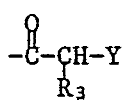 ,

b) 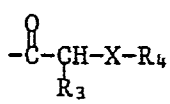 ,

c) 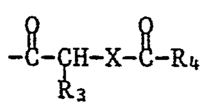 oder

d) 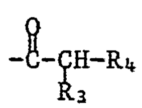 repräsentiert, wobei

X für Sauerstoff oder Schwefel steht;

Y eine nukleophil ersetzbare Abgangsgruppe darstellt;
$R_3$ Wasserstoff, $C_1$-$C_4$-Alkyl oder Halogen bedeutet; und
$R_4$ für Wasserstoff, für unsubstituiertes oder durch Halogen, Hydroxy, $C_1$-$C_6$-Alkoxy oder $C_1$-$C_6$-Haloalkoxy substituiertes $C_1$-$C_{10}$-Alkyl, für einen unsubstituierten oder substituierten Rest ausgewählt aus der Reihe $C_3$-$C_{10}$-Cycloalkyl, $C_2$-$C_6$-Alkenyl und $C_3$-$C_6$-Alkinyl, wobei die Substituenten ausgewählt sind aus der Reihe Halogen, Hydroxy, $C_1$-$C_6$-Alkoxy und $C_1$-$C_6$-Alkanoyloxy, für unsubstituiertes oder durch Halogen, Cyano, $C_1$-$C_3$-Alkyl, $C_1$-$C_3$-Haloalkyl, $C_1$-$C_3$-Alkoxy, $C_1$-$C_3$-Haloalkoxy und/oder Nitro substituiertes Phenyl oder für einen unsubstituierten oder substituierten, ungesättigten oder gesättigten, fünf-oder sechsgliedrigen heterocyclischen Ring mit ein bis drei Heteroatomen, ausgewählt aus der Reihe Stickstoff, Sauerstoff und Schwefel, dessen Substituenten ausgewählt sind aus der Reihe Oxo, Halogen, $C_1$-$C_4$-Alkyl, $C_1$-$C_4$-Haloalkyl und $C_1$-$C_4$-Alkoxy steht, unter Einschluss der Säureadditionssalze und Metallkomplexe.

5. Mittel nach Anspruch 4, enthaltend als Wirkstoff eine der Verbindungen der Formel I, worin R für $C_1$-$C_6$-Alkyl steht;
$R_2$ für Methyl, Ethyl, Isopropyl oder sek.-Butyl steht;
$R_1$ eine der Acylgruppen

a) 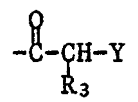 ,

b) 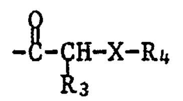 ,

c) 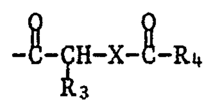 oder

d) 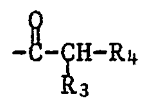 repräsentiert, wobei

X für Sauerstoff oder Schwefel steht;
Y für Halogen, Azido oder einen Sulfonsäurerest steht;
$R_3$ für Wasserstoff, Fluor oder Methyl steht; und
$R_4$ für Wasserstoff, für unsubstituiertes oder durch Fluor, Chlor, Brom, Hydroxy, $C_1$-$C_3$-Alkoxy oder $C_1$-$C_3$-Haloalkoxy substituiertes $C_1$-$C_6$-Alkyl, für einen unsubstituierten oder substituierten Rest ausgewählt aus der Reihe $C_3$-$C_7$-Cycloalkyl, $C_2$-$C_4$-Alkenyl und $C_2$-$C_4$-Alkinyl, wobei die Substituenten ausgewählt sind aus der Reihe Fluor, Chlor, Brom, Hydroxy, $C_1$-$C_3$-Alkoxy und $C_1$-$C_4$-Alkanoyloxy, für unsubstituiertes oder Fluor, Chlor, Brom, Methyl, Ethyl, Methoxy, Halomethoxy, Trifluormethyl und/oder Nitro substituiertes Phenyl oder für einen unsubstituierten oder substituierten, ungesättigten oder gesättigten, fünf-oder sechsgliedrigen heterocyclischen Ring mit ein bis drei Heteroatomen, ausgewählt aus der Reihe Stickstoff, Sauerstoff und Schwefel, dessen Substituenten ausgewählt sind aus der Reihe Oxo, Halogen, $C_1$-$C_4$-Alkyl, $C_1$-$C_4$-Haloalkyl und $C_1$-$C_4$-Alkoxy steht, unter Einschluss der Säureadditionssalze und Metallkomplexe.

6. Mittel nach Anspruch 5, enthaltend als Wirkstoff eine der Verbindungen der Formel I, worin
R für $C_1$-$C_6$-Alkyl steht;
$R_2$ für Methyl, Ethyl, Isopropyl oder sek.-Butyl steht;
$R_1$ die Gruppe

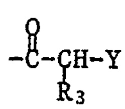

$R_3$ für Wasserstoff, Fluor oder Methyl steht; und
Y Chlor, Brom, Jod, Benzolsulfonyloxy, Paratosyloxy oder Mesyloxy bedeutet.

7. Mittel nach Anspruch 5, enthaltend als Wirkstoff eine der Verbindungen der Formel I, worin
R für $C_1$-$C_6$-Alkyl steht;
$R_1$ für Methyl, Ethyl, Isopropyl oder sek.-Butyl steht;
$R_2$ die Gruppe

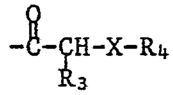

repräsentiert; wobei

X für Sauerstoff oder Schwefel steht;

$R_3$ Wasserstoff, Fluor oder Methyl bedeutet; und

$R_4$ für Wasserstoff, für unsubstituiertes oder durch Fluor, Chlor, Brom, Hydroxy, $C_1$-$C_3$-Alkoxy oder $C_1$-$C_3$-Haloalkoxy substituiertes $C_1$-$C_6$-Alkyl, für einen unsubstituierten oder substituierten Rest ausgewählt aus der Reihe $C_3$-$C_7$-Cycloalkyl, $C_2$-$C_4$-Alkenyl und $C_2$-$C_4$-Alkinyl, wobei die Substituenten ausgewählt sind aus der Reihe Fluor, Chlor, Brom, Hydroxy, $C_1$-$C_3$-Alkoxy und $C_1$-$C_4$-Alkanoyloxy, für unsubstituiertes oder Fluor, Chlor, Brom, Methyl, Ethyl, Methoxy, Halomethoxy, Trifluormethyl und/oder Nitro substituiertes Phenyl oder für einen unsubstituierten oder substituierten, ungesättigten oder gesättigten, fünf-oder sechsgliedrigen heterocyclischen Ring mit ein bis drei Heteroatomen, ausgewählt aus der Reihe Stickstoff, Sauerstoff und Schwefel, dessen Substituenten ausgewählt sind aus der Reihe Oxo, Halogen, $C_1$-$C_4$-Alkyl, $C_1$-$C_4$-Haloalkyl und $C_1$-$C_4$-Alkoxy steht, unter Einschluss der Säureadditionssalze und Metallkomplexe.

8. Mittel nach Anspruch 5, enthaltend als Wirkstoff eine der Verbindungen der Formel I, worin

R für $C_1$-$C_6$-Alkyl steht;

$R_2$ für Methyl, Ethyl, Isopropyl oder sek.-Butyl steht;

$R_1$ die Gruppe

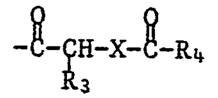

repräsentiert; wobei

X für Sauerstoff oder Schwefel steht;

$R_3$ Wasserstoff, Fluor oder Methyl bedeutet; und

$R_4$ für Wasserstoff, für unsubstituiertes oder durch Fluor, Chlor, Brom, Hydroxy, $C_1$-$C_3$-Alkoxy oder $C_1$-$C_3$-Haloalkoxy substituiertes $C_1$-$C_6$-Alkyl, für einen unsubstituierten oder substituierten Rest ausgewählt aus der Reihe $C_3$-$C_7$-Cycloalkyl, $C_2$-$C_4$-Alkenyl und $C_2$-$C_4$-Alkinyl, wobei die Substituenten ausgewählt sind aus der Reihe Fluor, Chlor, Brom, Hydroxy, $C_1$-$C_3$-Alkoxy und $C_1$-$C_4$-Alkanoyloxy, für unsubstituiertes oder Fluor, Chlor, Brom, Methyl, Ethyl, Methoxy, Halomethoxy, Trifluormethyl und/oder Nitro substituiertes Phenyl oder für einen unsubstituierten oder substituierten, ungesättigten oder gesättigten, fünf-oder sechsgliedrigen heterocyclischen Ring mit ein bis drei Heteroatomen, ausgewählt aus der Reihe Stickstoff, Sauerstoff und Schwefel, dessen Substituenten ausgewählt sind aus der Reihe Oxo, Halogen, $C_1$-$C_4$-Alkyl, $C_1$-$C_4$-Haloalkyl und $C_1$-$C_4$-Alkoxy steht, unter Einschluss der Säureadditionssalze und Metallkomplexe.

9. Mittel nach Anspruch 5, enthaltend als Wirkstoff eine der Verbindungen der Formel I, worin

R für $C_1$-$C_6$-Alkyl steht;

$R_2$ für Methyl, Ethyl, Isopropyl oder sek.-Butyl steht;

$R_1$ die Gruppe

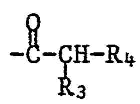

repräsentiert; wobei

$R_3$ für Wasserstoff, Fluor oder Methyl steht; und

$R_4$ für einen in 1-Position verknüpften, 5-gliedrigen Heteroaromaten mit 2 bis 3 Stickstoffatom steht, der unsubstituiert oder ein-bis dreifach durch $C_1$-$C_6$-Alkyl substituiert ist, steht, unter Einschluss der Säureadditionssalze mit organischen und anorganischen Säuren sowie der Metallkomplexe mit Metallkationen der ersten, zweiten, vierten oder achten Nebengruppe des Periodensystems der Elemente.

10. Mittel nach Anspruch 3, enthaltend als Wirkstoff eine der Verbindungen der Formel I, worin

R für $C_1$-$C_6$-Alkyl steht;

$R_2$ für Methyl, Ethyl, Isopropyl oder sek.-Butyl steht;

$R_1$ eine der Acylgruppen

a) 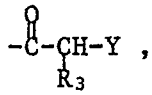 ,

b) 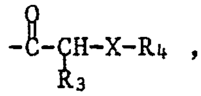 ,

c) 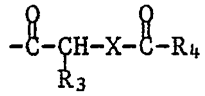 oder

d) 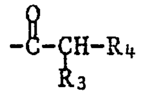 repräsentiert, wobei

X für Sauerstoff oder Schwefel steht;
Y Halogen bedeutet;
$R_3$ Wasserstoff, Halogen oder Methyl repräsentiert; und
$R_4$ für Wasserstoff, unsubstituiertes oder durch Halogen oder $C_1$-$C_4$-Alkoxy substituiertes $C_1$-$C_4$-Alkyl steht, für Cyclopropyl, für Cyclopentyl, für Cyclohexyl, für unsubstituiertes oder durch Fluor, Chlor, Brom, Methoxy, Trifluormethyl, Methyl und/oder Nitro substituiertes Phenyl, für 4H-2,3-Dihydropyran-2-yl oder für einen in 1-Position verknüpften, 5-gliedrigen Heteroaromaten mit 2 bis 3 Stickstoffatom steht, der unsubstituiert oder einbis dreifach durch $C_1$-$C_6$-Alkyl substituiert ist, steht, unter Einschluss der Säureadditionssalze mit organischen und anorganischen Säuren sowie der Metallkomplexe mit Metallkationen der ersten, zweiten, vierten oder achten Nebengruppe des Periodensystems der Elemente.

11. Mittel nach Anspruch 3, enthaltend als Wirkstoff eine der Verbindung der Formel I, ausgewählt aus der Reihe:
5-O-Chloracetyl-13β-methyl-milbemycin D,
5-O-Chloracetyl-13β-methyl-milbemycin $A_3$,
5-O-Chloracetyl-13β-methyl-milbemycin $A_4$,
5-O-Chloracetyl-13β-ethyl-milbemycin D,
5-O-Chloracetyl-13β-ethyl-milbemycin $A_3$,
5-O-Chloracetyl-13β-ethyl-milbemycin $A_4$,
5-O-Acetoxyacetyl-13β-methyl-milbemycin D,
5-O-Acetoxyacetyl-13β-methyl-milbemycin $A_3$,
5-O-Acetoxyacetyl-13β-methyl-milbemycin $A_4$,
5-O-Acetoxyacetyl-13β-ethyl-milbemycin D,
5-O-Acetoxyacetyl-13β-ethyl-milbemycin $A_3$,
5-O-Acetoxyacetyl-13β-ethyl-milbemycin $A_4$,
5-O-[3,4-Dihydro-2H-pyran-2-yl]carboxyacetyl-13β-methyl-milbemycin D,
5-O-[3,4-Dihydro-2H-pyran-2-yl]carboxyacetyl-13β-methyl-milbemycin $A_3$,
5-O-[3,4-Dihydro-2H-pyran-2-yl]carboxyacetyl-13β-methyl-milbemycin $A_4$,
5-O-[3,4-Dihydro-2H-pyran-2-yl]carboxyacetyl-13β-ethyl-milbemycin D,
5-O-[3,4-Dihydro-2H-pyran-2-yl]carboxyacetyl-13β-ethyl-milbemycin $A_3$,
5-O-[3,4-Dihydro-2H-pyran-2-yl]carboxyacetyl-13β-ethyl-milbemycin $A_4$,
5-O-[1,2,4-Triazol-4'-yl]acetyl-13β-methyl-milbemycin D,
5-O-[1,2,4-Triazol-4'-yl]acetyl-13β-methyl-milbemycin $A_3$,
5-O-[1,2,4-Triazol-4'-yl]acetyl-13β-methyl-milbemycin $A_4$,
5-O-[1,2,4-Triazol-4'-yl]acetyl-13β-ethyl-milbemycin D,
5-O-[1,2,4-Triazol-4'-yl]acetyl-13β-ethyl-milbemycin $A_3$,
5-O-[1,2,4-Triazol-4'-yl]acetyl-13β-ethyl-milbemycin $A_4$,
5-O-Methoxyacetyl-13β-methyl-milbemycin D,
5-O-Methoxyacetyl-13β-methyl-milbemycin $A_3$,
5-O-Methoxyacetyl-13β-methyl-milbemycin $A_4$,
5-O-Methoxyacetyl-13β-ethyl-milbemycin D,
5-O-Methoxyacetyl-13β-ethyl-milbemycin $A_3$,
5-O-Methoxyacetyl-13β-ethyl-milbemycin $A_4$,
5-O-[3-Chlorbenzoyloxy]acetyl-13β-methyl-milbemycin D,
5-O-[3-Chlorbenzoyloxy]acetyl-13β-methyl-milbemycin $A_3$,
5-O-[3-Chlorbenzoyloxy]acetyl-13β-methyl-milbemycin $A_4$,

5-O-[3-Chlorbenzoyloxy]acetyl-13β-ethyl-milbemycin D,
5-O-[3-Chlorbenzoyloxy]acetyl-13β-ethyl-milbemycin $A_3$, und
5-O-[3-Chlorbenzoyloxy]acetyl-13β-ethyl-milbemycin $A_4$.

12. Mittel nach Anspruch 3, enthaltend als Wirkstoff eine der Verbindung der Formel I, ausgewählt aus der Reihe:
5-O-Acetoxyacetyl-13β-propyl-milbemycin D,
5-O-Acetoxyacetyl-13β-propyl-milbemycin $A_3$,
5-O-Acetoxyacetyl-13β-propyl-milbemycin $A_4$,
5-O-Acetyl-13β-ethyl-milbemycin D,
5-O-Acetyl-13β-ethyl-milbemycin $A_3$,
5-O-Acetyl-13β-ethyl-milbemycin $A_4$,
5-O-Acetyl-13β-methyl-milbemycin D,
5-O-Acetyl-13β-methyl-milbemycin $A_3$,
5-O-Acetyl-13β-methyl-milbemycin $A_4$,
5-O-Methoxyacetyl-13β-butyl-milbemycin D,
5-O-Methoxyacetyl-13β-butyl-milbemycin $A_3$,
5-O-Methoxyacetyl-13β-butyl-milbemycin $A_4$,
5-O-Benzoyloxy-13β-methyl-milbemycin D,
5-O-Benzoyloxy-13β-methyl-milbemycin $A_3$ und
5-O-Benzoyloxy-13β-methyl-milbemycin $A_4$.

Europäisches Patentamt

# EUROPÄISCHER TEILRECHERCHENBERICHT,
der nach Regel 45 des Europäischen Patent-übereinkommens für das weitere Verfahren als europäischer Recherchenbericht gilt

Nummer der Anmeldung

EP 87103098.7

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| A | EP - A2/A3 - 0 147 852 (CIBA-GEIGY AG)<br>* Ansprüche 1,11,16-21 *<br>-- | 1,11, 13-16, 19,20 | C 07 D 493/22<br>A 01 N 43/20<br>A 61 K 31/365<br>//(C 07 D 493/22,<br>C 07 D 313:00,<br>C 07 D 311:00,<br>C 07 D 311:00,<br>C 07 D 307:00) |
| A | EP - A1 - 0 142 969 (SANKYO COMPANY LIMITED)<br>* Ansprüche 1, 13 *<br>-- | 1,11, 13-16, 19,20 | |
| A | EP - A2 - 0 165 900 (CIBA-GEIGY AG)<br>* Ansprüche 1,9,10-16 *<br>---- | 1,11, 13-16, 19,20 | |
| | | | RECHERCHIERTE SACHGEBIETE (Int. Cl 4) |
| | | | C 07 D 493/00 |

## UNVOLLSTÄNDIGE RECHERCHE

Nach Auffassung der Recherchenabteilung entspricht die vorliegende europäische Patentanmeldung den Vorschriften des Europäischen Patentübereinkommens so wenig, daß es nicht möglich ist, auf der Grundlage einiger Patentansprüche sinnvolle Ermittlungen über den Stand der Technik durchzuführen.

Vollständig recherchierte Patentansprüche: 1-14

Unvollständig recherchierte Patentansprüche: 15,16,19,20

Nicht recherchierte Patentansprüche: 17,18

Grund für die Beschränkung der Recherche:

(Artikel 52(4) EPÜ; Verfahren zur therapeutischen Behandlung des menschlichen oder tierischen Körpers)

| Recherchenort | Abschlußdatum der Recherche | Prüfer |
|---|---|---|
| WIEN | 12-06-1987 | BRUS |

KATEGORIE DER GENANNTEN DOKUMENTEN
X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPA Form 1505.1 03 82